(19) Europäisches Patentamt / European Patent Office / Office européen des brevets

(11) **EP 3 606 947 B1**

(12) **EUROPEAN PATENT SPECIFICATION**

(45) Date of publication and mention of the grant of the patent:
**21.12.2022 Bulletin 2022/51**

(21) Application number: **18715009.9**

(22) Date of filing: **29.03.2018**

(51) International Patent Classification (IPC):
**C07K 14/55** *(2006.01)* **A61K 38/20** *(2006.01)*
**C07K 16/28** *(2006.01)* **A61K 39/00** *(2006.01)*

(52) Cooperative Patent Classification (CPC):
**C07K 14/55; A61P 35/00; C07K 16/2818;**
A61K 38/00; A61K 2039/505; C07K 2317/73;
C07K 2319/74

(86) International application number:
**PCT/EP2018/058037**

(87) International publication number:
**WO 2018/184965 (11.10.2018 Gazette 2018/41)**

(54) **IMMUNOCONJUGATES OF IL-2 WITH AN ANTI-PD-1 AND TIM-3 BISPECIFIC ANTIBODY**

IMMUNOKONJUGAT VON IL-2 MIT EINEM BISPEZIFISCHEN ANTIKÖRPER GERICHTET GEGEN PD-1 UND TIM-3

IMMUNOCONJUGUÉ DE IL-2 AVEC UN ANTICORPS BISPÉCIFIQUE CONTRE PD-1 ET TIM-3

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2017 EP 17164538**

(43) Date of publication of application:
**12.02.2020 Bulletin 2020/07**

(73) Proprietor: **F. Hoffmann-La Roche AG**
**4070 Basel (CH)**

(72) Inventors:
• **CODARRI DEAK, Laura**
**8952 Schlieren (CH)**
• **KLEIN, Christian**
**8952 Schlieren (CH)**
• **LAUENER, Laura**
**8952 Schlieren (CH)**
• **SEEBER, Stefan**
**82404 Sindelsdorf (DE)**
• **UMAÑA, Pablo**
**8952 Schlieren (CH)**
• **WALDHAUER, Inja**
**8952 Schlieren (CH)**

(74) Representative: **Kaschau, Nikolai Elias et al**
**F. Hoffmann-La Roche AG**
**Patent Department CLP**
**Grenzacherstrasse 124**
**4070 Basel (CH)**

(56) References cited:
**WO-A1-2012/107417  WO-A1-2014/023679**
**WO-A1-2017/055404  WO-A2-2011/159877**

• **HU C.Y. ET AL: "Interleukin-2 reverses CD8+ T cell exhaustion in clinical malignang pleural effusion of lung cancer", CLIN. EXP. IMMUNOL., vol. 186, 2016, pages 106-114, XP002781415,**
• **SAKUISHI K. ET AL.: "Targeting Tim-3 and PD-1 pathways to reverse T cell exhaustion and restore anti-tumor immunity", J.EXP:MED., vol. 207, 27 September 2010 (2010-09-27), pages 2187-2194, XP002781421,**

Remarks:
The complete document including Reference Table(s) and the Sequence Listing(s) can be downloaded from the EPO website

**Description**

**Field of the invention**

The present invention generally relates to immunoconjugates, particularly immunoconjugates comprising a mutant interleukin-2 polypeptide and a bispecific antigen binding molecule that binds to PD-1 and Tim-3. In addition, the invention relates to polynucleotide molecules encoding the immunoconjugates, and vectors and host cells comprising such polynucleotide molecules. The invention further relates to methods for producing the mutant immunoconjugates, pharmaceutical compositions comprising the same, and uses thereof.

**Background**

Interleukin-2 (IL-2), also known as T cell growth factor (TCGF), is a 15.5 kDa globular glycoprotein playing a central role in lymphocyte generation, survival and homeostasis. It has a length of 133 amino acids and consists of four antiparallel, amphiphatic α-helices that form a quaternary structure indispensable of its function (Smith, Science 240, 1169-76 (1988); Bazan, Science 257, 410-413 (1992)). Sequences of IL-2 from different species are found under NCBI RefSeq Nos. NP000577 (human), NP032392 (mouse), NP446288 (rat) or NP517425 (chimpanzee).

IL-2 mediates its action by binding to IL-2 receptors (IL-2R), which consist of up to three individual subunits, the different association of which can produce receptor forms that differ in their affinity to IL-2. Association of the α (CD25), β (CD122), and γ ($\gamma_c$, CD132) subunits results in a trimeric, high-affinity receptor for IL-2. Dimeric IL-2 receptor consisting of the β and γ subunits is termed intermediate-affinity IL-2R. The α subunit forms the monomeric low affinity IL-2 receptor. Although the dimeric intermediate-affinity IL-2 receptor binds IL-2 with approximately 100-fold lower affinity than the trimeric high-affinity receptor, both the dimeric and the trimeric IL-2 receptor variants are able to transmit signal upon IL-2 binding (Minami et al., Annu Rev Immunol 11, 245-268 (1993)). Hence, the α-subunit, CD25, is not essential for IL-2 signalling. It confers high-affinity binding to its receptor, whereas the β subunit, CD122, and the γ-subunit are crucial for signal transduction (Krieg et al., Proc Natl Acad Sci 107, 11906-11 (2010)). Trimeric IL-2 receptors including CD25 are expressed by (resting) CD4[+] forkhead box P3 (FoxP3)[+] regulatory T ($T_{reg}$) cells. They are also transiently induced on conventional activated T cells, whereas in the resting state these cells express only dimeric IL-2 receptors. $T_{reg}$ cells consistently express the highest level of CD25 in vivo (Fontenot et al., Nature Immunol 6, 1142-51 (2005)).

IL-2 is synthesized mainly by activated T-cells, in particular CD4[+] helper T cells. It stimulates the proliferation and differentiation of T cells, induces the generation of cytotoxic T lymphocytes (CTLs) and the differentiation of peripheral blood lymphocytes to cytotoxic cells and lymphokine-activated killer (LAK) cells, promotes cytokine and cytolytic molecule expression by T cells, facilitates the proliferation and differentiation of B-cells and the synthesis of immunoglobulin by B-cells, and stimulates the generation, proliferation and activation of natural killer (NK) cells (reviewed e.g. in Waldmann, Nat Rev Immunol 6, 595-601 (2009); Olejniczak and Kasprzak, Med Sci Monit 14, RA179-89 (2008); Malek, Annu Rev Immunol 26, 453-79 (2008)). Moreover, it has been indicated that Malignant pleural effusion (MPE) CD8[+] T cells exhibit exhaustion phenotype, which can be reversed by IL-2 therapy (Hu et al., Clin. Exp. Immunol. 186:106-114 (2016)).

Its ability to expand lymphocyte populations in vivo and to increase the effector functions of these cells confers antitumor effects to IL-2, making IL-2 immunotherapy an attractive treatment option for certain metastatic cancers. Consequently, high-dose IL-2 treatment has been approved for use in patients with metastatic renal-cell carcinoma and malignant melanoma.

However, IL-2 has a dual function in the immune response in that it not only mediates expansion and activity of effector cells, but also is crucially involved in maintaining peripheral immune tolerance.

A major mechanism underlying peripheral self-tolerance is IL-2 induced activation-induced cell death (AICD) in T cells. AICD is a process by which fully activated T cells undergo programmed cell death through engagement of cell surface-expressed death receptors such as CD95 (also known as Fas) or the TNF receptor. When antigen-activated T cells expressing a high-affinity IL-2 receptor (after previous exposure to IL-2) during proliferation are re-stimulated with antigen via the T cell receptor (TCR)/CD3 complex, the expression of Fas ligand (FasL) and/or tumor necrosis factor (TNF) is induced, making the cells susceptible for Fas-mediated apoptosis. This process is IL-2 dependent (Lenardo, Nature 353, 858-61 (1991)) and mediated via STAT5. By the process of AICD in T lymphocytes tolerance can not only be established to self-antigens, but also to persistent antigens that are clearly not part of the host's makeup, such as tumor antigens.

Moreover, IL-2 is also involved in the maintenance of peripheral CD4[+] CD25[+] regulatory T ($T_{reg}$) cells (Fontenot et al., Nature Immunol 6, 1142-51 (2005); D'Cruz and Klein, Nature Immunol 6, 1152-59 (2005); Maloy and Powrie, Nature Immunol 6, 1171-72 (2005), which are also known as suppressor T cells. They suppress effector T cells from destroying their (self-)target, either through cell-cell contact by inhibiting T cell help and activation, or through release of immunosuppressive cytokines such as IL-10 or TGF-β. Depletion of $T_{reg}$ cells was shown to enhance IL-2 induced anti-tumor immunity (Imai et al., Cancer Sci 98, 416-23 (2007)).

Therefore, IL-2 is not optimal for inhibiting tumor growth, because in the presence of IL-2 either the CTLs generated might recognize the tumor as self and undergo AICD or the immune response might be inhibited by IL-2 dependent $T_{reg}$ cells.

A further concern in relation to IL-2 immunotherapy are the side effects produced by recombinant human IL-2 treatment. Patients receiving high-dose IL-2 treatment frequently experience severe cardiovascular, pulmonary, renal, hepatic, gastrointestinal, neurological, cutaneous, haematological and systemic adverse events, which require intensive monitoring and in-patient management. The majority of these side effects can be explained by the development of so-called vascular (or capillary) leak syndrome (VLS), a pathological increase in vascular permeability leading to fluid extravasation in multiple organs (causing e.g. pulmonary and cutaneous edema and liver cell damage) and intravascular fluid depletion (causing a drop in blood pressure and compensatory increase in heart rate). There is no treatment of VLS other than withdrawal of IL-2. Low-dose IL-2 regimens have been tested in patients to avoid VLS, however, at the expense of suboptimal therapeutic results. VLS was believed to be caused by the release of proinflammatory cytokines, such as tumor necrosis factor (TNF)-$\alpha$ from IL-2-activated NK cells, however it has recently been shown that IL-2-induced pulmonary edema resulted from direct binding of IL-2 to lung endothelial cells, which expressed low to intermediate levels of functional $\alpha\beta\gamma$ IL-2 receptors (Krieg et al., Proc Nat Acad Sci USA 107, 11906-11 (2010)).

Several approaches have been taken to overcome these problems associated with IL-2 immunotherapy. For example, it has been found that the combination of IL-2 with certain anti-IL-2 monoclonal antibodies enhances treatment effects of IL-2 in vivo (Kamimura et al., J Immunol 177, 306-14 (2006); Boyman et al., Science 311, 1924-27 (2006)). In an alternative approach, IL-2 has been mutated in various ways to reduce its toxicity and/or increase its efficacy. Hu et al. (Blood 101, 4853-4861 (2003), US Pat. Publ. No. 2003/0124678) have substituted the arginine residue in position 38 of IL-2 by tryptophan to eliminate IL-2's vasopermeability activity. Shanafelt et al. (Nature Biotechnol 18, 1197-1202 (2000)) have mutated asparagine 88 to arginine to enhance selectivity for T cells over NK cells. Heaton et al. (Cancer Res 53, 2597-602 (1993); US Pat. No. 5,229,109) have introduced two mutations, Arg38Ala and Phe42Lys, to reduce the secretion of proinflammatory cytokines from NK cells. Gillies et al. (US Pat. Publ. No. 2007/0036752) have substituted three residues of IL-2 (Asp20Thr, Asn88Arg, and Gln126Asp) that contribute to affinity for the intermediate-affinity IL-2 receptor to reduce VLS. Gillies et al. (WO 2008/0034473) have also mutated the interface of IL-2 with CD25 by amino acid substitution Arg38Trp and Phe42Lys to reduce interaction with CD25 and activation of $T_{reg}$ cells for enhancing efficacy. To the same aim, Wittrup et al. (WO 2009/061853) have produced IL-2 mutants that have enhanced affinity to CD25, but do not activate the receptor, thus act as antagonists. The mutations introduced were aimed at disrupting the interaction with the $\beta$- and/or $\gamma$-subunit of the receptor.

A particular mutant IL-2 polypeptide, designed to overcome the above-mentioned problems associated with IL-2 immunotherapy (toxicity caused by the induction of VLS, tumor tolerance caused by the induction of AICD, and immunosuppression caused by activation of $T_{reg}$ cells), is described in WO 2012/107417. Substitution of the phenylalanine residue at position 42 by alanine, the tyrosine residue at position 45 by alanine and the leucine residue at position 72 of IL-2 by glycine essentially abolishes binding of this mutant IL-2 polypeptide to the $\alpha$-subunit of the IL-2 receptor (CD25).

Further to the above-mentioned approaches, IL-2 immunotherapy may be improved by selectively targeting IL-2 to tumors, e.g. in the form of immunoconjugates comprising an antibody that binds to an antigen expressed on tumor cells. Several such immunoconjugates have been described (see e.g. Ko et al., J Immunother (2004) 27, 232-239; Klein et al., Oncoimmunology (2017) 6(3), e1277306).

Tumors may be able, however, to escape such targeting by shedding, mutating or downregulating the target antigen of the antibody. Moreover, tumor-targeted IL-2 may not come into optimal contact with effector cells such as cytotoxic T lymphocytes (CTLs), in tumor microenvironments that actively exclude lymphocytes.

Thus there remains a need to further improve IL-2 immunotherapy. An approach, which may circumvent the problems of tumor-targeting, is to target IL-2 directly to effector cells, in particular CTLs.

Ghasemi et al. have described a fusion protein of IL-2 and an NKG2D binding protein (Ghashemi et al., Nat Comm (2016) 7, 12878), for targeting IL-2 to NKG2D-bearing cells such as natural killer (NK) cells.

Programmed cell death protein 1 (PD-1 or CD279) is an inhibitory member of the CD28 family of receptors, that also includes CD28, CTLA-4, ICOS and BTLA. PD-1 is a cell surface receptor and is expressed on activated B cells, T cells, and myeloid cells (Okazaki et al (2002) Curr. Opin. Immunol. 14: 391779-82; Bennett et al. (2003) J Immunol 170:711-8). The structure of PD-1 is a monomeric type 1 transmembrane protein, consisting of one immunoglobulin variable-like extracellular domain and a cytoplasmic domain containing an immunoreceptor tyrosine-based inhibitory motif (ITIM) and an immunoreceptor tyrosine-based switch motif (ITSM). Two ligands for PD-1 have been identified, PD-L1 and PD-L2, that have been shown to downregulate T cell activation upon binding to PD-1 (Freeman et al (2000) J Exp Med 192: 1027-34; Latchman et al (2001) Nat Immunol 2:261-8; Carter etal (2002) Eur J Immunol 32:634-43). Both PD-L1 and PD-L2 are B7 homologs that bind to PD-1, but do not bind to other CD28 family members. One ligand for PD-1, PD-L1 is abundant in a variety of human cancers (Dong et al (2002) Nat. Med 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression (T cell dysfunction or exhaustion) can be reversed by inhibiting the local interaction of PD-1 with PD-L1, and the effect is additive when the interaction of PD-1 with PD-L2 is blocked as well (Iwai et al. (2002) Proc. Nat 7. Acad. ScL USA 99: 12293-7; Brown et al. (2003) J. Immunol. 170:1257-66).

However, targeting the PD-1-PD-L1 pathway alone does not always result in reversal of T cell exhaustion (Gehring et al., Gastroenterology 137 (2009), 682-690), indicating that other molecules are likely involved in T cell exhaustion (Sakuishi, J. Experimental Med. 207 (2010), 2187-2194).

Tim-3 is a molecule originally identified as being selectively expressed on IFN-γ-secreting Th1 and Tc1 cells (Monney et al., Nature 415 (2002), 536-541). The interaction of Tim-3 with its ligand, galectin-9, triggers cell death in Tim-3+ T cells. Thus, both Tim-3 and PD-1 can function as negative regulators of T cell responses. It has been shown that Tim-3 marks the most suppressed or dysfunctional population of CD8+ T cells in preclinical models of both solid and hematologic malignancy (Sakuishi, J. Experimental Med. 207 (2010), 2187-2194; Zhou, Blood 117 (2011), 4501-4510; Majeti R et al., PNAS, 106 (2009), 3396-3401). In these models, all of the CD8+ Tim-3+ T cells coexpress PD-1, and these dual-expressing cells exhibit greater defects in both cell-cycle progression and effector cytokine production [interleukin (IL)-2, TNF, and IFN-γ] than cells that express PD-1 alone. Thus, the Tim-3 pathway may cooperate with the PD-1 pathway to promote the development of a severe dysfunctional phenotype in CD8+ T cells in cancer. The combined targeting of the Tim-3 and PD-1 pathways is thus expected to be highly effective in controlling tumor growth.

Bispecific antibodies that bind to PD-1 and Tim-3 are described e.g. PCT patent application no. PCT/EP2016/073192. These bispecific antibodies do not only effectively block PD-1 and Tim-3 on T cells overexpressing both antigens, they are very selective for these cells and thereby may avoid side effects associated with blocking of Tim-3 on other cells such as innate immune cells (e.g. naive dendritic cells (DCs) and monocytes).

## Summary of the invention

The present invention provides a novel approach of targeting a mutant form of IL-2 with advantageous properties for immunotherapy directly to immune effector cells, such as cytotoxic T lymphocytes, rather than tumor cells. Targeting to immune effector cells is achieved by conjugation of the mutant IL-2 molecule to bispecific antigen binding molecule that binds to PD-1 and Tim-3.

The IL-2 mutant used in the present invention has been designed to overcome the problems associated with IL-2 immunotherapy, in particular toxicity caused by the induction of VLS, tumor tolerance caused by the induction of AICD, and immunosuppression caused by activation of $T_{reg}$ cells. In addition to circumventing escape of tumors from tumor-targeting as mentioned above, targeting of the IL-2 mutant to immune effector cells may further increase the preferential activation of CTLs over immunosuppressive $T_{reg}$ cells. By using a bispecific antigen binding molecule that binds to PD-1 and Tim-3, the suppression of T-cell activity induced by the PD-1/PD-L1 pathway and/or Tim-3 may additionally be reversed, thus further enhancing the immune response. Combined targeting to PD-1 and Tim-3 further enhances targeting of the immunoconjugate particularly to exhausted T cells, which express both antigens.

In a first aspect, the invention provides an immunoconjugate comprising a mutant IL-2 polypeptide and a bispecific antigen binding molecule that binds to PD-1 and Tim-3, wherein the mutant IL-2 polypeptide is a human IL-2 molecule comprising the amino acid substitutions F42A, Y45A and L72G (numbering relative to the human IL-2 sequence SEQ ID NO: 22), wherein the mutant IL-2 has reduced or abolished affinity to the alpha subunit of the IL-2 receptor but preserves affinity to the intermediate affinity IL-2 receptor; and wherein the bispecific antigen binding molecule comprises (i) a first antigen binding moiety that binds to PD-1, and (ii) a second antigen binding moiety that binds to Tim-3.

In some embodiments, the first antigen binding moiety comprises (a) a heavy chain variable region (VH) comprising a HVR-H1 comprising the amino acid sequence of SEQ ID NO:1, a HVR-H2 comprising the amino acid sequence of SEQ ID NO:2, and a HVR-H3 comprising the amino acid sequence of SEQ ID NO:3, and (b) a light chain variable region (VL) comprising a HVR-L1 comprising the amino acid sequence of SEQ ID NO:4, a HVR-L2 comprising the amino acid sequence of SEQ ID NO:5, and a HVR-L3 comprising the amino acid sequence of SEQ ID NO:6. In some embodiments, the first antigen binding moiety comprises (a) a heavy chain variable region (VH) comprising an amino acid sequence that is at least about 95%, 96%, 97%, 98%, 99% or 100% identical to the amino acid sequence of SEQ ID NO:7, and (b) a light chain variable region (VL) comprising an amino acid sequence that is at least about 95%, 96%, 97%, 98%, 99% or 100% identical to an amino acid sequence selected from the group consisting of SEQ ID NO:8, SEQ ID NO:9, SEQ ID NO: 10, and SEQ ID NO:11.

In some embodiments, the second antigen binding moiety comprises (a) a heavy chain variable region (VH) comprising a HVR-H1 comprising the amino acid sequence of SEQ ID NO:12, a HVR-H2 comprising the amino acid sequence of SEQ ID NO:13, and a HVR-H3 comprising the amino acid sequence of SEQ ID NO:14, and (b) a light chain variable region (VL) comprising a HVR-L1 comprising the amino acid sequence of SEQ ID NO: 15, a HVR-L2 comprising the amino acid sequence of SEQ ID NO:16, and a HVR-L3 comprising the amino acid sequence of SEQ ID NO: 17. In some embodiments, the second antigen binding moiety comprises (a) a heavy chain variable region (VH) comprising an amino acid sequence that is at least about 95%, 96%, 97%, 98%, 99% or 100% identical to the amino acid sequence of SEQ ID NO:18, and (b) a light chain variable region (VL) comprising an amino acid sequence that is at least about 95%, 96%, 97%, 98%, 99% or 100% identical to the amino acid sequence of SEQ ID NO:19, or (a) a heavy chain variable region (VH) comprising an amino acid sequence that is at least about 95%, 96%, 97%, 98%, 99% or 100% identical to the amino acid sequence of SEQ ID NO:20, and (b) a light chain variable region (VL) comprising an amino acid sequence that is at least about 95%, 96%, 97%, 98%, 99% or 100% identical to the amino acid sequence of SEQ ID NO:21.

In some embodiments, the mutant IL-2 polypeptide further comprises the amino acid substitution T3A and/or the amino acid substitution C125A.In some embodiments, the mutant IL-2 polypeptide comprises the sequence of SEQ ID NO: 23. In some embodiments, the immunoconjugate comprises not more than one mutant IL-2 polypeptide.

In some embodiments, the first and/or the second antigen binding moiety is a Fab molecule. In some embodiments, the first or the second antigen binding moiety is a Fab molecule wherein the variable domains VL and VH or the constant domains CL and CH1, particularly the variable domains VL and VH, of the Fab light chain and the Fab heavy chain are replaced by each other. In some embodiments, the first or the second antigen binding moiety is a Fab molecule wherein in the constant domain the amino acid at position 124 is substituted independently by lysine (K), arginine (R) or histidine (H) (numbering according to Kabat) and the amino acid at position 123 is substituted independently by lysine (K), arginine (R) or histidine (H) (numbering according to Kabat), and in the constant domain CH1 the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index). In some embodiments, the first antigen binding moiety is a Fab molecule wherein the variable domains VL and VH of the Fab light chain and the Fab heavy chain are replaced by each other, and the second antigen binding moiety is a Fab molecule wherein in the constant domain the amino acid at position 124 is substituted lysine (K) (numbering according to Kabat) and the amino acid at position 123 is substituted independently by lysine (K) or arginine (R), particularly by arginine (R) (numbering according to Kabat), and in the constant domain CH1 the amino acid at position 147 is substituted by glutamic acid (E) (numbering according to Kabat EU index) and the amino acid at position 213 is substituted by glutamic acid (E) (numbering according to Kabat EU index).

In some embodiments, the bispecific antigen binding molecule further comprises an Fc domain composed of a first and a second subunit. In some embodiments, the Fc domain is an IgG class, particularly an IgG$_1$ subclass, Fc domain. In some embodiments, the Fc domain is a human Fc domain. In some embodiments, the Fc domain comprises a modification promoting the association of the first and the second subunit of the Fc domain. In some embodiments, in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable. In some embodiments, in the first subunit of the Fc domain the threonine residue at position 366 is replaced with a tryptophan residue (T366W), and in the CH3 domain of te second subunit of the Fc domain the tyrosine residue at position 407 is replaced with a valine residue (Y407V) and optionally the threonine residue at position 366 is replaced with a serine residue (T366S) and the leucine residue at position 368 is replaced with an alanine residue (L368A) (numberings according to Kabat EU index). In some such embodiments, in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C) (numberings according to Kabat EU index).

In some embodiments, the mutant IL-2 polypeptide is fused at its amino-terminal amino acid to the carboxy-terminal amino acid of one of the subunits of the Fc domain, particularly the first subunit of the Fc domain, optionally through a linker peptide. In some embodiments, the linker peptide has the amino acid sequence of SEQ ID NO:24.

In some embodiments, the first antigen binding moiety is a Fab molecule and is fused at the C-terminus of the Fab heavy chain to the N-terminus of one of the subunits of the Fc domain, particularly to the first subunit of the Fc domain, and the second antigen binding moiety is a Fab molecule and is fused at the C-terminus of the Fab heavy chain to the N-terminus of one of the subunits of the Fc domain, particularly to the second subunit of the Fc domain. In some embodiments, the first and the second antigen binding moiety are each fused to the Fc domain through an immunoglobulin hinge region.

In some embodiments, the Fc domain comprises one or more amino acid substitution that reduces binding to an Fc receptor, particularly an Fcγ receptor, and/or effector function, particularly antibody-dependent cell-mediated cytotoxicity (ADCC). In some such embodiments, said one or more amino acid substitution is at one or more position selected from the group of L234, L235, and P329 (Kabat EU index numbering). In some embodiments, each subunit of the Fc domain comprises the amino acid substitutions L234A, L235A and P329G (Kabat EU index numbering).

In some embodiments, the immunoconjugate comprises a polypeptide comprising an amino acid sequence that is at least about 80%, 85%, 90%, 95%, 96%, 97%, 98%, 99% or 100% identical to the sequence of SEQ ID NO:25, a polypeptide comprising an amino acid sequence that is at least about 80%, 85%, 90%, 95%, 96%, 97%, 98%, 99% or 100% identical to the sequence of SEQ ID NO:26, a polypeptide comprising an amino acid sequence that is at least about 80%, 85%, 90%, 95%, 96%, 97%, 98%, 99% or 100% identical to the sequence of SEQ ID NO:27, and a polypeptide comprising an amino acid sequence that is at least about 80%, 85%, 90%, 95%, 96%, 97%, 98%, 99% or 100% identical to the sequence of SEQ ID NO:28.

In some embodiments, the immunoconjugate essentially consists of the mutant IL-2 polypeptide and an IgG$_1$ immunoglobulin molecule as defined in the claims, wherein the heavy and light chain variable or constant regions in one of the Fab molecules are replaced by each other, joined by a linker sequence.

The invention further provides one or more isolated polynucleotide encoding an immunoconjugate of the invention, one or more vector (particularly expression vector) comprising said polynucleotides, and host cells comprising said polynucleotide(s) or said vector(s).

Also provided is a method of producing an immunoconjugate comprising a mutant IL-2 polypeptide and a bispecific antigen binding molecule that binds to PD-1 and Tim-3, comprising (a) culturing the host cell of the invention under conditions suitable for the expression of the immunoconjugate, and optionally (b) recovering the immunoconjugate.

The invention further provides a pharmaceutical composition comprising an immunoconjugate of the invention and a pharmaceutically acceptable carrier.

In particular, the invention encompasses an immunoconjugate according to the invention for use as a medicament, and for use in the treatment of cancer.

## Detailed Description

The technical disclosure set out below may in some respects go beyond the scope of the invention, which is defined by the appended claims. Elements of the disclosure which do not fall within the scope of the claims are provided for information, namely to place the actual invention in a broader technical context.

## Definitions

Terms are used herein as generally used in the art, unless otherwise defined in the following.

The term "interleukin-2" or "IL-2" as used herein, refers to any native IL-2 from any vertebrate source, including mammals such as primates (e.g. humans) and rodents (e.g., mice and rats), unless otherwise indicated. The term encompasses unprocessed IL-2 as well as any form of IL-2 that results from processing in the cell. The term also encompasses naturally occurring variants of IL-2, e.g. splice variants or allelic variants. The amino acid sequence of an exemplary human IL-2 is shown in SEQ ID NO: 22. Unprocessed human IL-2 additionally comprises an N-terminal 20 amino acid signal peptide having the sequence of SEQ ID NO: 32, which is absent in the mature IL-2 molecule.

The term "IL-2 mutant" or "mutant IL-2 polypeptide" as used herein is intended to encompass any mutant forms of various forms of the IL-2 molecule including full-length IL-2, truncated forms of IL-2 and forms where IL-2 is linked to another molecule such as by fusion or chemical conjugation. "Full-length" when used in reference to IL-2 is intended to mean the mature, natural length IL-2 molecule. For example, full-length human IL-2 refers to a molecule that has 133 amino acids (see e.g. SEQ ID NO: 22). The various forms of IL-2 mutants are characterized in having a at least one amino acid mutation affecting the interaction of IL-2 with CD25. This mutation may involve substitution, deletion, truncation or modification of the wild-type amino acid residue normally located at that position. Mutants obtained by amino acid substitution are preferred. Unless otherwise indicated, an IL-2 mutant may be referred to herein as a mutant IL-2 peptide sequence, a mutant IL-2 polypeptide, a mutant IL-2 protein or a mutant IL-2 analog.

Designation of various forms of IL-2 is herein made with respect to the sequence shown in SEQ ID NO: 22. Various designations may be used herein to indicate the same mutation. For example a mutation from phenylalanine at position 42 to alanine can be indicated as 42A, A42, $A_{42}$, F42A, or Phe42Ala.

By a "human IL-2 molecule" as used herein is meant an IL-2 molecule comprising an amino acid sequence that is at least about 90%, at least about 91%, at least about 92%, at least about 93%, at least about 94%, at least about 95% or at least about 96% identical to the human IL-2 sequence of SEQ ID NO:22. Particularly, the sequence identity is at least about 95%, more particularly at least about 96%. In particular embodiments, the human IL-2 molecule is a full-length IL-2 molecule.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g. reduced binding to CD25. Amino acid sequence deletions and insertions include amino- and/or carboxy-terminal deletions and insertions of amino acids. An example of a terminal deletion is the deletion of the alanine residue in position 1 of full-length human IL-2. Preferred amino acid mutations are amino acid substitutions. For the purpose of altering e.g. the binding characteristics of an IL-2 polypeptide, non-conservative amino acid substitutions, i.e. replacing one amino acid with another amino acid having different structural and/or chemical properties, are particularly preferred. Preferred amino acid substitions include replacing a hydrophobic by a hydrophilic amino acid. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful.

As used herein, a "wild-type" form of IL-2 is a form of IL-2 that is otherwise the same as the mutant IL-2 polypeptide except that the wild-type form has a wild-type amino acid at each amino acid position of the mutant IL-2 polypeptide. For example, if the IL-2 mutant is the full-length IL-2 (i.e. IL-2 not fused or conjugated to any other molecule), the wild-type form of this mutant is full-length native IL-2. If the IL-2 mutant is a fusion between IL-2 and another polypeptide encoded downstream of IL-2 (e.g. an antibody chain) the wild-type form of this IL-2 mutant is IL-2 with a wild-type amino acid sequence, fused to the same downstream polypeptide. Furthermore, if the IL-2 mutant is a truncated form of IL-2 (the mutated or modified sequence within the non-truncated portion of IL-2) then the wild-type form of this IL-2 mutant is a similarly truncated IL-2 that has a wild-type sequence. For the purpose of comparing IL-2 receptor binding affinity or biological activity of various forms of IL-2 mutants to the corresponding wild-type form of IL-2, the term wild-type encompasses forms of IL-2 comprising one or more amino acid mutation that does not affect IL-2 receptor binding compared to the naturally occurring, native IL-2, such as e.g. a substitution of cysteine at a position corresponding to residue 125 of human IL-2 to alanine. In certain embodiments according to the invention the wild-type IL-2 polypeptide to which the mutant IL-2 polypeptide is compared comprises the amino acid sequence of SEQ ID NO: 22. The term "CD25" or "a-subunit of the IL-2 receptor" as used herein, refers to any native CD25 from any vertebrate source, including mammals such as primates (e.g. humans) and rodents (e.g., mice and rats), unless otherwise indicated. The term encompasses "full-length", unprocessed CD25 as well as any form of CD25 that results from processing in the cell. The term also encompasses naturally occurring variants of CD25, e.g. splice variants or allelic variants. In certain embodiments CD25 is human CD25. The amino acid sequence of human CD25 is found e.g. in UniProt entry no. P01589 (version 185).

The term "high-affinity IL-2 receptor" as used herein refers to the heterotrimeric form of the IL-2 receptor, consisting of the receptor γ-subunit (also known as common cytokine receptor γ-subunit, $γ_c$, or CD132, see UniProt entry no. P14784 (version 192)), the receptor β-subunit (also known as CD122 or p70, see UniProt entry no. P31785 (version 197)) and the receptor α-subunit (also known as CD25 or p55, see UniProt entry no. P01589 (version 185)). The term "intermediate-affinity IL-2 receptor" by contrast refers to the IL-2 receptor including only the γ-subunit and the β-subunit, without the α-subunit (for a review see e.g. Olejniczak and Kasprzak, Med Sci Monit 14, RA179-189 (2008)).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant ($K_D$), which is the ratio of dissociation and association rate constants ($k_{off}$ and $k_{on}$, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

The affinity of the mutant or wild-type IL-2 polypeptide for various forms of the IL-2 receptor can be determined in accordance with the method set forth in the WO 2012/107417 by surface plasmon resonance (SPR), using standard instrumentation such as a BIAcore instrument (GE Healthcare) and receptor subunits such as may be obtained by recombinant expression (see e.g. Shanafelt et al., Nature Biotechnol 18, 1197-1202 (2000)). Alternatively, binding affinity of IL-2 mutants for different forms of the IL-2 receptor may be evaluated using cell lines known to express one or the other such form of the receptor. Specific illustrative and exemplary embodiments for measuring binding affinity are described hereinafter.

By "regulatory T cell" or "$T_{reg}$ cell" is meant a specialized type of CD4+ T cell that can suppress the responses of other T cells. $T_{reg}$ cells are characterized by expression of the α-subunit of the IL-2 receptor (CD25) and the transcription factor forkhead box P3 (FOXP3) (Sakaguchi, Annu Rev Immunol 22, 531-62 (2004)) and play a critical role in the induction and maintenance of peripheral self-tolerance to antigens, including those expressed by tumors. $T_{reg}$ cells require IL-2 for their function and development and induction of their suppressive characteristics.

As used herein, the term "effector cells" refers to a population of lymphocytes that mediate the cytotoxic effects of IL-2. Effector cells include effector T cells such as CD8+cytotoxic T cells, NK cells, lymphokine-activated killer (LAK) cells and macrophages/monocytes.

As used herein, the term "PD1", "human PD1", "PD-1" or "human PD-1" (also known as Programmed cell death protein 1, or Programmed Death 1) refers to the human protein PD1 (SEQ ID NO: 33, protein without signal sequence) / (SEQ ID NO: 34, protein with signal sequence). See also UniProt entry no. Q15116 (version 156). As used herein, an antibody (or antigen binding moiety) "binding to PD-1", "specifically binding to PD-1", "that binds to PD-1" or "anti-PD-1 antibody" refers to an antibody (or antigen binding moiety) that is capable of binding PD-1, especially a PD-1 polypeptide expressed on a cell surface, with sufficient affinity such that the antibody is useful as a diagnostic and/or therapeutic agent in targeting PD-1. In one embodiment, the extent of binding of an anti-PD-1 antibody to an unrelated, non-PD-1 protein is less than about 10% of the binding of the antibody to PD-1 as measured, e.g., by radioimmunoassay (RIA) or flow cytometry (FACS) or by a Surface Plasmon Resonance assay using a biosensor system such as a Biacore® system. In certain embodiments, an antibody (or antigen binding moiety) that binds to PD-1 has a KD value of the binding affinity for binding to human PD-1 of $\leq 1\ \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In one embodiment, the KD value of the binding affinity is determined in a Surface Plasmon Resonance assay using the Extracellular domain (ECD) of human PD-1 (PD-1-ECD, see SEQ ID NO: 35) as antigen.

As used herein, the term "Tim-3", "human Tim-3", "TIM3" or "human TIM3" (also known as "T cell Immunoglobulin- and Mucin domain-containing molecule 3") refers to the human protein Tim-3 (SEQ ID NO: 36, protein without signal sequence) / (SEQ ID NO: 37, protein with signal sequence). See also UniProt entry no. Q8TDQ0 (version 123). As used herein, an antibody (or antigen binding domain) "binding to Tim-3", "specifically binding to Tim-3", "that binds to Tim-3" or "anti-Tim-3 antibody" refers to an antibody (or antigen binding domain) that is capable of binding Tim-3, especially a Tim-3 polypeptide expressed on a cell surface, with sufficient affinity such that the antibody is useful as a diagnostic and/or therapeutic agent in targeting Tim-3. In one embodiment, the extent of binding of an anti-Tim-3 antibody to an unrelated, non-Tim-3 protein is less than about 10% of the binding of the antibody to Tim-3 as measured, e.g., by radioimmunoassay (RIA) or flow cytometry (FACS) or by a Surface Plasmon Resonance assay using a biosensor system such as a Biacore® system. In certain embodiments, an antibody (or antigen binding moiety) that binds to Tim-3 has a KD value of the binding affinity for binding to human Tim-3 of $\leq 1\ \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In one embodiment, the KD value of the binding affinity is determined in a Surface Plasmon Resonance assay using the Extracellular domain (ECD) of human Tim-3 (Tim-3-ECD, see SEQ ID NO: 38) as antigen.

By "specific binding" is meant that the binding is selective for the antigen and can be discriminated from unwanted or non-specific interactions. The ability of an antibody to bind to a specific antigen (e.g. PD-1) can be measured either through an enzyme-linked immunosorbent assay (ELISA) or other techniques familiar to one of skill in the art, e.g. surface plasmon resonance (SPR) technique (analyzed e.g. on a BIAcore instrument) (Liljeblad et al., Glyco J 17, 323-329 (2000)), and traditional binding assays (Heeley, Endocr Res 28, 217-229 (2002)). In one embodiment, the extent of binding of an antibody to an unrelated protein is less than about 10% of the binding of the antibody to the antigen as measured, e.g., by SPR. The antigen binding moieties of the bispecific antigen binding molecule comprised in the immunoconjugate described herein specifically bind to PD-1 or Tim-3.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein", "amino acid chain", or any other term used to refer to a chain of two or more amino acids, are included within the definition of "polypeptide", and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, Clustal W, Megalign (DNASTAR) software or the FASTA program package. Those skilled in the art can determine appropriate parameters for aligning sequences, including any algorithms needed to achieve maximal alignment over the full length of the sequences being compared. For purposes herein, however, % amino acid sequence identity values are generated using the ggsearch program of the FASTA package version 36.3.8c or later with a BLOSUM50 comparison matrix. The FASTA program package was authored by W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227-258; and Pearson et. al. (1997) Genomics 46:24-36, and is publicly available from http://fasta.bioch.virginia.edu/fasta_www2/fasta_down.shtml. Alternatively, a public server accessible at http://fasta.bioch.virginia.edu/fasta_www2/index.cgi can be used to compare the sequences, using the ggsearch (global protein:protein) program and default options (BLOSUM50; open: -10; ext: -2; Ktup = 2) to ensure a global, rather than local, alignment is performed. Percent amino acid identity is given in the output alignment header.

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

"Isolated polynucleotide (or nucleic acid) encoding [e.g. an immunoconjugate of the invention]" refers to one or more polynucleotide molecules encoding antibody heavy and light chains and/or IL-2 polypeptides (or fragments thereof), including such polynucleotide molecule(s) in a single vector or separate vectors, and such nucleic acid molecule(s) present at one or more locations in a host cell.

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette comprises polynucleotide sequences that encode immunoconjugates of the invention or fragments thereof.

The term "vector" or "expression vector" refers to a DNA molecule that is used to introduce and direct the expression of a specific gene to which it is operably associated in a cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the cell, the ribonucleic acid molecule or protein that is encoded by the gene is produced by the cellular transcription and/or translation machinery. In one embodiment, the expression vector of the invention comprises an expression cassette that comprises polynucleotide sequences that encode immunoconjugates of the invention or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the immunoconjugates of the present invention. Host cells include cultured cells, *e.g.* mammalian cultured cells, such as HEK cells, CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

The term "antibody" herein is used in the broadest sense and encompasses various antibody structures, including but not limited to monoclonal antibodies, polyclonal antibodies, multispecific antibodies (e.g. bispecific antibodies), and antibody fragments so long as they exhibit the desired antigen binding activity.

As used herein, the term "antigen binding molecule" refers in its broadest sense to a molecule that specifically binds an antigenic determinant. Examples of antigen binding molecules are immunoglobulins and derivatives, e.g.

fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant..

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigen binding moiety" refers to a polypeptide molecule that specifically binds to an antigenic determinant. An antigen binding moiety is able to direct the entity to which it is attached (e.g. an IL-2 polypeptide) to a target site, for example to a specific type of tumor cell bearing the antigenic determinant. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: $\alpha$, $\delta$, $\varepsilon$, $\gamma$, or $\mu$. Useful light chain constant regions include any of the two isotypes: $\kappa$ and $\lambda$.

As used herein, the term "antigenic determinant" is synonymous with "antigen" and "epitope", and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM).

The term "monoclonal antibody" as used herein refers to an antibody obtained from a population of substantially homogeneous antibodies, i.e. the individual antibodies comprised in the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

An "isolated" antibody is one which has been separated from a component of its natural environment, i.e. that is not in its natural milieu. No particular level of purification is required. For example, an isolated antibody can be removed from its native or natural environment.

Recombinantly produced antibodies expressed in host cells are considered isolated for the purpose of the invention, as are native or recombinant antibodies which have been separated, fractionated, or partially or substantially purified by any suitable technique. As such, the immunoconjugates of the present invention are isolated. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC) methods. For review of methods for assessment of antibody purity, see, e.g., Flatman et al., J. Chromatogr. B 848:79-87 (2007).

The terms "full-length antibody," "intact antibody," and "whole antibody" are used herein interchangeably to refer to an antibody having a structure substantially similar to a native antibody structure.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$, diabodies, linear antibodies, single-chain antibody molecules (e.g. scFv), and single-domain antibodies. For a review of certain antibody fragments, see Holliger and Hudson, Nature Biotechnology 23:1126-1136 (2005). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable domain (VH), also called a variable heavy domain or a heavy chain variable region, followed by three constant domains (CH1, CH2, and CH3), also called a heavy chain constant region. Similarly, from N- to C-terminus, each light chain has a variable domain (VL), also called a variable light domain or a light chain variable region, followed by a constant light (CL) domain, also called a light chain constant region. The heavy chain of an immunoglobulin may be assigned to one of five types, called $\alpha$ (IgA), $\delta$ (IgD), $\epsilon$ (IgE), $\gamma$ (IgG), or $\mu$ (IgM), some of which may be further divided into subtypes, e.g. $\gamma_1$ (IgG$_1$), $\gamma_2$ (IgG$_2$), $\gamma_3$ (IgG$_3$), $\gamma_4$ (IgG$_4$), $\alpha_1$ (IgA$_1$) and $\alpha_2$ (IgA$_2$). The light chain of an immunoglobulin may be assigned to one of two types, called kappa ($\kappa$) and lambda ($\lambda$), based on the amino acid sequence of its constant domain. An immunoglobulin essentially consists of two Fab molecules and an Fc domain, linked via the immunoglobulin hinge region.

A "Fab molecule" refers to a protein consisting of the VH and CH1 domain of the heavy chain (the "Fab heavy chain") and the VL and CL domain of the light chain (the "Fab light chain") of an immunoglobulin.

By a "crossover" Fab molecule (also termed "Crossfab") is meant a Fab molecule wherein the variable domains or the constant domains of the Fab heavy and light chain are exchanged (i.e. replaced by each other), i.e. the crossover Fab molecule comprises a peptide chain composed of the light chain variable domain VL and the heavy chain constant domain 1 CH1 (VL-CH1, in N-to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e. comprising a heavy chain composed of the heavy chain variable and constant domains (VH-CH1, in N- to C-terminal direction), and a light chain composed of the light chain variable and constant domains (VL-CL, in N- to C-terminal direction).

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable domain (VL) and an antibody heavy chain variable domain (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity. As used herein in connection with variable region sequences, "Kabat numbering" refers to the numbering system set forth by Kabat et al., Sequences of Proteins of Immunological Interest, 5th Ed. Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991), referred to as "numbering according to Kabat" or "Kabat numbering" herein. Specifically the Kabat numbering system (see pages 647-660 of Kabat, et al., Sequences of Proteins of Immunological Interest, 5th ed., Public Health Service, National Institutes of Health, Bethesda, MD (1991)) is used for the light chain constant domain CL of kappa and lambda isotype and the Kabat EU index numbering system (see pages 661-723) is used for the heavy chain constant domains (CH1, Hinge, CH2 and CH3), which is herein further clarified by referring to "numbering according to Kabat EU index" in this case.

The term "hypervariable region" or "HVR", as used herein, refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs; three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1(L1)-FR2-H2(L2)-FR3-H3(L3)-FR4.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. Such variable domains are referred to herein as "humanized variable region". A humanized antibody optionally may comprise at least a portion of an antibody constant region derived from a human antibody. In some embodiments, some FR residues in a humanized antibody are substituted with corresponding residues from a non-human antibody (e.g., the antibody from which the HVR residues are derived), e.g., to restore or improve antibody specificity or affinity. A "humanized form" of an antibody, e.g. of a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. A human antibody may bederived from a non-human transgenic mammal, for example a mouse, a rat, or a rabbit. In certain embodiments, a human antibody is derived from a hybridoma cell line. Antibodies or antibody fragments isolated from human antibody libraries are also considered human antibodies or human antibody fragments herein.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (K447), of the Fc region may or may not be present. Amino acid sequences of heavy chains including Fc domains (or a subunit of an Fc domain as defined herein) are denoted herein without C-terminal glycine-lysine dipeptide if not indicated otherwise. In one embodiment of the invention, a heavy chain including a subunit of an Fc domain as specified herein, comprised in an immunoconjugate according to the invention, comprises an additional C-terminal glycine-lysine dipeptide (G446 and K447, numbering according to EU index of Kabat). In one embodiment of the invention, a heavy chain including a subunit of an Fc domain as specified herein, comprised in an immunoconjugate according to the invention, comprises an additional C-terminal glycine residue (G446, numbering according to EU index of Kabat). Compositions of the invention, such as the pharmaceutical compositions described herein, comprise a population of immunoconjugates of the invention. The population of immunoconjugates may comprise molecules having a full-length heavy chain and molecules having a cleaved variant heavy chain. The population of immunoconjugates may consist of a mixture of molecules having a full-length heavy chain and molecules having a cleaved variant heavy chain, wherein at least 50%, at least 60%, at least 70%, at least 80% or at least 90% of the immunoconjugates have a cleaved variant heavy chain. In one embodiment of the invention, a composition comprising a population of immunoconjugates of the invention comprises an immunoconjugate comprising a heavy chain including a subunit of an Fc domain as specified herein with an additional C-terminal glycine-lysine dipeptide (G446 and K447, numbering according to EU index of Kabat). In one embodiment of the invention, a composition comprising a population of immunoconjugates of the invention comprises an immunoconjugate comprising a heavy chain including a subunit of an Fc domain as specified herein with an additional C-terminal glycine residue (G446, numbering according to EU index of Kabat). In one embodiment of the invention, such a composition comprises a population of immunoconjugates comprised of molecules comprising a heavy chain including a subunit of an Fc domain as specified herein; molecules comprising a heavy chain including a subunit of a Fc domain as specified herein with an additional C-terminal glycine residue (G446, numbering according to EU index of Kabat); and molecules comprising a heavy chain including a subunit of an Fc domain as specified herein with an additional C-terminal glycine-lysine dipeptide (G446 and K447, numbering according to EU index of Kabat). Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" when used in reference to antibodies refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced ADCC" is defined as either a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g. PCT publication no. WO 2006/082515 or PCT publication no. WO 2012/130831).

An "activating Fc receptor" is an Fc receptor that following engagement by an Fc domain of an antibody elicits signaling events that stimulate the receptor-bearing cell to perform effector functions. Human activating Fc receptors include FcγRIIIa (CD16a), FcγRI (CD64), FcγRIIa (CD32), and FcaRI (CD89).

As used herein, the terms "engineer, engineered, engineering", are considered to include any manipulation of

13

the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

"Reduced binding", for example reduced binding to an Fc receptor or CD25, refers to a decrease in affinity for the respective interaction, as measured for example by SPR. For clarity, the term includes also reduction of the affinity to zero (or below the detection limit of the analytic method), i.e. complete abolishment of the interaction. Conversely, "increased binding" refers to an increase in binding affinity for the respective interaction.

As used herein, the term "immunoconjugate" refers to a polypeptide molecule that includes at least one IL-2 molecule and at least one antibody. The IL-2 molecule can be joined to the antibody by a variety of interactions and in a variety of configurations as described herein. In particular embodiments, the IL-2 molecule is fused to the antibody via a peptide linker. Particular immunoconjugates according to the invention essentially consist of one IL-2 molecule and an antibody (such as a bispecific antigen binding molecule) joined by one or more linker sequences.

By "fused" is meant that the components (e.g. an antibody and an IL-2 molecule) are linked by peptide bonds, either directly or via one or more peptide linkers.

As used herein, the terms "first" and "second" with respect to Fc domain subunits etc., are used for convenience of distinguishing when there is more than one of each type of moiety. Use of these terms is not intended to confer a specific order or orientation of the immunoconjugate unless explicitly so stated.

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the composition would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. As disclosed herein, immunoconjugates of the invention may be used to delay development of a disease or to slow the progression of a disease.

**Technical Details**

Mutant IL-2 polypeptide

The immunoconjugates according to the present invention comprise a mutant IL-2 polypeptide as defined in the claims, having advantageous properties for immunotherapy. In particular, pharmacological properties of IL-2 that contribute to toxicity but are not essential for efficacy of IL-2 are eliminated in the mutant IL-2 polypeptide. Such mutant IL-2 polypeptides are described in detail in WO 2012/107417. As discussed above, different forms of the IL-2 receptor consist of different subunits and exhibit different affinities for IL-2. The intermediate-affinity IL-2 receptor, consisting of the β and γ receptor subunits, is expressed on resting effector cells and is sufficient for IL-2 signaling. The high-affinity IL-2 receptor, additionally comprising the α-subunit of the receptor, is mainly expressed on regulatory T (T$_{reg}$) cells as well as on activated effector cells where its engagement by IL-2 can promote T$_{reg}$ cell-mediated immunosuppression or activation-induced cell death (AICD), respectively. Thus, without wishing to be bound by theory, reducing or abolishing the affinity of IL-2 to the α-subunit of the IL-2 receptor should reduce IL-2 induced downregulation of effector cell function by regulatory T cells and development of tumor tolerance by the process of AICD. On the other hand, maintaining the affinity to the intermediate-affinity IL-2 receptor should preserve the induction of proliferation and activation of effector cells like NK and T cells by IL-2.

Mutants of human IL-2 (hIL-2) with decreased affinity to CD25 may for example be generated by amino acid substitution at amino acid position 35, 38, 42, 43, 45 or 72 or combinations thereof (numbering relative to the human IL-2 sequence SEQ ID NO: 22). Exemplary amino acid substitutions include K35E, K35A, R38A, R38E, R38N, R38F, R38S, R38L, R38G, R38Y, R38W, F42L, F42A, F42G, F42S, F42T, F42Q, F42E, F42N, F42D, F42R, F42K, K43E, Y45A, Y45G, Y45S, Y45T, Y45Q, Y45E, Y45N, Y45D, Y45R, Y45K, L72G, L72A, L72S, L72T, L72Q, L72E, L72N, L72D, L72R, and L72K. In one embodiment said amino acid mutations are F42A, Y45A and L72G. These mutants exhibit substantially similar binding affinity to the intermediate-affinity IL-2 receptor, and have substantially reduced affinity to the α-subunit of the IL-2 receptor and the high-affinity IL-2 receptor compared to a wild-type form of the IL-2 mutant.

Other characteristics of useful mutants may include the ability to induce proliferation of IL-2 receptor-bearing T and/or NK cells, the ability to induce IL-2 signaling in IL-2 receptor-bearing T and/or NK cells, the ability to generate interferon (IFN)-γ as a secondary cytokine by NK cells, a reduced ability to induce elaboration of secondary cytokines - particularly IL-10 and TNF-α-by peripheral blood mononuclear cells (PBMCs), a reduced ability to activate regulatory T cells, a reduced ability to induce apoptosis in T cells, and a reduced toxicity profile in vivo.

The particular mutant IL-2 polypeptides useful in the invention comprise three amino acid mutations that abolish or reduce affinity of the mutant IL-2 polypeptide to the α-subunit of the IL-2 receptor but preserve affinity of the mutant IL-2 polypeptide to the intermediate affinity IL-2 receptor. The three amino acid mutations are amino acid substitutions F42A, Y45A and L72G (numbering relative to the human IL-2 sequence of SEQ ID NO: 22).

In one embodiment said combination of amino acid mutations abolishes the affinity of the mutant IL-2 polypeptide to the α-subunit of the IL-2 receptor so that no binding is detectable by surface plasmon resonance.

Substantially similar binding to the intermediate-affinity receptor, i.e. preservation of the affinity of the mutant IL-2 polypeptide to said receptor, is achieved when the IL-2 mutant exhibits greater than about 70% of the affinity of a wild-type form of the IL-2 mutant to the intermediate-affinity IL-2 receptor. IL-2 mutants of the invention may exhibit greater than about 80% and even greater than about 90% of such affinity.

Reduction of the affinity of IL-2 for the α-subunit of the IL-2 receptor in combination with elimination of the O-glycosylation of IL-2 results in an IL-2 protein with improved properties. For example, elimination of the O-glycosylation site results in a more homogenous product when the mutant IL-2 polypeptide is expressed in mammalian cells such as CHO or HEK cells.

Thus, in certain embodiments the mutant IL-2 polypeptide comprises an additional amino acid mutation which eliminates the O-glycosylation site of IL-2 at a position corresponding to residue 3 of human IL-2. In one embodiment said additional amino acid mutation which eliminates the O-glycosylation site of IL-2 at a position corresponding to residue 3 of human IL-2 is an amino acid substitution. Exemplary amino acid substitutions include T3A, T3G, T3Q, T3E, T3N, T3D, T3R, T3K, and T3P. In a specific embodiment, said additional amino acid mutation is the amino acid substitution T3A.

In certain embodiments the mutant IL-2 polypeptide is essentially a full-length IL-2 molecule. In certain embodiments the mutant IL-2 polypeptide is a human IL-2 molecule. In one embodiment the mutant IL-2 polypeptide comprises the sequence of SEQ ID NO: 22 with at least one amino acid mutation that abolishes or reduces affinity of the mutant IL-2 polypeptide to the α-subunit of the IL-2 receptor but preserve affinity of the mutant IL-2 polypeptide to the intermediate affinity IL-2 receptor, compared to an IL-2 polypeptide comprising SEQ ID NO: 22 without said mutation. In another embodiment, the mutant IL-2 polypeptide comprises the sequence of SEQ ID NO: 39 with at least one amino acid mutation that abolishes or reduces affinity of the mutant IL-2 polypeptide to the α-subunit of the IL-2 receptor but preserve affinity of the mutant IL-2 polypeptide to the intermediate affinity IL-2 receptor, compared to an IL-2 polypeptide comprising SEQ ID NO: 39 without said mutation.

In a specific embodiment, the mutant IL-2 polypeptide can elicit one or more of the cellular responses selected from the group consisting of: proliferation in an activated T lymphocyte cell, differentiation in an activated T lymphocyte cell, cytotoxic T cell (CTL) activity, proliferation in an activated B cell, differentiation in an activated B cell, proliferation in a natural killer (NK) cell, differentiation in a NK cell, cytokine secretion by an activated T cell or an NK cell, and NK/lymphocyte activated killer (LAK) antitumor cytotoxicity.

In one embodiment the mutant IL-2 polypeptide has a reduced ability to induce IL-2 signaling in regulatory T cells, compared to a wild-type IL-2 polypeptide. In one embodiment the mutant IL-2 polypeptide induces less activation-induced cell death (AICD) in T cells, compared to a wild-type IL-2 polypeptide. In one embodiment the mutant IL-2 polypeptide has a reduced toxicity profile in vivo, compared to a wild-type IL-2 polypeptide. In one embodiment the mutant IL-2 polypeptide has a prolonged serum half-life, compared to a wild-type IL-2 polypeptide.

A particular mutant IL-2 polypeptide useful in the invention comprises four amino acid substitutions T3A, F42A, Y45A and L72G. As demonstrated in WO 2012/107417, said quadruple mutant IL-2 polypeptide exhibits no detectable binding to CD25, reduced ability to induce apoptosis in T cells, reduced ability to induce IL-2 signaling in T_reg cells, and a reduced toxicity profile in vivo. However, it retains ability to activate IL-2 signaling in effector cells, to induce proliferation of effector cells, and to generate IFN-γ as a secondary cytokine by NK cells.

Moreover, said mutant IL-2 polypeptide has further advantageous properties, such as reduced surface hydrophobicity, good stability, and good expression yield, as described in WO 2012/107417. Unexpectedly, said mutant IL-2 polypeptide also provides a prolonged serum half-life, compared to wild-type IL-2.

IL-2 mutants useful in the invention, in addition to having mutations in the region of IL-2 that forms the interface of IL-2 with CD25 or the glycosylation site, also may have one or more mutations in the amino acid sequence outside these regions. Such additional mutations in human IL-2 may provide additional advantages such as increased expression or stability. For example, the cysteine at position 125 may be replaced with a neutral amino acid such as serine, alanine, threonine or valine, yielding C125S IL-2, C125A IL-2, C125T IL-2 or C125V IL-2 respectively, as described in U.S. Patent no. 4,518,584. As described therein, one may also delete the N-terminal alanine residue of IL-2 yielding such mutants as des-A1 C125S or des-A1 C125A. Alternatively or conjunctively, the IL-2 mutant may include a mutation whereby methionine normally occurring at position 104 of wild-type human IL-2 is replaced by a neutral amino acid such as alanine (see U.S. Patent no. 5,206,344). The resulting mutants, e. g., des-A1 M104A IL-2, des-A1 M104A C125S IL-2, M104A IL-2, M104A C125A IL-2, des-A1 M104A C125A IL-2, or M104A C125S IL-2 (these and other mutants may be found in U.S. Patent No. 5,116,943 and in Weiger et al., Eur J Biochem 180, 295-300 (1989)) may be used in conjunction with the particular IL-2 mutations of the invention.

Thus, in certain embodiments the mutant IL-2 polypeptide comprises an additional amino acid mutation at a position corresponding to residue 125 of human IL-2. In one embodiment said additional amino acid mutation is the amino acid substitution C125A.

The skilled person will be able to determine which additional mutations may provide additional advantages for the purpose of the invention. For example, he will appreciate that amino acid mutations in the IL-2 sequence that reduce or abolish the affinity of IL-2 to the intermediate-affinity IL-2 receptor, such as D20T, N88R or Q126D (see e.g. US 2007/0036752), may not be suitable to include in the mutant IL-2 polypeptide according to the invention.

In one embodiment, the mutant IL-2 polypeptide comprises no more than 12, no more than 11, no more than 10, no more than 9, no more than 8, no more than 7, no more than 6, or no more than 5 amino acid mutations as compared to the corresponding wild-type IL-2 sequence, e.g. the human IL-2 sequence of SEQ ID NO: 22. In a particular embodiment, the mutant IL-2 polypeptide comprises no more than 5 amino acid mutations as compared to the corresponding wild-type IL-2 sequence, e.g. the human IL-2 sequence of SEQ ID NO: 22.

In one embodiment the mutant IL-2 polypeptide comprises the sequence of SEQ ID NO: 23. In one embodiment the mutant IL-2 polypeptide consists of the sequence of SEQ ID NO: 23.

Immunoconjugates

Immunoconjugates as described herein comprise an IL-molecule and a bispecific antigen binding molecule. Such immunoconjugates significantly increase the efficacy of IL-2 therapy by directly targeting IL-2 e.g. into a tumor microenvironment. According to the invention, an bispecific antigen binding molecule comprised in the immunoconjugate can be a whole antibody or immunoglobulin, or a portion or variant thereof that has a biological function such as antigen specific binding affinity.

The general benefits of immunoconjugate therapy are readily apparent. For example, a bispecific antigen binding molecule comprised in an immunoconjugate recognizes a tumor-specific epitope and results in targeting of the immunoconjugate molecule to the tumor site. Therefore, high concentrations of IL-2 can be delivered into the tumor microenvironment, thereby resulting in activation and proliferation of a variety of immune effector cells mentioned herein using a much lower dose of the immunoconjugate than would be required for unconjugated IL-2. Moreover, since application of IL-2 in form of immunoconjugates allows lower doses of the cytokine itself, the potential for undesirable side effects of IL-2 is restricted, and targeting the IL-2 to a specific site in the body by means of an immunoconjugate may also result in a reduction of systemic exposure and thus less side effects than obtained with unconjugated IL-2. In addition, the increased circulating half-life of an immunoconjugate compared to unconjugated IL-2 contributes to the efficacy of the immunoconjugate. However, this characteristic of IL-2 immunoconjugates may again aggravate potential side effects of the IL-2 molecule: Because of the significantly longer circulating half-life of IL-2 immunoconjugate in the bloodstream relative to unconjugated IL-2, the probability for IL-2 or other portions of the fusion protein molecule to activate components generally present in the vasculature is increased. The same concern applies to other fusion proteins that contain IL-2 fused to another moiety such as Fc or albumin, resulting in an extended half-life of IL-2 in the circulation. Therefore an immunoconjugate comprising a mutant IL-2 polypeptide as described herein and in WO 2012/107417, with reduced toxicity compared to wild-type forms of IL-2, is particularly advantageous.

As described hereinabove, targeting IL-2 directly to immune effector cells rather than tumor cells may be advantageous for IL-2 immunotherapy.

Accordingly, the invention provides a mutant IL-2 polypeptide as described hereinbefore, and a bispecific antigen binding molecule that binds to PD-1 and Tim-3. In one embodiment the immunoconjugate comprises not more than one mutant IL-2 polypeptide. In one embodiment the mutant IL-2 polypeptide and the bispecific antigen binding molecule form a fusion protein, i.e. the mutant IL-2 polypeptide shares a peptide bond with the bispecific antigen binding molecule. In some embodiments, the bispecific antigen binding molecule comprises an Fc domain composed of a first and a second subunit. In a specific embodiment the mutant IL-2 polypeptide is fused at its amino-terminal amino acid to the carboxy-terminal amino acid of one of the subunits of the Fc domain, optionally through a linker peptide. In some embodiments, the bispecific antigen binding molecule is a full-length antibody. In some embodiments, the bispecific antigen binding molecule is an immunoglobulin molecule, particularly an IgG class immunoglobulin molecule, more particularly an IgG$_1$ subclass immunoglobulin molecule, wherein in one of the Fab molecules (binding arms) the variable or constant regions of the heavy and light chain (VH/VL or CH1/CL, respectively) are exchanged / replaced by each other. In one such embodiment, the mutant IL-2 polypeptide shares an amino-terminal peptide bond with one of the immunoglobulin heavy chains. In certain embodiments the bispecific antigen binding molecule is an antibody fragment. In some embodiments the bispecific antigen binding molecule comprises a Fab molecule or a scFv molecule. In one embodiment the bispecific antigen binding molecule comprises a Fab molecule. In another embodiment the bispecific antigen binding molecule comprises a scFv molecule. The immunoconjugates of the invention comprise a first and a second antigen binding moiety. Each antigen binding moiety can be independently selected from various forms of antibodies and antibody fragments. For example, the first antigen binding moiety can be a Fab molecule and the second antigen binding moiety can be a scFv molecule. In a specific embodiment each of said first and said second antigen binding moieties is a scFv molecule or each of said first and said second antigen binding moieties is a Fab molecule. In a particular embodiment each of said first and said second antigen binding moieties is a Fab molecule.

*Immunoconjugate Formats*

Exemplary immunoconjugate formats are described in PCT publication no. WO 2011/020783. These immunoconjugates comprise at least two antigen binding moieties. Thus, in one embodiment, the immunoconjugate according to the present invention comprises a mutant IL-2 polypeptide as described herein, and at least a first and a second antigen binding moiety. In a particular embodiment, said first and second antigen binding moiety are independently selected from the group consisting of an Fv molecule, particularly a scFv molecule, and a Fab molecule. In a specific embodiment, said mutant IL-2 polypeptide shares an amino- or carboxy-terminal peptide bond with said first antigen binding moiety and said second antigen binding moiety shares an amino- or carboxy-terminal peptide bond with either i) the mutant IL-2 polypeptide or ii) the first antigen binding moiety. In a particular embodiment, the immunoconjugate consists essentially of a mutant IL-2 polypeptide and first and second antigen binding moiety, particularly Fab molecules, joined by one or more linker sequences. Such formats have the advantage that they bind with high affinity to the target antigen (PD-1 and Tim-3), but provide only monomeric binding to the IL-2 receptor, thus avoiding targeting the immunoconjugate to IL-2 receptor bearing immune cells at other locations than the target site. In a particular embodiment, a mutant IL-2 polypeptide shares a carboxy-terminal peptide bond with a first antigen binding moiety, particularly a first Fab molecule, and further shares an amino-terminal peptide bond with a second antigen binding moiety, particularly a second Fab molecule. In another embodiment, a first antigen binding moiety, particularly a first Fab molecule, shares a carboxy-terminal peptide bond with a mutant IL-2 polypeptide, and further shares an amino-terminal peptide bond with a second antigen binding moiety, particularly a second Fab molecule. In another embodiment, a first antigen binding moiety, particularly a first Fab molecule, shares an amino-terminal peptide bond with a first mutant IL-2 polypeptide, and further shares a carboxy-terminal peptide with a second antigen binding moiety, particularly a second Fab molecule. In a particular embodiment, a mutant IL-2 polypeptide shares a carboxy-terminal peptide bond with a first heavy chain variable region and further shares an amino-terminal peptide bond with a second heavy chain variable region. In another embodiment a mutant IL-2 polypeptide shares a carboxy-terminal peptide bond with a first light chain variable region and further shares an amino-terminal peptide bond with a second light chain variable region. In another embodiment, a first heavy or light chain variable region is joined by a carboxy-terminal peptide bond to a mutant IL-2 polypeptide and is further joined by an amino-terminal peptide bond to a second heavy or light chain variable region. In another embodiment, a first heavy or light chain variable region is joined by an amino-terminal peptide bond to a mutant IL-2 polypeptide and is further joined by a carboxy-terminal peptide bond to a second heavy or light chain variable region. In one embodiment, a mutant IL-2 polypeptide shares a carboxy-terminal peptide bond with a first Fab heavy or light chain and further shares an amino-terminal peptide bond with a second Fab heavy or light chain. In another embodiment, a first Fab heavy or light chain shares a carboxy-terminal peptide bond with a mutant IL-2 polypeptide and further shares an amino-terminal peptide bond with a second Fab heavy or light chain. In other embodiments, a first Fab heavy or light chain shares an amino-terminal peptide bond with a mutant IL-2 polypeptide and further shares a carboxy-terminal peptide bond with a second Fab heavy or light chain. In one embodiment, the immunoconjugate comprises a mutant IL-2 polypeptide sharing an amino-terminal peptide bond with one or more scFv molecules and further sharing a carboxy-terminal peptide bond with one or more scFv molecules.

Other exemplary immunconjugate formats are described in PCT publication no. WO 2012/146628. These immunoconjugates comprise an immunoglobulin molecule as antigen binding moiety.

The immunoconjugate of the present invention, in particular embodiments, comprises a mutant IL-2 polypeptide as described herein and a bispecific antigen binding molecule that binds to PD-1 and Tim-3, wherein the bispecific antigen binding molecule is an immunoglobulin molecule, particularly an IgG molecule, more particularly an IgG$_1$ molecule, wherein in one of the Fab molecules (binding arms) the variable or constant regions of the heavy and light chain (VH/VL or CH1/CL, respectively) are exchanged / replaced by each other. In one embodiment the immunoglobulin molecule is human. In one embodiment, the immunoglobulin molecule comprises a human constant region, e.g. a human CH1, CH2, CH3 and/or CL domain. In one embodiment, the immunoglobulin comprises a human Fc domain, particularly a human IgG$_1$ Fc domain. In one embodiment the mutant IL-2 polypeptide shares an amino- or carboxy-terminal peptide bond with the immunoglobulin molecule. In one embodiment, the immunoconjugate essentially consists of the mutant IL-2 polypeptide and the immunoglobulin molecule, particularly IgG molecule, more particularly IgG$_1$ molecule, joined by one or more linker peptides. In a specific embodiment the mutant IL-2 polypeptide is fused at its amino-terminal amino acid to the carboxy-terminal amino acid of one of the immunoglobulin heavy chains, optionally through a linker peptide.

The mutant IL-2 polypeptide may be fused to the bispecific antigen binding molecule directly or through a linker peptide, comprising one or more amino acids, typically about 2-20 amino acids. Linker peptides are known in the art and are described herein. Suitable, non-immunogenic linker peptides include, for example, (G$_4$S)n, (SG$_4$)n, (G$_4$S)n or G$_4$(SG$_4$)n linker peptides. "n" is generally an integer from 1 to 10, typically from 2 to 4. In one embodiment the linker peptide has a length of at least 5 amino acids, in one embodiment a length of 5 to 100, in a further embodiment of 10 to 50 amino acids. In a particular embodiment, the linker peptide has a length of 15 amino acids. In one embodiment the linker peptide is (GxS)n or (GxS)$_n$G$_m$ with G=glycine, S=serine, and (x=3, n= 3, 4, 5 or 6, and m=0, 1, 2 or 3) or (x=4, n=2, 3, 4 or 5 and m= 0, 1, 2 or 3), in one embodiment x=4 and n=2 or 3, in a further embodiment x=4 and n=3. In a particular embodiment the linker peptide is (G$_4$S)$_3$ (SEQ ID NO: 24). In one embodiment, the linker peptide has (or consists of) the amino acid sequence of SEQ ID NO: 24.

In a particular embodiment, the immunoconjugate comprises a mutant IL-2 molecule and a bispecific antigen binding molecule that binds to PD-1 and Tim-3, wherein the bispecific antigen binding molecule is an immunoglobulin molecule, particularly an IgG class immunoglobulin molecule, more particularly an IgG$_1$ subclass immunoglobulin molecule, wherein in one of the Fab molecules (binding arms) the variable or constant regions of the heavy and light chain (VH/VL or CH1/CL, respectively) are exchanged / replaced by each other, wherein the mutant IL-2 molecule is fused at its amino-terminal amino acid to the carboxy-terminal amino acid of one of the immunoglobulin heavy chains through the linker peptide of SEQ ID NO: 24.

In a particular embodiment, the immunoconjugate comprises a mutant IL-2 molecule and an bispecific antigen binding molecule that binds to PD-1 and Tim-3, wherein the bispecific antigen binding molecule comprises an Fc domain, particularly a human IgG$_1$ Fc domain, composed of a first and a second subunit, and the mutant IL-2 molecule is fused at its amino-terminal amino acid to the carboxy-terminal amino acid of one of the subunits of the Fc domain through the linker peptide of SEQ ID NO: 24.

Bispecific antigen binding molecules that bind to PD-1 and Tim-3

The bispecific antigen binding molecule comprised in the immunoconjugate of the invention binds to PD-1 and Tim-3, particularly human PD-1 and human Tim-3, and is able to direct the mutant IL-2 polypeptide to a target site where PD-1 and/or Tim-3 is expressed, particularly to a T cell that expresses PD-1 and/or Tim-3, for example associated with a tumor.

Suitable PD-1/Tim-3 bispecific antigen binding molecules that may be used in the immunoconjugate of the invention are described in PCT patent application no. PCT/EP2016/073192.

According to particular embodiments of the invention, the antigen binding moieties comprised in the bispecific antigen binding molecule are Fab molecules (i.e. antigen binding domains composed of a heavy and a light chain, each comprising a variable and a constant domain). In one embodiment, the first and/or the second antigen binding moiety is a Fab molecule. In one embodiment, said Fab molecule is human. In a particular embodiment, said Fab molecule is humanized. In yet another embodiment, said Fab molecule comprises human heavy and light chain constant domains.

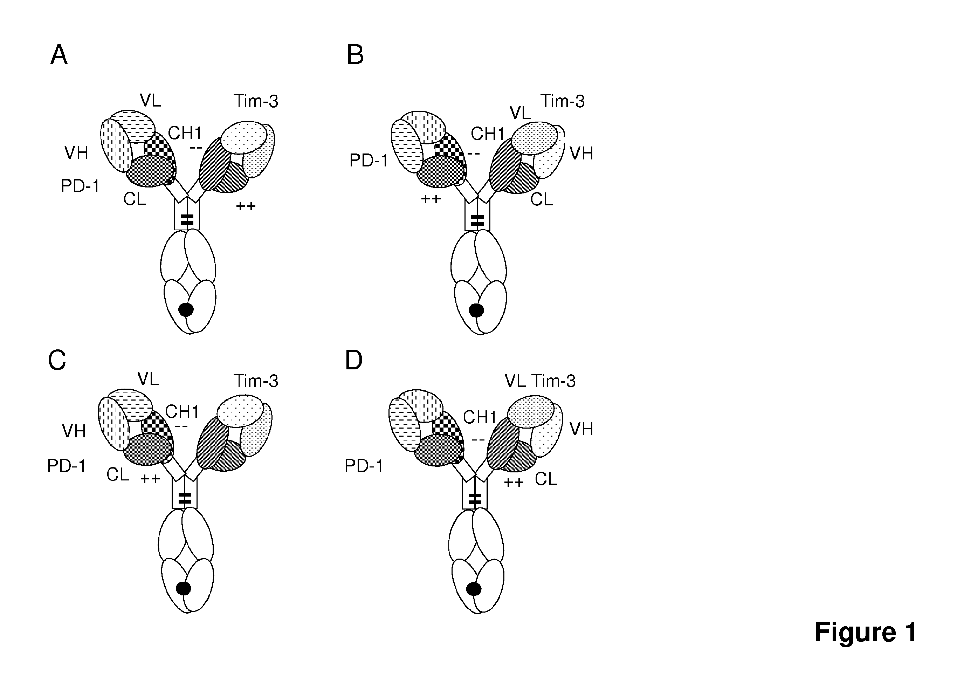

Preferably, at least one of the antigen binding moieties is a crossover Fab molecule. Such modification reduces mispairing of heavy and light chains from different Fab molecules, thereby improving the yield and purity of the bispecific antigen binding molecule in recombinant production. In a particular crossover Fab molecule useful for the bispecific antigen binding molecule comprised in the immunoconjugate of the invention, the variable domains of the Fab light chain and the Fab heavy chain (VL and VH, respectively) are exchanged. Even with this domain exchange, however, the preparation of the bispecific antigen binding molecule may comprise certain side products due to a so-called Bence Jones-type interaction between mispaired heavy and light chains (see Schaefer et al, PNAS, 108 (2011) 11187-11191). To further reduce mispairing of heavy and light chains from different Fab molecules and thus increase the purity and yield of the desired bispecific antigen binding molecule, charged amino acids with opposite charges may be introduced at specific amino acid positions in the CH1 and CL domains of either the Fab molecule binding to PD-1, or the Fab molecule binding to Tim-3, as further described herein. Charge modifications are made either in the conventional Fab molecule comprised in the bispecific antigen binding molecule (such as shown e.g. in **Figure 1A, B),** or in the (VH/VL) crossover Fab molecule comprised in the bispecific antigen binding molecule (such as shown e.g. in **Figure 1C, D**) (but not in both). In particular embodiments, the charge modifications are made in the conventional Fab molecule comprised in the bispecific antigen binding molecule (which in particular embodiments binds to Tim-3).

*First antigen binding moiety*

The bispecific antigen binding molecule comprised in the immunoconjugate of the invention comprises at least one antigen binding moiety, particularly a Fab molecule, that binds to PD-1, particularly human PD-1 (first antigen). In particular embodiments, the antigen binding moiety that binds to PD-1 is a crossover Fab molecule as described herein, i.e. a Fab molecule wherein the variable domains VH and VL or the constant domains CH1 and CL of the Fab heavy and light chains are exchanged / replaced by each other. In such embodiments, the antigen binding moiety that binds to Tim-3 is a conventional Fab molecule (such as shown e.g. in **Figure 1A, C**). In alternative embodiments, the antigen binding moiety which binds to Tim-3 is a crossover Fab molecule as described herein, i.e. a Fab molecule wherein the variable domains VH and VL or the constant domains CH1 and CL of the Fab heavy and light chains are exchanged / replaced by each other. In such embodiments, the antigen binding moiety that binds to PD-1 is a conventional Fab molecule (such as shown e.g. in **Figure 1B, D**).

In some embodiments, the first antigen binding moiety comprises a HVR-H1 comprising the amino acid sequence of SEQ ID NO:1, a HVR-H2 comprising the amino acid sequence of SEQ ID NO:2, a HVR-H3 comprising the amino acid sequence of SEQ ID NO:3, a HVR-L1 comprising the amino acid sequence of SEQ ID NO:4, a HVR-L2 comprising the amino acid sequence of SEQ ID NO:5, and a HVR-L3 comprising the amino acid sequence of SEQ ID NO:6. In some embodiments, the first antigen binding moiety comprises (a) a heavy chain variable region (VH) comprising a HVR-H1 comprising the amino acid sequence of SEQ ID NO:1, a HVR-H2 comprising the amino acid sequence of SEQ ID NO:2, and a HVR-H3 comprising the amino acid sequence of SEQ ID NO:3, and (b) a light chain variable region (VL) comprising a HVR-L1 comprising the amino acid sequence of SEQ ID NO:4, a HVR-L2 comprising the amino acid sequence of SEQ ID NO:5, and a HVR-L3 comprising the amino acid sequence of SEQ ID NO:6.

In some embodiments, the first antigen binding moiety is (derived from) a humanized antibody. In one embodiment, the VH is a humanized VH and/or the VL is a humanized VL. In one embodiment, the first antigen binding moiety comprises HVRs as in any of the above embodiments, and further comprises an acceptor human framework, e.g. a human immunoglobulin framework or a human consensus framework. In some embodiments, the heavy and/or light chain variable region comprises human framework regions (FR).

In some embodiments, the first antigen binding moiety comprises (a) a heavy chain variable region (VH) comprising an amino acid sequence that is at least about 95%, 96%, 97%, 98%, 99% or 100% identical to the amino acid sequence of SEQ ID NO:7. In some embodiments, the first antigen binding moiety comprises a light chain variable region (VL) comprising an amino acid sequence that is at least about 95%, 96%, 97%, 98%, 99% or 100% identical to an amino acid sequence selected from the group consisting of SEQ ID NO:8, SEQ ID NO:9, SEQ ID NO: 10, and SEQ ID NO:11. In some embodiments, the first antigen binding moiety comprises (a) a heavy chain variable region (VH) comprising an amino acid sequence that is at least about 95%, 96%, 97%, 98%, 99% or 100% identical to the amino acid sequence of SEQ ID NO:7, and (b) a light chain variable region (VL) comprising an amino acid sequence that is at least about 95%, 96%, 97%, 98%, 99% or 100% identical to an amino acid sequence selected from the group consisting of SEQ ID NO:8, SEQ ID NO:9, SEQ ID NO: 10, and SEQ ID NO:11.

In some embodiments, the first antigen binding moiety comprises a VH sequence that is at least about 95%, 96%, 97%, 98%, 99% or 100% identical to the amino acid sequence of SEQ ID NO: 7, and a VL sequence that is at least about 95%, 96%, 97%, 98%, 99% or 100% identical to an amino acid sequence selected from the group of SEQ ID NO: 8, SEQ ID NO: 9, SEQ ID NO: 10, and SEQ ID NO: 11.

In some embodiments, the first antigen binding moiety comprises (a) a heavy chain variable region (VH) comprising the amino acid sequence of SEQ ID NO:7, and (b) a light chain variable region (VL) comprising an amino acid sequence selected from the group consisting of SEQ ID NO:8, SEQ ID NO:9, SEQ ID NO: 10, and SEQ ID NO:11.

In some embodiments, the first antigen binding moiety comprises the VH sequence of SEQ ID NO: 7, and a VL sequence selected from the group of SEQ ID NO: 8, SEQ ID NO: 9, SEQ ID NO: 10, and SEQ ID NO: 11.

In a particular embodiment, the first antigen binding moiety comprises a VH comprising the amino acid sequence of SEQ ID NO: 7 and a VL comprising the amino acid sequence of SEQ ID NO: 8.

In a particular embodiment, the first antigen binding moiety comprises the VH sequence of SEQ ID NO: 7 and the VL sequence of SEQ ID NO: 8.

In one embodiment, the first antigen binding moiety comprises a human constant region. In one embodiment, the first antigen binding moiety is a Fab molecule comprising a human constant region, particularly a human CH1 and/or CL domain. Exemplary sequences of human constant domains are given in SEQ ID NOs 41 and 42 (human kappa and lambda CL domains, respectively) and SEQ ID NO: 43 (human IgG$_1$ heavy chain constant domains CH1-CH2-CH3). In some embodiments, the first antigen binding moiety comprises a light chain constant region comprising an amino acid sequence that is at least about 95%, 96%, 97%, 98%, 99% or 100% identical to the amino acid sequence of SEQ ID NO: 41 or SEQ ID NO: 42, particularly the amino acid sequence of SEQ ID NO: 41. Particularly, the light chain constant region may comprise amino acid mutations as described herein under "charge modifications" and/or may comprise deletion or substitutions of one or more (particularly two) N-terminal amino acids if in a crossover Fab molecule. In some embodiments, the first antigen binding moiety comprises a heavy chain constant region comprising an amino acid sequence that is at least about 95%, 96%, 97%, 98%, 99% or 100% identical to the CH1 domain sequence comprised in the amino acid sequence of SEQ ID NO: 43. Particularly, the heavy chain constant region (specifically CH1 domain) may comprise amino acid mutations as described herein under "charge modifications".

In one embodiment, not more than one antigen binding moiety that binds to PD-1 is present in the bispecific antigen binding molecule (i.e. the bispecific antigen binding molecule provides monovalent binding to PD-1).

*Second antigen binding moiety*

The bispecific antigen binding molecule comprised in the immunoconjugate of the invention comprises at least one antigen binding moiety, particularly a Fab molecule, that binds to Tim-3, particularly human Tim-3 (second antigen).

In particular embodiments, the antigen binding moiety that binds to Tim-3 is a conventional Fab molecule. In such embodiments, the antigen binding moiety that binds to PD-1 is preferably a crossover Fab molecule as described herein, i.e. a Fab molecule wherein the variable domains VH and VL or the constant domains CH1 and CL of the Fab heavy and light chains are exchanged / replaced by each other (see e.g. **Figure 1A, C**).

In alternative embodiments, the antigen binding moiety that binds to PD-1 is a conventional Fab molecule. In such embodiments, the antigen binding moiety that binds to Tim-3 is a crossover Fab molecule as described herein, i.e. a Fab molecule wherein the variable domains VH and VL or the constant domains CH1 and CL of the Fab heavy and light chains are exchanged / replaced by each other (see e.g. **Figure 1B, D)**

In some embodiments, the second antigen binding moiety comprises a HVR-H1 comprising the amino acid sequence of SEQ ID NO:12, a HVR-H2 comprising the amino acid sequence of SEQ ID NO:13, a HVR-H3 comprising the amino acid sequence of SEQ ID NO:14, a HVR-L1 comprising the amino acid sequence of SEQ ID NO:15, a HVR-L2 comprising the amino acid sequence of SEQ ID NO:16, and a HVR-L3 comprising the amino acid sequence of SEQ ID NO:17.

In some embodiments, the second antigen binding moiety comprises (a) a heavy chain variable region (VH) comprising a HVR-H1 comprising the amino acid sequence of SEQ ID NO:12, a HVR-H2 comprising the amino acid sequence of SEQ ID NO:13, and a HVR-H3 comprising the amino acid sequence of SEQ ID NO:14, and (b) a light chain variable region (VL) comprising a HVR-L1 comprising the amino acid sequence of SEQ ID NO: 15, a HVR-L2 comprising the amino acid sequence of SEQ ID NO:16, and a HVR-L3 comprising the amino acid sequence of SEQ ID NO:17.

In some embodiments, the second antigen binding moiety is (derived from) a humanized antibody. In one embodiment, the VH is a humanized VH and/or the VL is a humanized VL. In one embodiment, the second antigen binding moiety comprises HVRs as in any of the above embodiments, and further comprises an acceptor human framework, e.g. a human immunoglobulin framework or a human consensus framework. In some embodiments, the heavy and/or light chain variable region comprises human framework regions (FR).

In some embodiments, the second antigen binding moiety comprises a heavy chain variable region (VH) comprising an amino acid sequence that is at least about 95%, 96%, 97%, 98%, 99% or 100% identical to the amino acid sequence of SEQ ID NO:18 or SEQ ID NO: 20. In some embodiments, the second antigen binding moiety comprises a light chain variable region (VL) comprising an amino acid sequence that is at least about 95%, 96%, 97%, 98%, 99% or 100% identical to the amino acid sequence of SEQ ID NO:19 or SEQ ID NO: 21. In some embodiments, the second antigen binding moiety comprises (a) a heavy chain variable region (VH) comprising an amino acid sequence that is at least about 95%, 96%, 97%, 98%, 99% or 100% identical to the amino acid sequence of SEQ ID NO:18, and (b) a light chain variable region (VL) comprising an amino acid sequence that is at least about 95%, 96%, 97%, 98%, 99% or 100% identical to the amino acid sequence of SEQ ID NO:19. In other embodiments, the second antigen binding moiety comprises (a) a heavy chain variable region (VH) comprising an amino acid sequence that is at least about 95%, 96%, 97%, 98%, 99% or 100% identical to the amino acid sequence of SEQ ID NO:20, and (b) a light chain variable region (VL) comprising an amino acid sequence that is at least about 95%, 96%, 97%, 98%, 99% or 100% identical to the amino acid sequence of SEQ ID NO:21.

In some embodiments, the second antigen binding moiety comprises a VH sequence that is at least about 95%, 96%, 97%, 98%, 99% or 100% identical to the amino acid sequence of SEQ ID NO: 18, and a VL sequence that is at least about 95%, 96%, 97%, 98%, 99% or 100% identical to the amino acid sequence of SEQ ID NO: 19. In some embodiments, the second antigen binding moiety comprises a VH sequence that is at least about 95%, 96%, 97%, 98%, 99% or 100% identical to the amino acid sequence of SEQ ID NO: 20, and a VL sequence that is at least about 95%, 96%, 97%, 98%, 99% or 100% identical to the amino acid sequence of SEQ ID NO: 21.

In a particular embodiment, the second antigen binding moiety comprises (a) a heavy chain variable region (VH) comprising the amino acid sequence of SEQ ID NO:18, and (b) a light chain variable region (VL) comprising the amino acid sequence of SEQ ID NO:19. In a further particular embodiment, the second antigen binding moiety comprises the VH sequence of SEQ ID NO: 18, and the VL sequence of SEQ ID NO: 19.

In one embodiment, the second antigen binding moiety comprises a VH comprising the amino acid sequence of SEQ ID NO: 20 and a VL comprising the amino acid sequence of SEQ ID NO: 21. In a further embodiment, the second antigen binding moiety comprises the VH sequence of SEQ ID NO: 20 and the VL sequence of SEQ ID NO: 21.

In one embodiment, the second antigen binding moiety comprises a human constant region. In one embodiment, the second antigen binding moiety is a Fab molecule comprising a human constant region, particularly a human CH1 and/or CL domain. Exemplary sequences of human constant domains are given in SEQ ID NOs 41 and 42 (human kappa and lambda CL domains, respectively) and SEQ ID NO: 43 (human IgG$_1$ heavy chain constant domains CH1-CH2-CH3). In some embodiments, the second antigen binding moiety comprises a light chain constant region comprising an amino acid sequence that is at least about 95%, 96%, 97%, 98%, 99% or 100% identical to the amino acid sequence of SEQ ID NO: 41 or SEQ ID NO: 42, particularly the amino acid sequence of SEQ ID NO: 41. Particularly, the light chain constant region may comprise amino acid mutations as described herein under "charge modifications" and/or may comprise deletion or substitutions of one or more (particularly two) N-terminal amino acids if in a crossover Fab molecule. In some embodiments, the first antigen binding moiety comprises a heavy chain constant region comprising an amino acid sequence that is at least about 95%, 96%, 97%, 98%, 99% or 100% identical to the CH1 domain sequence comprised in the amino acid sequence of SEQ ID NO: 43. Particularly, the heavy chain constant region (specifically CH1 domain) may comprise amino acid mutations as described herein under "charge modifications".

In one embodiment, not more than one antigen binding moiety that binds to Tim-3 is present in the bispecific antigen binding molecule (i.e. the bispecific antigen binding molecule provides monovalent binding to Tim-3).

*Charge modifications*

The bispecific antigen binding molecule comprised in the immunconjugate of the invention may comprise amino acid substitutions in Fab molecules comprised therein which are particularly efficient in reducing mispairing of light chains with non-matching heavy chains (Bence-Jones-type side products), which can occur in the production of Fab-based bi-/multispecific antigen binding molecules with a VH/VL exchange in one (or more, in case of molecules comprising more than two antigen-binding Fab molecules) of their binding arms (see also PCT publication no. WO 2015/150447, particularly the examples therein). The ratio of a desired bispecific antigen binding molecule compared to undesired side products, in particular Bence Jones-type side products occurring in bispecific antigen binding molecules with a VH/VL domain exchange in one of their binding arms, can be improved by the introduction of charged amino acids with opposite charges at specific amino acid positions in the CH1 and CL domains (sometimes referred to herein as "charge modifications").

Accordingly, in some embodiments wherein the first and the second antigen binding moiety of the bispecific antigen binding molecule are both Fab molecules, and in one of the antigen binding moieties (particularly the first antigen binding moiety) the variable domains VL and VH of the Fab light chain and the Fab heavy chain are replaced by each other,

i) in the constant domain CL of the second antigen binding moiety the amino acid at position 124 is substituted by a positively charged amino acid (numbering according to Kabat), and wherein in the constant domain CH1 of the second antigen binding moiety the amino acid at position 147 or the amino acid at position 213 is substituted by a negatively charged amino acid (numbering according to Kabat EU index); or

ii) in the constant domain CL of the first antigen binding moiety the amino acid at position 124 is substituted by a positively charged amino acid (numbering according to Kabat), and wherein in the constant domain CH1 of the first antigen binding moiety the amino acid at position 147 or the amino acid at position 213 is substituted by a negatively charged amino acid (numbering according to Kabat EU index).

The bispecific antigen binding molecule does not comprise both modifications mentioned under i) and ii). The constant domains CL and CH1 of the antigen binding moiety having the VH/VL exchange are not replaced by each other (i.e. remain unexchanged).

In a more specific embodiment,

i) in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 or the amino acid at position 213 is substituted independently by glutamic acid (E), or aspartic acid (D) (numbering according to Kabat EU index); or

ii) in the constant domain CL of the first antigen binding moiety the amino acid at position 124 is substituted independently by lysine (K), arginine (R) or histidine (H) (numbering according to Kabat), and in the constant domain CH1 of the first antigen binding moiety the amino acid at position 147 or the amino acid at position 213 is substituted independently by glutamic acid (E), or aspartic acid (D) (numbering according to Kabat EU index).

In one such embodiment, in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 or the amino acid at position 213 is substituted independently by glutamic acid (E), or aspartic acid (D) (numbering according to Kabat EU index).

In a further embodiment, in the constant domain CL of the second antigen binding moiety the amino acid at position 124 is substituted independently by lysine (K), arginine (R) or histidine (H) (numbering according to Kabat), and in the constant domain CH1 of the second antigen binding moiety the amino acid at position 147 is substituted independently by glutamic acid (E), or aspartic acid (D) (numbering according to Kabat EU index).

In a particular embodiment, in the constant domain CL of the second antigen binding moiety the amino acid at position 124 is substituted independently by lysine (K), arginine (R) or histidine (H) (numbering according to Kabat) and the amino acid at position 123 is substituted independently by lysine (K), arginine (R) or histidine (H) (numbering according to Kabat), and in the constant domain CH1 of the second antigen binding moiety the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In a more particular embodiment, in the constant domain CL of the second antigen binding moiety the amino acid at position 124 is substituted by lysine (K) (numbering according to Kabat) and the amino acid at position 123 is substituted by lysine (K) (numbering according to Kabat), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In an even more particular embodiment, in the constant domain CL of the second antigen binding moiety the amino acid at position 124 is substituted by lysine (K) (numbering according to Kabat) and the amino acid at position 123 is substituted by arginine (R) (numbering according to Kabat), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In particular embodiments, if amino acid substitutions according to the above embodiments are made in the constant domain CL and the constant domain CH1 of the second antigen binding moiety, the constant domain CL of the second antigen binding moiety is of kappa isotype.

Alternatively, the amino acid substitutions according to the above embodiments may be made in the constant domain CL and the constant domain CH1 of the first antigen binding moiety instead of in the constant domain CL and the constant domain CH1 of the second antigen binding moiety. In particular such embodiments, the constant domain CL of the first antigen binding moiety is of kappa isotype.

Accordingly, in one embodiment, in the constant domain CL of the first antigen binding moiety the amino acid at position 124 is substituted independently by lysine (K), arginine (R) or histidine (H) (numbering according to Kabat), and in the constant domain CH1 of the first antigen binding moiety the amino acid at position 147 or the amino acid at position 213 is substituted independently by glutamic acid (E), or aspartic acid (D) (numbering according to Kabat EU index).

In a further embodiment, in the constant domain CL of the first antigen binding moiety the amino acid at position 124 is substituted independently by lysine (K), arginine (R) or histidine (H) (numbering according to Kabat), and in the constant domain CH1 of the first antigen binding moiety the amino acid at position 147 is substituted independently by glutamic acid (E), or aspartic acid (D) (numbering according to Kabat EU index).

In still another embodiment, in the constant domain CL of the first antigen binding moiety the amino acid at position 124 is substituted independently by lysine (K), arginine (R) or histidine (H) (numbering according to Kabat) and the amino acid at position 123 is substituted independently by lysine (K), arginine (R) or histidine (H) (numbering according to Kabat), and in the constant domain CH1 of the first antigen binding moiety the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In one embodiment, in the constant domain CL of the first antigen binding moiety the amino acid at position 124 is substituted by lysine (K) (numbering according to Kabat) and the amino acid at position 123 is substituted by lysine (K) (numbering according to Kabat), and in the constant domain CH1 of the first antigen binding moiety the amino acid at position 147 is substituted by glutamic acid (E) (numbering according to Kabat EU index) and the amino acid at position 213 is substituted by glutamic acid (E) (numbering according to Kabat EU index).

In another embodiment, in the constant domain CL of the first antigen binding moiety the amino acid at position 124 is substituted by lysine (K) (numbering according to Kabat) and the amino acid at position 123 is substituted by arginine (R) (numbering according to Kabat), and in the constant domain CH1 of the first antigen binding moiety the amino acid at position 147 is substituted by glutamic acid (E) (numbering according to Kabat EU index) and the amino acid at position 213 is substituted by glutamic acid (E) (numbering according to Kabat EU index).

In a particular embodiment, the bispecific antigen binding molecule comprised in the immunoconjugate of the invention comprises

(a) a first antigen binding moiety that binds to PD-1, wherein the first antigen binding moiety is a Fab molecule wherein the variable domains VL and VH of the Fab light chain and the Fab heavy chain are replaced by each other, and

(b) a second antigen binding moiety that binds to Tim-3, wherein the second antigen binding moiety is a (conventional) Fab molecule;

wherein in the constant domain CL of the second antigen binding moiety the amino acid at position 124 is substituted by lysine (K) (numbering according to Kabat) and the amino acid at position 123 is substituted by arginine (R) (numbering according to Kabat), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In a particular embodiment, the bispecific antigen binding molecule comprised in the immunoconjugate of the invention comprises

(a) a first antigen binding moiety that binds to PD-1, wherein the first antigen binding moiety is a Fab molecule wherein the variable domains VL and VH of the Fab light chain and the Fab heavy chain are replaced by each other, and

(b) a second antigen binding moiety that binds to Tim-3, wherein the second antigen binding moiety is a (conventional) Fab molecule;

wherein in the constant domain CL of the second antigen binding moiety the amino acid at position 124 is substituted independently by lysine (K), arginine (R) or histidine (H) (numbering according to Kabat) (in a particular embodiment independently by lysine (K) or arginine (R)) and the amino acid at position 123 is substituted independently by lysine (K), arginine (R) or histidine (H) (numbering according to Kabat) (in a particular embodiment independently by lysine (K) or arginine (R)), and in the constant domain CH1 of the second antigen binding moiety the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

*Bispecific antigen binding molecule formats*

The components of the bispecific antigen binding molecule comprised in the immunoconjugate according to the present invention can be fused to each other in a variety of configurations. Exemplary configurations are depicted in **Figure 1.** Suitable bispecific antigen binding molecule formats are described in PCT publication nos. WO2009080252 and WO2009080253.

In particular embodiments, the antigen binding moieties comprised in the bispecific antigen binding molecule are Fab molecules. In such embodiments, the first, second, etc. antigen binding moiety may be referred to herein as first, second, etc. Fab molecule, respectively.

In particular embodiments, the bispecific antigen binding molecule comprises an Fc domain composed of a first and a second subunit. The first and the second subunit of the Fc domain are capable of stable association.

The bispecific antigen binding molecule can have different configurations, i.e. the first and second antigen binding moiety may be fused to each other and to the Fc domain in different ways. The components may be fused to each other directly or, preferably, via one or more suitable linker peptides. Where fusion of a Fab molecule is to the N-terminus of a subunit of the Fc domain, it is typically via an immunoglobulin hinge region.

In some embodiments, the first and the second antigen binding moiety are each a Fab molecule and are each fused at the C-terminus of the Fab heavy chain to the N-terminus of one of the subunits of the Fc domain. In particular such embodiments, the second antigen binding moiety is a conventional Fab molecule, and the first antigen binding moiety is a crossover Fab molecule as described herein, i.e. a Fab molecule wherein the variable domains VH and VL or the constant domains CL and CH1 of the Fab heavy and light chains are exchanged / replaced by each other. In other such embodiments, the second Fab molecule is a crossover Fab molecule and the first Fab molecule is a conventional Fab molecule.

In some embodiments, the bispecific antigen binding molecule essentially consists of the first and the second Fab molecule, the Fc domain composed of a first and a second subunit, and optionally one or more linker peptides, wherein the first and the second Fab molecule are each fused at the C-terminus of the Fab heavy chain to the N-terminus of one of the subunits of the Fc domain. Such a configuration is schematically depicted in **Figure 1** (in the examples in **Figure 1A** and **1C** with the first antigen binding domain being a VH/VL crossover Fab molecule and the second antigen binding moiety being a conventional Fab molecule, and in the examples in **Figure 1B** and **ID** with the second antigen binding domain being a VH/VL crossover Fab molecule and the first antigen binding moiety being a conventional Fab molecule). The first and the second Fab molecule may be fused to the Fc domain directly or through a linker peptide. In a particular embodiment the first and the second Fab molecule are each fused to the Fc domain through an immunoglobulin hinge region. In a specific embodiment, the immunoglobulin hinge region is a human $IgG_1$ hinge region, particularly where the Fc domain is an $IgG_1$ Fc domain.

In configurations of the bispecific antigen binding molecule wherein a Fab molecule is fused at the C-terminus of the Fab heavy chain to the N-terminus of each of the subunits of the Fc domain through an immunoglobulin hinge regions, the two Fab molecules, the hinge regions and the Fc domain essentially form an immunoglobulin molecule. In a particular embodiment the immunoglobulin molecule is an IgG class immunoglobulin. In an even more particular embodiment the immunoglobulin is an $IgG_1$ subclass immunoglobulin. In another embodiment the immunoglobulin is an $IgG_4$ subclass immunoglobulin. In a further particular embodiment the immunoglobulin is a human immunoglobulin. In other embodiments the immunoglobulin is a chimeric immunoglobulin or a humanized immunoglobulin. In one embodiment, the immunoglobulin comprises a human constant region, particularly a human Fc region.

The antigen binding moieties may be fused to the Fc domain (or to each other) directly or through a linker peptide, comprising one or more amino acids, typically about 2-20 amino acids. Linker peptides are known in the art and are described herein. Suitable, non-immunogenic linker peptides include, for example, $(G_4S)n$, $(SG_4)n$, $(G_4S)n$ or $G_4(SG_4)n$ linker peptides. "n" is generally an integer from 1 to 10, typically from 2 to 4. In one embodiment said linker peptide has a length of at least 5 amino acids, in one embodiment a length of 5 to 100, in a further embodiment of 10 to 50 amino acids. In one embodiment said linker peptide is $(GxS)_n$ or $(GxS)_nG_m$ with G=glycine, S=serine, and (x=3, n= 3, 4, 5 or 6, and m=0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said linker peptide is $(G_4S)_2$. Linker peptides may also comprise (a portion of) an immunoglobulin hinge region. Particularly where a Fab molecule is fused to the N-terminus of an Fc domain subunit, it may be fused via an immunoglobulin hinge region or a portion thereof, with or without an additional linker peptide.

In particular embodiments, the bispecific antigen binding molecule comprises a polypeptide wherein the Fab light chain variable region of the first Fab molecule shares a carboxy-terminal peptide bond with the Fab heavy chain constant region of the first Fab molecule (i.e. the first Fab molecule comprises a crossover Fab heavy chain, wherein the heavy chain variable region is replaced by a light chain variable region), which in turn shares a carboxy-terminal peptide bond with an Fc domain subunit ($VL_{(1)}$-$CH1_{(1)}$-CH2-CH3(-CH4)), and a polypeptide wherein the Fab heavy chain of the second Fab molecule shares a carboxy-terminal peptide bond with an Fc domain subunit ($VH_{(2)}$-$CH1_{(2)}$-CH2-CH3(-CH4)). In some embodiments the bispecific antigen binding molecule further comprises a polypeptide wherein the Fab heavy chain variable region of the first Fab molecule shares a carboxy-terminal peptide bond with the Fab light chain constant region of the first Fab molecule ($VH_{(1)}$-$CL_{(1)}$) and the Fab light chain polypeptide of the second Fab molecule ($VL_{(2)}$-$CL_{(2)}$). In certain embodiments the polypeptides are covalently linked, e.g., by a disulfide bond. See e.g. **Figure 1A** and **1C**.

In other embodiments, the bispecific antigen binding molecule comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with an Fc domain subunit ($VL_{(2)}$-$CH1_{(2)}$-CH2-CH3(-CH4)), and a polypeptide wherein the Fab heavy chain of the first Fab molecule shares a carboxy-terminal peptide bond with an Fc domain subunit ($VH_{(1)}$-$CH1_{(1)}$-CH2-CH3(-CH4)). In some embodiments the bispecific antigen binding molecule further comprises a polypeptide wherein the Fab heavy chain variable region of the second Fab molecule shares a carboxy-terminal peptide bond with the Fab light chain constant region of the second Fab molecule ($VH_{(2)}$-$CL_{(2)}$) and the Fab light chain polypeptide of the first Fab molecule ($VL_{(1)}$-$CL_{(1)}$). In certain embodiments the polypeptides are covalently linked, e.g., by a disulfide bond. See e.g. **Figure 1B** and **1D.**

In certain embodiments, the bispecific antigen binding molecule comprises a polypeptide wherein the Fab heavy chain variable region of the first Fab molecule shares a carboxy-terminal peptide bond with the Fab light chain constant region of the first Fab molecule (i.e. the first Fab molecule comprises a crossover Fab heavy chain, wherein the heavy chain constant region is replaced by a light chain constant region), which in turn shares a carboxy-terminal peptide bond with an Fc domain subunit ($VH_{(1)}$-$CL_{(1)}$-CH2-CH3(-CH4)), and a polypeptide wherein the Fab heavy chain of the second Fab molecule shares a carboxy-terminal peptide bond with an Fc domain subunit ($VH_{(2)}$-$CH1_{(2)}$-CH2-

24

CH3(-CH4)). In some embodiments the bispecific antigen binding molecule further comprises a polypeptide wherein the Fab light chain variable region of the first Fab molecule shares a carboxy-terminal peptide bond with the Fab heavy chain constant region of the first Fab molecule ($VL_{(1)}$-$CH1_{(1)}$) and the Fab light chain polypeptide of the second Fab molecule ($VL_{(2)}$-$CL_{(2)}$). In certain embodiments the polypeptides are covalently linked, e.g., by a disulfide bond.

In still other embodiments, the bispecific antigen binding molecule comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with an Fc domain subunit ($VH_{(2)}$-$CL_{(2)}$-CH2-CH3(-CH4)), and a polypeptide wherein the Fab heavy chain of the first Fab molecule shares a carboxy-terminal peptide bond with an Fc domain subunit ($VH_{(1)}$-$CH1_{(1)}$-CH2-CH3(-CH4)). In some embodiments the bispecific antigen binding molecule further comprises a polypeptide wherein the Fab light chain variable region of the second Fab molecule shares a carboxy-terminal peptide bond with the Fab heavy chain constant region of the second Fab molecule ($VL_{(2)}$-$CH1_{(2)}$) and the Fab light chain polypeptide of the first Fab molecule ($VL_{(1)}$-$CL_{(1)}$). In certain embodiments the polypeptides are covalently linked, e.g., by a disulfide bond.

In a particular embodiment, the bispecific antigen binding molecule comprised in the immunoconjugate of the invention comprises

  a) a first antigen binding moiety that binds to PD-1, wherein the first antigen binding moiety is a Fab molecule wherein the variable domains VL and VH or the constant domains CL and CH1 of the Fab light chain and the Fab heavy chain are replaced by each other;
  b) a second antigen binding moiety that binds to Tim-3, wherein the second antigen binding moiety is a (conventional) Fab molecule; and
  c) an Fc domain composed of a first and a second subunit;

wherein

the first antigen binding moiety under a) is fused at the C-terminus of the Fab heavy chain to the N-terminus of one of the subunits (particularly the first subunit) of the Fc domain under c), and the second antigen binding moiety under b) is fused at the C-terminus of the Fab heavy chain to the N-terminus of the other one of the subunits (particularly the second subunit) of the Fc domain under c).

In a particular embodiment, the first and the second antigen binding moiety are each fused to the Fc domain through an immunoglobulin hinge region.

In another embodiment, the bispecific antigen binding molecule comprised in the immunoconjugate of the invention comprises

  a) a first antigen binding moiety that binds to PD-1, wherein the first antigen binding moiety is a (conventional) Fab molecule;
  b) a second antigen binding moiety that binds to Tim-3, wherein the second antigen binding moiety is a Fab molecule wherein the variable domains VL and VH or the constant domains CL and CH1 of the Fab light chain and the Fab heavy chain are replaced by each other; and
  c) an Fc domain composed of a first and a second subunit;

wherein

the first antigen binding moiety under a) is fused at the C-terminus of the Fab heavy chain to the N-terminus of one of the subunits (particularly the first subunit) of the Fc domain under c), and the second antigen binding moiety under b) is fused at the C-terminus of the Fab heavy chain to the N-terminus of the other one of the subunits (particularly the second subunit) of the Fc domain under c).

In a particular embodiment, the first and the second antigen binding moiety are each fused to the Fc domain through an immunoglobulin hinge region.

In all of the different configurations of the bispecific antigen binding molecule, the amino acid substitutions described herein, if present, may either be in the CH1 and CL domains of the first antigen binding moiety/Fab molecule, or in the CH1 and CL domains of the second antigen binding moiety/Fab molecule. Preferably, they are in the CH1 and CL domains of the second antigen binding moiety/Fab molecule. In accordance with the concept of the invention, if amino acid substitutions as described herein are made in the second antigen binding moiety/Fab molecule, no such amino acid substitutions are made in the first antigen binding moiety/Fab molecule. Conversely, if amino acid substitutions as described herein are made in the first antigen binding moiety/Fab molecule, no such amino acid substitutions are made in the second antigen binding moiety/Fab molecule. Amino acid substitutions are particularly made in bispecific antigen binding molecules comprising a Fab molecule wherein the variable domains VL and VH1 of the Fab light chain and the Fab heavy chain are replaced by each other.

In particular embodiments of the bispecific antigen binding molecule, particularly wherein amino acid substitutions as described herein are made in the second antigen binding moiety/Fab molecule, the constant domain CL of the second Fab molecule is of kappa isotype. In other embodiments of the bispecific antigen binding molecule, particularly wherein amino acid substitutions as described herein are made in the first antigen binding moiety/Fab molecule, the constant domain CL of the first antigen binding moiety/Fab molecule is of kappa isotype. In some embodiments, the constant domain CL of the first antigen binding moiety/Fab molecule and the constant domain CL of the second antigen binding moiety/Fab molecule are of kappa isotype.

In a particular embodiment, the bispecific antigen binding molecule comprised in the immunoconjugate of the invention comprises

a) a first antigen binding moiety that binds to PD-1, wherein the first antigen binding moiety is a Fab molecule wherein the variable domains VL and VH of the Fab light chain and the Fab heavy chain are replaced by each other;
b) a second antigen binding moiety that binds to Tim-3, wherein the second antigen binding moiety is a (conventional) Fab molecule;
c) an Fc domain composed of a first and a second subunit;

wherein in the constant domain CL of the second antigen binding moiety under b) the amino acid at position 124 is substituted by lysine (K) (numbering according to Kabat) and the amino acid at position 123 is substituted by lysine (K) or arginine (R) (numbering according to Kabat) (most particularly by arginine (R)), and wherein in the constant domain CH1 of the second antigen binding moiety under b) the amino acid at position 147 is substituted by glutamic acid (E) (numbering according to Kabat EU index) and the amino acid at position 213 is substituted by glutamic acid (E) (numbering according to Kabat EU index); and
wherein
the first antigen binding moiety under a) is fused at the C-terminus of the Fab heavy chain to the N-terminus of one of the subunits (particularly the first subunit) of the Fc domain under c), and the second antigen binding moiety under b) is fused at the C-terminus of the Fab heavy chain to the N-terminus of the other one of the subunits (particularly the second subunit) of the Fc domain under c).

In a particular embodiment, the first and the second antigen binding moiety are each fused to the Fc domain through an immunoglobulin hinge region

In another embodiment, the bispecific antigen binding molecule comprised in the immunoconjugate of the invention comprises

a) a first antigen binding moiety that binds to PD-1, wherein the first antigen binding moiety is a (conventional) Fab molecule;
b) a second antigen binding moiety that binds to Tim-3, wherein the second antigen binding moiety is a Fab molecule wherein the variable domains VL and VH of the Fab light chain and the Fab heavy chain are replaced by each other;
c) an Fc domain composed of a first and a second subunit;

wherein in the constant domain CL of the first antigen binding moiety under a) the amino acid at position 124 is substituted by lysine (K) (numbering according to Kabat) and the amino acid at position 123 is substituted by lysine (K) or arginine (R) (numbering according to Kabat) (most particularly by arginine (R)), and wherein in the constant domain CH1 of the first antigen binding moiety under a) the amino acid at position 147 is substituted by glutamic acid (E) (numbering according to Kabat EU index) and the amino acid at position 213 is substituted by glutamic acid (E) (numbering according to Kabat EU index); and
wherein
the first antigen binding moiety under a) is fused at the C-terminus of the Fab heavy chain to the N-terminus of one of the subunits (particularly the first subunit) of the Fc domain under c), and the second antigen binding moiety under b) is fused at the C-terminus of the Fab heavy chain to the N-terminus of the other one of the subunits (particularly the second subunit) of the Fc domain under c).

In a particular embodiment, the first and the second antigen binding moiety are each fused to the Fc domain through an immunoglobulin hinge region.

*Fc domain*

In particular embodiments, the bispecific antigen binding molecule comprised in the immunoconjugate of the invention comprises an Fc domain composed of a first and a second subunit.

The Fc domain of the bispecific antigen binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment, the bispecific antigen binding molecule comprises not more than one Fc domain.

In one embodiment, the Fc domain of the bispecific antigen binding molecule is an IgG Fc domain. In a particular embodiment, the Fc domain is an $IgG_1$ Fc domain. In another embodiment the Fc domain is an $IgG_4$ Fc domain. In a more specific embodiment, the Fc domain is an $IgG_4$ Fc domain comprising an amino acid substitution at position S228 (Kabat EU index numbering), particularly the amino acid substitution S228P. This amino acid substitution reduces *in vivo* Fab arm exchange of $IgG_4$ antibodies (see Stubenrauch et al., Drug Metabolism and Disposition 38, 84-91 (2010)). In a further particular embodiment, the Fc domain is a human Fc domain. In an even more particular embodiment, the Fc domain is a human $IgG_1$ Fc domain. An exemplary sequence of a human $IgG_1$ Fc region is given in SEQ ID NO: 40.

*Fc domain modifications promoting heterodimerization*

Immunoconjugates according to the invention comprise a mutant IL-2 polypeptide, particularly a single (not more than one) mutant IL-2 polypeptide, and different antigen binding moieties, which may be fused to one or the other of the two subunits of the Fc domain of the bispecific antigen binding molecule, thus the two subunits of the Fc domain are typically comprised in two non-identical polypeptide chains. Recombinant co-expression of these polypeptides and subsequent dimerization leads to several possible combinations of the two polypeptides. To improve the yield and purity of bispecific antigen binding molecules in recombinant production, it will thus be advantageous to introduce in the Fc domain of the bispecific antigen binding molecule a modification promoting the association of the desired polypeptides.

Accordingly, in particular embodiments, the Fc domain of the bispecific antigen binding molecule comprised in the immunoconjugate according to the invention comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

There exist several approaches for modifications in the CH3 domain of the Fc domain in order to enforce heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically, in all such approaches the CH3 domain of the first subunit of the Fc domain and the CH3 domain of the second subunit of the Fc domain are both engineered in a complementary manner so that each CH3 domain (or the heavy chain comprising it) can no longer homodimerize with itself but is forced to heterodimerize with the complementarily engineered other CH3 domain (so that the first and second CH3 domain heterodimerize and no homodimers between the two first or the two second CH3 domains are formed). These different approaches for improved heavy chain heterodimerization are contemplated as different alternatives in combination with the heavy-light chain modifications (e.g. VH and VL exchange/replacement in one binding arm and the introduction of substitutions of charged amino acids with opposite charges in the CH1/CL interface) in the bispecific antigen binding molecule which reduce heavy/light chain mispairing and Bence Jones-type side products.

In a specific embodiment said modification promoting the association of the first and the second subunit of the Fc domain is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the bispecific antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

Preferably said amino acid residue having a larger side chain volume is selected from the group consisting of arginine (R), phenylalanine (F), tyrosine (Y), and tryptophan (W).

Preferably said amino acid residue having a smaller side chain volume is selected from the group consisting of alanine (A), serine (S), threonine (T), and valine (V).

The protuberance and cavity can be made by altering the nucleic acid encoding the polypeptides, e.g. by site-specific mutagenesis, or by peptide synthesis.

In a specific embodiment, in (the CH3 domain of) the first subunit of the Fc domain (the "knobs" subunit) the threonine residue at position 366 is replaced with a tryptophan residue (T366W), and in (the CH3 domain of) the second subunit of the Fc domain (the "hole" subunit) the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A) (numberings according to Kabat EU index).

In yet a further embodiment, in the first subunit of the Fc domain additionally the serine residue at position 354 is replaced with a cysteine residue (S354C) or the glutamic acid residue at position 356 is replaced with a cysteine residue (E356C) (particularly the serine residue at position 354 is replaced with a cysteine residue), and in the second subunit of the Fc domain additionally the tyrosine residue at position 349 is replaced by a cysteine residue (Y349C) (numberings according to Kabat EU index). Introduction of these two cysteine residues results in formation of a disulfide bridge between the two subunits of the Fc domain, further stabilizing the dimer (Carter, J Immunol Methods 248, 7-15 (2001)).

In a particular embodiment, the first subunit of the Fc domain comprises the amino acid substitutions S354C and T366W, and the second subunit of the Fc domain comprises the amino acid substitutions Y349C, T366S, L368A and Y407V (numbering according to Kabat EU index).

In a particular embodiment the mutant IL-2 polypeptide is fused (optionally through a linker peptide) to the first subunit of the Fc domain (comprising the "knob" modification). Without wishing to be bound by theory, fusion of the mutant IL-2 polypeptide to the knob-containing subunit of the Fc domain will (further) minimize the generation of immunoconjugates comprising two mutant IL-2 polypeptides (steric clash of two knob-containing polypeptides). Other techniques of CH3-modification for enforcing the heterodimerization are contemplated as alternatives according to the invention and are described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one embodiment, the heterodimerization approach described in EP 1870459, is used alternatively. This approach is based on the introduction of charged amino acids with opposite charges at specific amino acid positions in the CH3/CH3 domain interface between the two subunits of the Fc domain. One preferred embodiment for the bispecific antigen binding molecule comprised in the immunoconjugate of the invention are amino acid mutations R409D; K370E in one of the two CH3 domains (of the Fc domain) and amino acid mutations D399K; E357K in the other one of the CH3 domains of the Fc domain (numbering according to Kabat EU index).

In another embodiment, the bispecific antigen binding molecule comprised in the immunoconjugate of the invention comprises amino acid mutation T366W in the CH3 domain of the first subunit of the Fc domain and amino acid mutations T366S, L368A, Y407V in the CH3 domain of the second subunit of the Fc domain, and additionally amino acid mutations R409D; K370E in the CH3 domain of the first subunit of the Fc domain and amino acid mutations D399K; E357K in the CH3 domain of the second subunit of the Fc domain (numberings according to Kabat EU index).

In another embodiment, the bispecific antigen binding molecule comprises amino acid mutations S354C, T366W in the CH3 domain of the first subunit of the Fc domain and amino acid mutations Y349C, T366S, L368A, Y407V in the CH3 domain of the second subunit of the Fc domain, or said bispecific antigen binding molecule comprises amino acid mutations Y349C, T366W in the CH3 domain of the first subunit of the Fc domain and amino acid mutations S354C, T366S, L368A, Y407V in the CH3 domains of the second subunit of the Fc domain and additionally amino acid mutations R409D; K370E in the CH3 domain of the first subunit of the Fc domain and amino acid mutations D399K; E357K in the CH3 domain of the second subunit of the Fc domain (all numberings according to Kabat EU index).

In one embodiment, the heterodimerization approach described in WO 2013/157953 is used alternatively. In one embodiment, a first CH3 domain comprises amino acid mutation T366K and a second CH3 domain comprises amino acid mutation L351D (numberings according to Kabat EU index). In a further embodiment, the first CH3 domain comprises further amino acid mutation L351K. In a further embodiment, the second CH3 domain comprises further an amino acid mutation selected from Y349E, Y349D and L368E (preferably L368E) (numberings according to Kabat EU index).

In one embodiment, the heterodimerization approach described in WO 2012/058768 is used alternatively. In one embodiment a first CH3 domain comprises amino acid mutations L351Y, Y407A and a second CH3 domain comprises amino acid mutations T366A, K409F. In a further embodiment the second CH3 domain comprises a further amino acid mutation at position T411, D399, S400, F405, N390, or K392, e.g. selected from a) T411N, T411R, T411Q, T411K, T411D, T411E or T411W, b) D399R, D399W, D399Y or D399K, c) S400E, S400D, S400R, or S400K, d) F405I, F405M, F405T, F405S, F405V or F405W, e) N390R, N390K or N390D, f) K392V, K392M, K392R, K392L, K392F or K392E (numberings according to Kabat EU index). In a further embodiment a first CH3 domain comprises amino acid mutations L351Y, Y407A and a second CH3 domain comprises amino acid mutations T366V, K409F. In a further embodiment, a first CH3 domain comprises amino acid mutation Y407A and a second CH3 domain comprises amino acid mutations T366A, K409F. In a further embodiment, the second CH3 domain further comprises amino acid mutations K392E, T411E, D399R and S400R (numberings according to Kabat EU index).

In one embodiment, the heterodimerization approach described in WO 2011/143545 is used alternatively, e.g. with the amino acid modification at a position selected from the group consisting of 368 and 409 (numbering according to Kabat EU index).

In one embodiment, the heterodimerization approach described in WO 2011/090762, which also uses the knobs-into-holes technology described above, is used alternatively. In one embodiment a first CH3 domain comprises amino acid mutation T366W and a second CH3 domain comprises amino acid mutation Y407A. In one embodiment, a first CH3 domain comprises amino acid mutation T366Y and a second CH3 domain comprises amino acid mutation Y407T (numberings according to Kabat EU index).

In one embodiment, the bispecific antigen binding molecule or its Fc domain is of IgG$_2$ subclass and the heterodimerization approach described in WO 2010/129304 is used alternatively.

In an alternative embodiment, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable. In one such embodiment, a first CH3 domain comprises amino acid substitution of K392 or N392 with a negatively charged amino acid (e.g. glutamic acid (E), or aspartic acid (D), preferably K392D or N392D) and a second CH3 domain comprises amino acid substitution of D399, E356, D356, or E357 with a positively charged amino acid (e.g. lysine (K) or arginine (R), preferably D399K, E356K, D356K, or E357K, and more preferably D399K and E356K). In a further embodiment, the first CH3 domain further comprises amino acid substitution of K409 or R409 with a negatively charged amino acid (e.g. glutamic acid (E), or aspartic acid (D), preferably K409D or R409D). In a further embodiment the first CH3 domain further or alternatively comprises amino acid substitution of K439 and/or K370 with a negatively charged amino acid (e.g. glutamic acid (E), or aspartic acid (D)) (all numberings according to Kabat EU index).

In yet a further embodiment, the heterodimerization approach described in WO 2007/147901 is used alternatively. In one embodiment, a first CH3 domain comprises amino acid mutations K253E, D282K, and K322D and a second CH3 domain comprises amino acid mutations D239K, E240K, and K292D (numberings according to Kabat EU index).

In still another embodiment, the heterodimerization approach described in WO 2007/110205 can be used alternatively.

In one embodiment, the first subunit of the Fc domain comprises amino acid substitutions K392D and K409D, and the second subunit of the Fc domain comprises amino acid substitutions D356K and D399K (numbering according to Kabat EU index).

*Fc domain modifications reducing Fc receptor binding and/or effector function*

The Fc domain confers to the bispecific antigen binding molecule (or the antibody) favorable pharmacokinetic properties, including a long serum half-life which contributes to good accumulation in the target tissue and a favorable tissue-blood distribution ratio. At the same time it may, however, lead to undesirable targeting of the bispecific antigen binding molecule (or the antibody) to cells expressing Fc receptors rather than to the preferred antigen-bearing cells. Moreover, the co-activation of Fc receptor signaling pathways may lead to cytokine release which, in combination with the IL-2 polypeptide and the long half-life of the bispecific antigen binding molecule, results in excessive activation of cytokine receptors and severe side effects upon systemic administration. In line with this, conventional IgG-IL-2 immunoconjugates have been described to be associated with infusion reactions (see e.g. King et al., J Clin Oncol 22, 4463-4473 (2004)).

Accordingly, in particular embodiments, the Fc domain of the bispecific antigen binding molecule comprised in the immunoconjugate according to the invention exhibits reduced binding affinity to an Fc receptor and/or reduced effector function, as compared to a native IgG$_1$ Fc domain. In one such embodiment the Fc domain (or the bispecific antigen binding molecule comprising said Fc domain) exhibits less than 50%, preferably less than 20%, more preferably less than 10% and most preferably less than 5% of the binding affinity to an Fc receptor, as compared to a native IgG$_1$ Fc domain (or a bispecific antigen binding molecule comprising a native IgG$_1$ Fc domain), and/or less than 50%, preferably less than 20%, more preferably less than 10% and most preferably less than 5% of the effector function, as compared to a native IgG$_1$ Fc domain domain (or a bispecific antigen binding molecule comprising a native IgG$_1$ Fc domain). In one embodiment, the Fc domain domain (or the bispecific antigen binding molecule comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcγRIIIa, FcγRI or FcγRIIa, most specifically human FcγRIIIa. In one embodiment the effector function is one or more selected from the group of CDC, ADCC, ADCP, and cytokine secretion. In a particular embodiment, the effector function is ADCC. In one embodiment, the Fc domain domain exhibits substantially similar binding affinity to neonatal Fc receptor (FcRn), as compared to a native IgG$_1$ Fc domain domain. Substantially similar binding to FcRn is achieved when the Fc domain (or the bispecific antigen binding molecule comprising said Fc domain) exhibits greater than about 70%, particularly greater than about 80%, more particularly greater than about 90% of the binding affinity of a native IgG$_1$ Fc domain (or the bispecific antigen binding molecule comprising a native IgG$_1$ Fc domain) to FcRn.

In certain embodiments the Fc domain is engineered to have reduced binding affinity to an Fc receptor and/or reduced effector function, as compared to a non-engineered Fc domain. In particular embodiments, the Fc domain of the bispecific antigen binding molecule comprises one or more amino acid mutation that reduces the binding affinity of the Fc domain to an Fc receptor and/or effector function. Typically, the same one or more amino acid mutation is present in each of the two subunits of the Fc domain. In one embodiment, the amino acid mutation reduces the binding affinity of the Fc domain to an Fc receptor. In one embodiment, the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the bispecific antigen binding molecule comprising an engineered Fc domain exhibits less than 20%, particularly less than 10%, more particularly less than 5% of the binding affinity to an Fc receptor as compared to a bispecific antigen binding molecule comprising a non-engineered Fc domain. In a particular embodiment, the Fc receptor is an receptor. In some embodiments, the Fc receptor is a human Fc receptor. In some embodiments, the Fc receptor is an activating Fc receptor. In a specific embodiment, the Fc receptor is an activating human receptor, more specifically human FcγRIIIa, FcγRI or FcγRIIa, most specifically human FcγRIIIa. Preferably, binding to each of these receptors is reduced. In some embodiments, binding affinity to a complement component, specifically binding affinity to C1q, is also reduced. In one embodiment, binding affinity to neonatal Fc receptor (FcRn) is not reduced. Substantially similar binding to FcRn, i.e. preservation of the binding affinity of the Fc domain to said receptor, is achieved when the Fc domain (or the bispecific antigen binding molecule comprising said Fc domain) exhibits greater than about 70% of the binding affinity of a non-engineered form of the Fc domain (or the bispecific antigen binding molecule comprising said non-engineered form of the Fc domain) to FcRn. The Fc domain, or bispecific antigen binding molecules comprising said Fc domain, may exhibit greater than about 80% and even greater than about 90% of such affinity. In certain embodiments, the Fc domain of the bispecific antigen binding molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment, the reduced effector function is one or more selected from the group of reduced CDC, reduced ADCC, reduced ADCP, and reduced cytokine secretion. In a particular embodiment, the reduced effector function is reduced ADCC. In one embodiment the reduced ADCC is less than 20% of the ADCC induced by a non-engineered Fc domain (or a bispecific antigen binding molecule comprising a non-engineered Fc domain).

In one embodiment, the amino acid mutation that reduces the binding affinity of the Fc domain to an Fc receptor and/or effector function is an amino acid substitution. In one embodiment, the Fc domain comprises an amino acid substitution at a position selected from the group of E233, L234, L235, N297, P331 and P329 (numberings according to Kabat EU index). In a more specific embodiment, the Fc domain comprises an amino acid substitution at a position selected from the group of L234, L235 and P329 (numberings according to Kabat EU index). In some embodiments, the Fc domain comprises the amino acid substitutions L234A and L235A (numberings according to Kabat EU index). In one such embodiment, the Fc domain is an IgG$_1$ Fc domain, particularly a human IgG$_1$ Fc domain. In one embodiment, the Fc domain comprises an amino acid substitution at position P329. In a more specific embodiment, the amino acid substitution is P329A or P329G, particularly P329G (numberings according to Kabat EU index). In one embodiment, the Fc domain comprises an amino acid substitution at position P329 and a further amino acid substitution at a position selected from E233, L234, L235, N297 and P331 (numberings according to Kabat EU index). In a more specific embodiment, the further amino acid substitution is E233P, L234A, L235A, L235E, N297A, N297D or P331S. In particular embodiments, the Fc domain comprises amino acid substitutions at positions P329, L234 and L235 (numberings according to Kabat EU index). In more particular embodiments, the Fc domain comprises the amino acid mutations L234A, L235A and P329G ("P329G LALA", "PGLALA" or "LALAPG"). Specifically, in particular embodiments, each subunit of the Fc domain comprises the amino acid substitutions L234A, L235A and P329G (Kabat EU index numbering), i.e. in each of the first and the second subunit of the Fc domain the leucine residue at position 234 is replaced with an alanine residue (L234A), the leucine residue at position 235 is replaced with an alanine residue (L235A) and the proline residue at position 329 is replaced by a glycine residue (P329G) (numbering according to Kabat EU index). . In one such embodiment, the Fc domain is an IgG$_1$ Fc domain, particularly a human IgG$_1$ Fc domain. The "P329G LALA" combination of amino acid substitutions almost completely abolishes Fcγ receptor (as well as complement) binding of a human IgG$_1$ Fc domain, as described in PCT publication no. WO 2012/130831. WO 2012/130831 also describes methods of preparing such mutant Fc domains and methods for determining its properties such as Fc receptor binding or effector functions.

IgG$_4$ antibodies exhibit reduced binding affinity to Fc receptors and reduced effector functions as compared to IgG$_1$ antibodies. Hence, in some embodiments, the Fc domain of the bispecific antigen binding molecule comprised in the immunoconjugate of the invention is an IgG$_4$ Fc domain, particularly a human IgG$_4$ Fc domain. In one embodiment, the IgG$_4$ Fc domain comprises amino acid substitutions at position S228, specifically the amino acid substitution S228P (numberings according to Kabat EU index). To further reduce its binding affinity to an Fc receptor and/or its effector function, in one embodiment, the IgG$_4$ Fc domain comprises an amino acid substitution at position L235, specifically the amino acid substitution L235E (numberings according to Kabat EU index). In another embodiment, the IgG$_4$ Fc domain comprises an amino acid substitution at position P329, specifically the amino acid substitution P329G (numberings according to Kabat EU index). In a particular embodiment, the IgG$_4$ Fc domain comprises amino acid substitutions at positions S228, L235 and P329, specifically amino acid substitutions S228P, L235E and P329G (numberings according to Kabat EU index). Such IgG$_4$ Fc domain mutants and their receptor binding properties are described in PCT publication no. WO 2012/130831.

In a particular embodiment, the Fc domain exhibiting reduced binding affinity to an Fc receptor and/or reduced effector function, as compared to a native IgG$_1$ Fc domain, is a human IgG$_1$ Fc domain comprising the amino acid substitutions L234A, L235A and optionally P329G, or a human IgG$_4$ Fc domain comprising the amino acid substitutions S228P, L235E and optionally P329G (numberings according to Kabat EU index).

In certain embodiments, N-glycosylation of the Fc domain has been eliminated. In one such embodiment, the Fc domain comprises an amino acid mutation at position N297, particularly an amino acid substitution replacing asparagine by alanine (N297A) or aspartic acid (N297D) (numberings according to Kabat EU index).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056) (numberings according to Kabat EU index). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. Alternatively, binding affinity of Fc domains or bispecific antigen binding molecules comprising an Fc domain for Fc receptors may be evaluated using cell lines known to express particular Fc receptors, such as human NK cells expressing FcγIIIa receptor.

Effector function of an Fc domain, or a bispecific antigen binding molecule comprising an Fc domain, can be measured by methods known in the art. Examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the Fc domain, or the bispecific antigen binding molecule comprising the Fc domain, is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

FcRn binding and *in vivo* clearance/half life determinations can also be performed using methods known in the art (see, e.g., Petkova, S.B. et al., Int'l. Immunol. 18(12):1759-1769 (2006); WO 2013/120929).

Particular aspects of the invention

In a particular aspect, the invention provides an immunoconjugate comprising a mutant IL-2 polypeptide and a bispecific antigen binding molecule that binds to PD-1 and Tim-3,

wherein the mutant IL-2 polypeptide is a human IL-2 molecule comprising the amino acid substitutions F42A, Y45A and L72G (numbering relative to the human IL-2 sequence SEQ ID NO: 22); and
wherein the bispecific antigen binding molecule comprises

(i) a first antigen binding moiety that binds to PD-1, comprising (a) a heavy chain variable region (VH) comprising the amino acid sequence that of SEQ ID NO:7, and (b) a light chain variable region (VL) comprising the amino acid sequence of SEQ ID NO:8; and
(ii) a second antigen binding moiety that binds to Tim-3, comprising (a) a heavy chain variable region (VH) comprising the amino acid sequence of SEQ ID NO:18, and (b) a light chain variable region (VL) comprising the amino acid sequence of SEQ ID NO:19.

In a particular aspect, the invention provides an immunoconjugate comprising a mutant IL-2 polypeptide and a bispecific antigen binding molecule that binds to PD-1 and Tim-3,

wherein the mutant IL-2 polypeptide is a human IL-2 molecule comprising the amino acid substitutions T3A, F42A, Y45A, L72G and C125A (numbering relative to the human IL-2 sequence SEQ ID NO: 22); and
wherein the bispecific antigen binding molecule comprises

(i) a first antigen binding moiety that binds to PD-1, comprising (a) a heavy chain variable region (VH) comprising the amino acid sequence that of SEQ ID NO:7, and (b) a light chain variable region (VL) comprising the amino acid sequence of SEQ ID NO:8; and
(ii) a second antigen binding moiety that binds to Tim-3, comprising (a) a heavy chain variable region (VH) comprising the amino acid sequence of SEQ ID NO:18, and (b) a light chain variable region (VL) comprising the amino acid sequence of SEQ ID NO:19.

In a particular aspect, the invention provides an immunoconjugate comprising a mutant IL-2 polypeptide and a bispecific antigen binding molecule that binds to PD-1 and Tim-3,
wherein the mutant IL-2 polypeptide comprises the amino acid sequence of SEQ ID NO: 23; and wherein the bispecific antigen binding molecule comprises

(i) a first antigen binding moiety that binds to PD-1, comprising (a) a heavy chain variable region (VH) comprising the amino acid sequence that of SEQ ID NO:7, and (b) a light chain variable region (VL) comprising the amino acid sequence of SEQ ID NO:8; and
(ii) a second antigen binding moiety that binds to Tim-3, comprising (a) a heavy chain variable region (VH) comprising the amino acid sequence of SEQ ID NO:18, and (b) a light chain variable region (VL) comprising the amino acid sequence of SEQ ID NO:19.

In some embodiments according to any one of the above aspects, the first antigen binding moiety is a Fab molecule wherein the variable domains VL and VH of the Fab light chain and the Fab heavy chain are replaced by each other, and the second antigen binding moiety is a (conventional) Fab molecule. In some such embodiments, in the constant domain CL of the second antigen binding moiety the amino acid at position 124 is substituted by lysine (K) (numbering according to Kabat) and the amino acid at position 123 is substituted by lysine (K) or arginine (R) (numbering according to Kabat) (most particularly by arginine (R)), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index).

In some embodiments according to any one of the above aspects, the bispecific antigen binding molecule further comprises an Fc domain composed of a first and a second subunit. In some such embodiments, the first antigen binding moiety is fused at the C-terminus of the Fab heavy chain to the N-terminus of one of the subunits of the Fc domain (particularly to the first subunit of the Fc domain), and the second antigen binding moiety is fused at C-terminus of the Fab heavy chain to the N-terminus of the other one of the subunits of the Fc domain (particularly to the second subunit of the Fc domain).

In a particular aspect, the invention provides an immunoconjugate comprising a mutant IL-2 polypeptide and a bispecific antigen binding molecule that binds to PD-1 and Tim-3,

wherein the mutant IL-2 polypeptide comprises the amino acid sequence of SEQ ID NO: 23; and wherein the bispecific antigen binding molecule comprises

(i) a first antigen binding moiety that binds to PD-1, wherein the first antigen binding moiety is a Fab molecule wherein the variable domains VL and VH of the Fab light chain and the Fab heavy chain are replaced by each other, comprising (a) a heavy chain variable region (VH) comprising the amino acid sequence that of SEQ ID NO:7, and (b) a light chain variable region (VL) comprising the amino acid sequence of SEQ ID NO:8; (ii) a second antigen binding moiety that binds to Tim-3, wherein the second antigen binding moiety is a (conventional) Fab molecule, comprising (a) a heavy chain variable region (VH) comprising the amino acid sequence of SEQ ID NO: 18, and (b) a light chain variable region (VL) comprising the amino acid sequence of SEQ ID NO:19, wherein in the constant domain CL of the second antigen binding moiety the amino acid at position 124 is substituted by lysine (K) (numbering according to Kabat) and the amino acid at position 123 is substituted by lysine (K) or arginine (R) (numbering according to Kabat) (most particularly by arginine (R)), and in the constant domain CH1 of the second antigen binding moiety the amino acid at position 147 is substituted by glutamic acid (E) (numbering according to Kabat EU index) and the amino acid at position 213 is substituted by glutamic acid (E) (numbering according to Kabat EU index); and (iii) an Fc domain composed of a first and a second subunit,

wherein the first antigen binding moiety is fused at the C-terminus of the Fab heavy chain to the N-terminus of one of the subunits of the Fc domain (particularly to the first subunit of the Fc domain), and the second antigen binding moiety is fused at C-terminus of the Fab heavy chain to the N-terminus of the other one of the subunits of the Fc domain (particularly to the second subunit of the Fc domain).

In some embodiments according to any of the above aspects of the invention, in the first subunit of the Fc domain the threonine residue at position 366 is replaced with a tryptophan residue (T366W), and in the second subunit of the Fc domain the tyrosine residue at position 407 is replaced with a valine residue (Y407V) and optionally the threonine residue at position 366 is replaced with a serine residue (T366S) and the leucine residue at position 368 is replaced with an alanine residue (L368A) (numberings according to Kabat EU index). In some such embodiments, in the first subunit of the Fc domain additionally the serine residue at position 354 is replaced with a cysteine residue (S354C) or the glutamic acid residue at position 356 is replaced with a cysteine residue (E356C) (particularly the serine residue at position 354 is replaced with a cysteine residue), and in the second subunit of the Fc domain additionally the tyrosine residue at position 349 is replaced by a cysteine residue (Y349C) (numberings according to Kabat EU index).

In some embodiments according to any of the above aspects of the invention, in each of the first and the second subunit of the Fc domain the leucine residue at position 234 is replaced with an alanine residue (L234A), the leucine residue at position 235 is replaced with an alanine residue (L235A) and the proline residue at position 329 is replaced by a glycine residue (P329G) (numbering according to Kabat EU index).

In some embodiments according to any of the above aspects of the invention, the Fc domain is a human IgG₁ Fc domain.

In some embodiments according to any of the above aspects of the invention, the mutant IL-2 polypeptide is fused at its amino-terminal amino acid to the carboxy-terminal amino acid of the first subunit of the Fc domain, through a linker peptide of SEQ ID NO: 24.

In particular specific embodiment, the immunoconjugate comprises a polypeptide comprising an amino acid sequence that is at least 95%, 96%, 97%, 98%, or 99% identical to the sequence of SEQ ID NO: 25, a polypeptide comprising an amino acid sequence that is at least 95%, 96%, 97%, 98%, or 99% identical to the sequence of SEQ ID NO: 26, a polypeptide comprising an amino acid sequence that is at least 95%, 96%, 97%, 98%, or 99% identical to the sequence of SEQ ID NO: 27, and a polypeptide comprising an amino acid sequence that is at least 95%, 96%, 97%, 98%, or 99% identical to the sequence of SEQ ID NO: 28. In a further particular specific embodiment, the bispecific antigen binding molecule comprises a polypeptide comprising the amino acid sequence of SEQ ID NO: 25, a polypeptide comprising the amino acid sequence of SEQ ID NO: 26, a polypeptide comprising the amino acid sequence of SEQ ID NO: 27 and a polypeptide comprising the amino acid sequence of SEQ ID NO: 28.

**Polynucleotides**

The invention further provides isolated polynucleotides encoding an immunoconjugate as claimed herein. The polynucleotides encoding immunoconjugates of the invention may be expressed as a single polynucleotide that encodes the entire immunoconjugate or as multiple (e.g., two or more) polynucleotides that are co-expressed. Polypeptides encoded by polynucleotides that are co-expressed may associate through, e.g., disulfide bonds or other means to form a functional immunoconjugate. For example, the light chain portion of an antibody may be encoded by a separate polynucleotide from the portion of the immunoconjugate comprising the heavy chain portion of the antibody and the mutant IL-2 polypeptide. When co-expressed, the heavy chain polypeptides will associate with the light chain polypeptides to form the immunoconjugate. In another example, the portion of the immunoconjugate comprising one of the two Fc domain subunits and the mutant IL-2 polypeptide could be encoded by a separate polynucleotide from the portion of the immunoconjugate comprising the the other of the two Fc domain subunits. When co-expressed, the Fc domain subunits will associate to form the Fc domain.

In certain embodiments the polynucleotide or nucleic acid is DNA. In other embodiments, a polynucleotide of the present invention is RNA, for example, in the form of messenger RNA (mRNA). RNA of the present invention may be single stranded or double stranded.

**Recombinant Methods**

Mutant IL-2 polypeptides useful in the invention can be prepared by deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing. In this regard, the nucleotide sequence of native IL-2 has been described by Taniguchi et al. (Nature 302, 305-10 (1983)) and nucleic acid encoding human IL-2 is available from public depositories such as the American Type Culture Collection (Rockville MD). The sequence of native human IL-2 is shown in SEQ ID NO: 22. Substitution or insertion may involve natural as well as non-natural amino acid residues. Amino acid modification includes well known methods of chemical modification such as the addition of glycosylation sites or carbohydrate attachments, and the like.

Immunoconjugates of the invention may be obtained, for example, by solid-state peptide synthesis (e.g. Merrifield solid phase synthesis) or recombinant production. For recombinant production one or more polynucleotide encoding the immunoconjugate (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides of the invention is provided. Methods which are well known to those skilled in the art can be used to construct expression vectors containing the coding sequence of an immunoconjugate (fragment) along with appropriate transcriptional/translational control signals. These methods include *in vitro* recombinant DNA techniques, synthetic techniques and *in vivo* recombination/genetic recombination. *See,* for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the immunoconjugate (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, *e.g.* a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the immunoconjugate of the invention, or variant or derivative thereof. Heterologous coding regions include without limitation specialized elements or motifs, such as a secretory signal peptide or a heterologous functional domain. An operable association is when a coding region for a gene product, *e.g.* a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (*e.g.* the immediate early promoter, in conjunction with intron-A), simian virus 40 (*e.g.* the early promoter), and retroviruses (such as, *e.g.* Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as inducible promoters (*e.g.* promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. For example, human IL-2 is translated with a 20 amino acid signal sequence at the N-terminus of the polypeptide, which is subsequently cleaved off to produce the mature, 133 amino acid human IL-2. In certain embodiments, the native signal peptide, *e.g.* the IL-2 signal peptide or an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the immunoconjugate may be included within or at the ends of the immunoconjugate (fragment) encoding polynucleotide.

In a further embodiment, a host cell comprising one or more polynucleotides of the invention is provided. In certain embodiments a host cell comprising one or more vectors of the invention is provided. The polynucleotides and vectors may incorporate any of the features, singly or in combination, described herein in relation to polynucleotides and vectors, respectively. In one such embodiment a host cell comprises (e.g. has been transformed or transfected with) one or more vector comprising one or more polynucleotide that encodes the immunoconjugate of the invention. As used herein, the term "host cell" refers to any kind of cellular system which can be engineered to generate the immunoconjugates of the invention or fragments thereof. Host cells suitable for replicating and for supporting expression of immunoconjugates are well known in the art. Such cells may be transfected or transduced as appropriate with the particular expression vector and large quantities of vector containing cells can be grown for seeding large scale fermenters to obtain sufficient quantities of the immunoconjugate for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a mutant-IL-2 polypeptide fused to either the heavy or the light chain of an antibody may be engineered so as to also express the other of the antibody chains such that the expressed mutant IL-2 fusion product is an antibody that has both a heavy and a light chain.

In one embodiment, a method of producing an immunoconjugate according to the invention is provided, wherein the method comprises culturing a host cell comprising one or more polynucleotide encoding the immunoconjugate, as provided herein, under conditions suitable for expression of the immunoconjugate, and optionally recovering the immunoconjugate from the host cell (or host cell culture medium).

In the immunoconjugate of the invention, the mutant IL-2 polypeptide may be genetically fused to the bispecific antigen binding molecule, or may be chemically conjugated to the bispecific antigen binding molecule. Genetic fusion of the IL-2 polypeptide to the bispecific antigen binding molecule can be designed such that the IL-2 sequence is fused directly to the polypeptide or indirectly through a linker sequence. The composition and length of the linker may be determined in accordance with methods well known in the art and may be tested for efficacy. Particular linker peptides are described herein. Additional sequences may also be included to incorporate a cleavage site to separate the individual components of the fusion if desired, for example an endopeptidase recognition sequence. In addition, an IL-2 fusion protein may also be synthesized chemically using methods of polypeptide synthesis as is well known in the art (e.g. Merrifield solid phase synthesis). Mutant IL-2 polypeptides may be chemically conjugated to other molecules, e.g. antibodies, using well known chemical conjugation methods. Bi-functional cross-linking reagents such as homofunctional and heterofunctional cross-linking reagents well known in the art can be used for this purpose. The type of cross-linking reagent to use depends on the nature of the molecule to be coupled to IL-2 and can readily be identified by those skilled in the art. Alternatively, or in addition, mutant IL-2 and/or the molecule to which it is intended to be conjugated may be chemically derivatized such that the two can be conjugated in a separate reaction as is also well known in the art.

The immunoconjugates of the invention comprise a bispecific antigen binding molecule, which may comprise (part of) an antibody. Methods to produce antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty). Immunoconjugates, antibodies, and methods for producing the same are also described in detail e.g. in PCT publication nos. WO 2011/020783, WO 2012/107417, and WO 2012/146628.

Any animal species of antibody, antibody fragment, antigen binding domain or variable region may be used in the immunoconjugates of the invention. Non-limiting antibodies, antibody fragments, antigen binding domains or variable regions useful in the present invention can be of murine, primate, or human origin. If the immunoconjugate is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling). Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

Human antibodies can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mousehuman heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolation from human antibody libraries, as described herein.

Antibodies useful in the invention may be isolated by screening combinatorial libraries for antibodies with the desired activity or activities. Methods for screening combinatorial libraries are reviewed, e.g., in Lerner et al. in Nature Reviews 16:498-508 (2016). For example, a variety of methods are known in the art for generating phage display libraries and screening such libraries for antibodies possessing the desired binding characteristics. Such methods are reviewed, e.g., in Frenzel et al. in mAbs 8:1177-1194 (2016); Bazan et al. in Human Vaccines and Immunotherapeutics 8:1817-1828 (2012) and Zhao et al. in Critical Reviews in Biotechnology 36:276-289 (2016) as well as in Hoogenboom et al. in Methods in Molecular Biology 178:1-37 (O'Brien et al., ed., Human Press, Totowa, NJ, 2001) and in Marks and Bradbury in Methods in Molecular Biology 248:161-175 (Lo, ed., Human Press, Totowa, NJ, 2003).

In certain phage display methods, repertoires of VH and VL genes are separately cloned by polymerase chain reaction (PCR) and recombined randomly in phage libraries, which can then be screened for antigen-binding phage as described in Winter et al. in Annual Review of Immunology 12: 433-455 (1994). Phage typically display antibody fragments, either as singlechain Fv (scFv) fragments or as Fab fragments. Libraries from immunized sources provide highaffinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al. in EMBO Journal 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter in Journal of Molecular Biology 227: 381-388 (1992). Patent publications describing human antibody phage libraries include, for example: US Patent Nos. 5,750,373; 7,985,840; 7,785,903 and 8,679,490 as well as US Patent Publication Nos. 2005/0079574, 2007/0117126, 2007/0237764 and 2007/0292936. Further examples of methods known in the art for screening combinatorial libraries for antibodies with a desired activity or activities include ribosome and mRNA display, as well as methods for antibody display and selection on bacteria, mammalian cells, insect cells or yeast cells. Methods for yeast surface display are reviewed, e.g., in Scholler et al. in Methods in Molecular Biology 503:135-56 (2012) and in Cherf et al. in Methods in Molecular biology 1319:155-175 (2015) as well as in the Zhao et al. in Methods in Molecular Biology 889:73-84 (2012). Methods for ribosome display are described, e.g., in He et al. in Nucleic Acids Research 25:5132-5134 (1997) and in Hanes et al. in PNAS 94:4937-4942 (1997).

Further chemical modification of the immunoconjugate of the invention may be desirable. For example, problems of immunogenicity and short half-life may be improved by conjugation to substantially straight chain polymers such as polyethylene glycol (PEG) or polypropylene glycol (PPG) (see e.g. WO 87/00056).

Immunoconjugates prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the immunoconjugate binds. For example, an antibody which specifically binds the mutant IL-2 polypeptide may be used. For affinity chromatography purification of immunoconjugates of the invention, a matrix with protein A or protein G may be used. For example, sequential Protein A or G affinity chromatography and size exclusion chromatography can be used to isolate an immunoconjugate essentially as described in the Examples. The purity of the immunoconjugate can be determined by any of a variety of well known analytical methods including gel electrophoresis, high pressure liquid chromatography, and the like.

## Compositions, Formulations, and Routes of Administration

In a further aspect, the invention provides pharmaceutical compositions comprising an immunoconjugate as described herein, e.g., for use in any of the below therapeutic methods. In one embodiment, a pharmaceutical composition comprises any of the immunoconjugates provided herein and a pharmaceutically acceptable carrier. In another embodiment, a pharmaceutical composition comprises any of the immunoconjugates provided herein and at least one additional therapeutic agent, e.g., as described below.

Further provided is a method of producing an immunoconjugate of the invention in a form suitable for administration in vivo, the method comprising (a) obtaining an immunoconjugate according to the invention, and (b) formulating the immunoconjugate with at least one pharmaceutically acceptable carrier, whereby a preparation of immunoconjugate is formulated for administration in vivo.

Pharmaceutical compositions of the present invention comprise a therapeutically effective amount of immunoconjugate dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains immunoconjugate and optionally an additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (e.g. antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

An immunoconjugate of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic.

Parenteral compositions include those designed for administration by injection, e.g. subcutaneous, intradermal, intralesional, intravenous, intraarterial intramuscular, intrathecal or intraperitoneal injection. For injection, the immunoconjugates of the invention may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the immunoconjugates may be in powder form for constitution with a suitable vehicle, e.g., sterile pyrogen-free water, before use. Sterile injectable solutions are prepared by incorporating the immunoconjugates of the invention in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatinmicrocapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

In addition to the compositions described previously, the immunoconjugates may also be formulated as a depot preparation. Such long acting formulations may be administered by implantation (for example subcutaneously or intramuscularly) or by intramuscular injection. Thus, for example, the immunoconjugates may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the immunoconjugates of the invention may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The immunoconjugates may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

## Compositions and their medical uses

Any of the immunoconjugates provided herein may be used in therapeutic methods. Immunoconjugates of the invention may be used as immunotherapeutic agents, for example in the treatment of cancers.

For use in therapeutic methods, immunoconjugates of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

Immunoconjugates of the invention may be particularly useful in treating disease states where stimulation of the immune system of the host is beneficial, in particular conditions where an enhanced cellular immune response is desirable. These may include disease states where the host immune response is insufficient or deficient. Disease states for which the immunoconjugates of the invention may be administered comprise, for example, a tumor or infection where a cellular immune response would be a critical mechanism for specific immunity. The immunoconjugates of the invention may be administered per se or in any suitable pharmaceutical composition.

In one aspect, immunoconjugates of the invention for use as a medicament are provided. In further aspects, immunoconjugates of the invention for use in treating cancer are provided. In one embodiment, the invention provides an immunoconjugate as described herein for use in the treatment of cancer in an individual in need thereof. In certain embodiments, the invention provides an immunoconjugate for use in a method of treating an individual having cancer comprising administering to the individual a therapeutically effective amount of the immunoconjugate. In certain embodiments the method further comprises administering to the individual a therapeutically effective amount of at least one additional therapeutic agent, e.g., an anti-cancer agent. An "individual" according to any of the above embodiments is a mammal, preferably a human. "Stimulation of the immune system" may include any one or more of a general increase in immune function, an increase in T cell function, an increase in B cell function, a restoration of lymphocyte function, an increase in the expression of IL-2 receptors, an increase in T cell responsiveness, an increase in natural killer cell activity or lymphokineactivated killer (LAK) cell activity, and the like.

In certain embodiments the disease to be treated is cancer. Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that may be treated using an immunoconjugate of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are precancerous conditions or lesions and cancer metastases. In certain embodiments the cancer is chosen from the group consisting of kidney cancer, skin cancer, lung cancer, colorectal cancer, breast cancer, brain cancer, head and neck cancer, prostate cancer and bladder cancer. A skilled artisan readily recognizes that in many cases the immunoconjugates may not provide a cure but may only provide partial benefit. In some embodiments, a physiological change having some benefit is also considered therapeutically beneficial. Thus, in some embodiments, an amount of immunoconjugate that provides a physiological change is considered an "effective amount" or a "therapeutically effective amount". The subject, patient, or individual in need of treatment is typically a mammal, more specifically a human.

For the prevention or treatment of disease, the appropriate dosage of an immunoconjugate of the invention (when used alone or in combination with one or more other additional therapeutic agents) will depend on the type of disease to be treated, the route of administration, the body weight of the patient, the type of molecule (e.g. comprising an Fc domain or not), the severity and course of the disease, whether the immunoconjugate is administered for preventive or therapeutic purposes, previous or concurrent therapeutic interventions, the patient's clinical history and response to the immunoconjugate, and the discretion of the attending physician.. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The immunoconjugate is suitably administered to the patient at one time or over a series of treatments. Depending on the type and severity of the disease, about 1 μg/kg to 15 mg/kg (e.g. 0.1 mg/kg - 10 mg/kg) of immunoconjugate can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the immunoconjugate would be in the range from about 0.005 mg/kg to about 10 mg/kg. In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the immunoconjugate). An initial higher loading dose, followed by one or more lower doses may be administered. However, other dosage regimens may be useful. The progress of this therapy is easily monitored by conventional techniques and assays.

The immunoconjugates of the invention will generally be used in an amount effective to achieve the intended purpose. For use to treat or prevent a disease condition, the immunoconjugates of the invention, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such as cell culture assays. A dose can then be formulated in animal models to achieve a circulating concentration range that includes the $IC_{50}$ as determined in cell culture. Such information can be used to more accurately determine useful doses in humans.

Initial dosages can also be estimated from *in vivo* data, *e.g.,* animal models, using techniques that are well known in the art. One having ordinary skill in the art could readily optimize administration to humans based on animal data.

Dosage amount and interval may be adjusted individually to provide plasma levels of the immunoconjugates which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the immunoconjugates may not be related to plasma concentration. One having skill in the art will be able to optimize therapeutically effective local dosages without undue experimentation.

A therapeutically effective dose of the immunoconjugates described herein will generally provide therapeutic benefit without causing substantial toxicity. Toxicity and therapeutic efficacy of an immunoconjugate can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Immunoconjugates that exhibit large therapeutic indices are preferred. In one embodiment, the immunoconjugate according to the present invention exhibits a high therapeutic index. The data obtained from cell culture assays and animal studies can be used in formulating a range of dosages suitable for use in humans. The dosage lies preferably within a range of circulating concentrations that include the $ED_{50}$ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition. (See, e.g., Fingl et al., 1975, In: The Pharmacological Basis of Therapeutics, Ch. 1, p. 1).

The attending physician for patients treated with immunoconjugates of the invention would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

The maximum therapeutic dose of an immunoconjugate comprising a mutant IL-2 polypeptide as described herein may be increased from those used for an immunoconjugate comprising wild-type IL-2.

**Other Agents and Treatments**

The immunoconjugates according to the invention may be administered in combination with one or more other agents in therapy. For instance, an immunoconjugate of the invention may be coadministered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of immunoconjugate used, the type of disorder or treatment, and other factors discussed above. The immunoconjugates are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the immunoconjugate of the invention can occur prior to, simultaneously, and/or following, administration of the additional therapeutic agent and/or adjuvant. Immunoconjugates of the invention may also be used in combination with radiation therapy.

**Articles of Manufacture**

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an immunoconjugate of the invention. The label or package insert indicates that the composition is used for treating the condition of choice. Moreover, the article of manufacture may comprise (a) a first container with a composition contained therein, wherein the composition comprises an immunoconjugate of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Brief Description of the Drawings**

**Figure 1.** Exemplary configurations of the bispecific antigen binding molecule comprised in the immunoconjugate described herein. (A, C) Bispecific antigen binding molecule comprising VH/VL crossover Fab molecule that binds to PD-1, (conventional) Fab molecule that binds to Tim-3, and Fc domain. (B, D) Bispecific antigen binding molecule comprising (conventional) Fab molecule that binds to PD-1, VH/VL crossover Fab molecule that binds to Tim-3, and Fc domain. Black dot: optional modification in the Fc domain promoting heterodimerization. ++, --: amino acids of opposite charges optionally introduced in the CH1 and CL domains. Crossover Fab molecules are depicted as comprising an exchange of VH and VL regions, but may - particularly in embodiments wherein no charge modifications are introduced in CH1 and CL domains - alternatively comprise an exchange of the CH1 and CL domains.

**Figure 2.** Binding of PD1-TIM3-IL2v in comparison to PD1 IgG and CEA-IL2v to CD4 T cells (A) and CD8 T cells (B) within CD3/CD28 activated PBMCs.

**Figure 3.** Proliferation of the human NK cell line NK92 induced by PD1-TIM3-IL2v, measured after 2 days.

**Figure 4.** Ability of CD4 T cells to secrete IL-2 (A), IL-2 and IFN-γ, or IFN-γ (C) upon 48 hours recall with CMV immunogenic protein pp65 in presence of either anti-PD-1 alone or in combination with IL-2v, of PD1-IL2v, PD1-TIM3-IL2v, or the combination of anti-PD-1, anti-TIM-3 and IL-2v.

**Figure 5.** Differentiation state of virus-specific CD4 T cells secreting (A and B) both IL-2 and IFN-γ or only IFN-γ (C, D, E) upon 48 hours recall with CMV immunogenic protein pp65 in presence of either anti-PD-1 alone or in combination with IL-2v, of PD1-IL2v, PD1-TIM3-IL2v, or the combination of anti-PD-1, anti-TIM-3 and IL-2v.

**Figure 6A-D.** Ability of CD4 T cells to secrete IL-2 (Figure 6A), IL-2 and IFN-γ (Figure 6B) or IFN-γ (Figure 6C) and to proliferate (Figure 6D) upon 48hours recall with CMV immunogenic protein pp65 in presence of either anti-PD-1 alone, in combination with TIM-3 and IL-2v, or as fusion protein.

**Figure 7.** Differentiation state, as per expression of CD45RO and CD62L, of virus-specific CD4 T cells secreting IFN-γ upon 48 hours recall with CMV immunogenic protein pp65 in presence of either anti-PD-1 alone, in combination with TIM-3 and IL-2v, or as fusion protein.

**Figure 8A and 8B.** Delta of the frequency of a given antibody bound on Tconv versus Treg within the same sample. Each symbol represents a separate donor, horizontal lines indicate medians with N=4 (Figure 8A). Data from one representative donor showing the binding to Tconv (black line) and Treg (grey) (Figure 8B).

**Figure 9.** Percentage of suppression by Tregs of granzyme B (Figure 9A) and interferon-y (Figure 9B) secreted by Tconv after 5 days of coculture. Each symbol represents a separate donor, horizontal lines indicate medians with N=5, dotted lines at 0% represents no suppression by Treg. P was calculated using one-way ANOVA (*$p<0.05$, **$p<0.01$, ***$p<0.001$, ****$p<0.0001$).

**Figure 10A-D.** STAT5 assay on resting PBMCs of a first donor (CD8 T-cells (A), NK cells (B), CD4 T-cells (C) and regulatory T-cells (D)).

**Figure 11A-D.** STAT5 assay on resting PBMCs of a second donor (CD4 T-cells (A), CD8 Tcells (B), regulatory T-cells (C) and NK cells (D)).

**Figure 12A-D.** STAT5 assay on resting PBMCs of a third donor (CD8 T-cells (A), NK cells (B), CD4 T-cells (C) and regulatory T-cells (D)).

**Figure 13A-D.** STAT5 assay on resting PBMCs of a fourth donor (CD8 T-cells (A), NK cells (B), CD4 T-cells (C) and regulatory T-cells (D).

**Amino Acid Sequences**

| | Amino Acid Sequence | SEQ ID NO |
|---|---|---|
| PD-1 HVR-H1 | GFSFSSY | 1 |
| PD-1 HVR-H2 | GGR | 2 |
| PD-1 HVR-H3 | TGRVYFALD | 3 |
| PD-1 HVR-L1 | SESVDTSDNSF | 4 |
| PD-1 HVR-L2 | RSS | 5 |
| PD-1 HVR-L3 | NYDVPW | 6 |
| PD-1 VH (1, 2, 3, 4) | EVQLLESGGGLVQPGGSLRLSCAASGFSFSSYTMSWVRQAPGKGLEWVATISGGGRDIYYPDSVKGRFTISRDNSKNTLYLQMNSLRAEDTAVYYCVLLTGRVYFALDSWGQGTLVTVSS | 7 |
| PD-1 VL (1) | DIVMTQSPDSLAVSLGERATINCKASESVDTSDNSFIHWYQQKPGQSPKLLIYRSSTLESGVPDRFSGSGSGTDFTLTISSLQAEDVAVYYCQQNYDVPWTFGQGTKVEIK | 8 |
| PD-1 VL (2) | DVVMTQSPLSLPVTLGQPASISCRASESVDTSDNSFIHWYQQRPGQSPRLLIYRSSTLESGVPDRFSGSGSGTDFTLKISRVEAEDVGVYYCQQNYDVPWTFGQGTKVEIK | 9 |
| PD-1 VL (3) | EIVLTQSPATLSLSPGERATLSCRASESVDTSDNSFIHWYQQKPGQSPRLLIYRSSTLESGIPARFSGSGSGTDFTLTISSLEPEDFAVYYCQQNYDVPWTFGQGTKVEIK | 10 |
| PD-1 VL (4) | EIVLTQSPATLSLSPGERATLSCRASESVDTSDNSFIHWYQQKPGQSPRLLIYRSSTLESGIPARFSGSGSGTDFTLTISSLEPEDFAVYYCQQNYDVPWTFGQGTKVEIK | 11 |
| Tim-3 HVR-H1 | GFNIKTT | 12 |
| Tim-3 HVR-H2 | ADD | 13 |

(continued)

| | Amino Acid Sequence | SEQ ID NO |
|---|---|---|
| Tim-3 HVR-H3 | FGYVAWFA | 14 |
| Tim-3 HVR-L1 | SQSVDNY | 15 |
| Tim-3 HVR-L2 | YAS | 16 |
| Tim-3 HVR-L3 | HYSSPY | 17 |
| Tim-3 VH (1) | EVQLVESGGGLVQPGGSLRLSCAASGFNIKTTYMHWVRQ APGKGLEWVGRIDPADDNTKYAPKFQGKATISADTSKNT AYLQMNSLRAEDTAVYYCVRDFGYVAWFAYWGQGTLV TVSS | 18 |
| Tim-3 VL (1) | DIVMTQSPLSLPVTPGEPASISCRASQSVDNYVAWYLQKP GQSPQLLIYYASNRYIGVPDRFSGSGSGTDFTLKISRVEAE DVGVYYCQQHYSSPYTFGQGTKVEIK | 19 |
| Tim-3 VH (2) | EVQLVESGGGLVQPGGSLRLSCAASGFNIKTTYMHWVRQ APGKGLEWVGRIDPADDNTKYAPKFQGKATISADTSKNT AYLQMNSLRAEDTAVYYCVRDFGYVAWFAYWGQGTLV TFSS | 20 |
| Tim-3 VL (2) | DIVMTQSPLSLPVTPGEPASISCRASQSVDNYVAWYLQKP GQSPQLLIYYASNRYIGVPDRFSGSGSGTDFTLKISRVEAE DVGVYYCQQHYSSPYTFGQGTKVEIK | 21 |
| Human IL-2 | APTSSSTKKTQLQLEHLLLDLQMILNGINNYKNPKLTRML TFKFYMPKKATELKHLQCLEEELKPLEEVLNLAQSKNFHL RPRDLISNINVIVLELKGSETTFMCEYADETATIVEFLNRW ITFCQSIISTLT | 22 |
| Human IL-2 (T3A, F42A, Y45Y, L72G, C125A) | APASSSTKKTQLQLEHLLLDLQMILNGINNYKNPKLTRML TAKFAMPKKATELKHLQCLEEELKPLEEVLNGAQSKNFH LRPRDLISNINVIVLELKGSETTFMCEYADETATIVEFLNR WITFAQSIISTLT | 23 |
| linker | GGGGSGGGGSGGGGS | 24 |

(continued)

| | Amino Acid Sequence | SEQ ID NO |
|---|---|---|
| PD1 Tim3 IL2v - PD1 HC with IL2v (VL-CH1, Fc knob LALAPG) | DIVMTQSPDSLAVSLGERATINCKASESVDTSDNSFIHWY QQKPGQSPKLLIYRSSTLESGVPDRFSGSGSGTDFTLTISSL QAEDVAVYYCQQNYDVPWTFGQGTKVEIKSSASTKGPSV FPLAPSSKSTSGGTAALGCLVKDYFPEPVTVSWNSGALTS GVHTFPAVLQSSGLYSLSSVVTVPSSSLGTQTYICNVNHK PSNTKVDKKVEPKSCDKTHTCPPCPAPEAAGGPSVFLFPP KPKDTLMISRTPEVTCVVVDVSHEDPEVKFNWYVDGVEV HNAKTKPREEQYNSTYRVVSVLTVLHQDWLNGKEYKCK VSNKALGAPIEKTISKAKGQPREPQVYTLPPCRDELTKNQ VSLWCLVKGFYPSDIAVEWESNGQPENNYKTTPPVLDSD GSFFLYSKLTVDKSRWQQGNVFSCSVMHEALHNHYTQK SLSLSPGGGGGSGGGGSGGGGSAPASSSTKKTQLQLEHLL LDLQMILNGINNYKNPKLTRMLTAKFAMPKKATELKHLQ CLEEELKPLEEVLNGAQSKNFHLRPRDLISNINVIVLELKG SETTFMCEYADETATIVEFLNRWITFAQSIISTLT | 25 |
| PD1 Tim3 IL2v - Tim3 HC (VH-CH1 (EE), Fc hole LALAPG) | evqlvesgggglvqpggslrlscaasgfnikttymhwvrqapgkglewvgridpaddnt kyapkfqgkatisadtskntaylqmnslraedtavyycvrdfgyvawfaywgqgtlvt vssastkgpsvfplapsskstsggtaalgclvedyfpepvtvswnsgaltsgvhtfpavlq ssglyslssvvtvpssslgtqtyicnvnhkpsntkvdekvepkscdkthtcppcpapeaa ggpsvflfppkpkdtlmisrtpevtcvvvdvshedpevkfnwyvdgvevhnaktkpr eeqynstyrvvsvltvlhqdwlngkeykckvsnkalgapiektiskakgqprepqvctl ppsrdeltknqvslscavkgfypsdiavewesngqpennykttppvldsdgsfflvsklt vdksrwqqgnvfscsvmhealhnhytqkslslsp | 26 |
| PD1 Tim3 IL2v - PD1 LC (VH-CL) | evqllesgggglvqpggslrlscaasgfsfssytmswvrqapgkglewvatisgggrdiyy pdsvkgrftisrdnskntlylqmnslraedtavyycvlltgrvyfaldswgqgtlvtvssas vaapsvfifppsdeqlksgtasvvcllnnfypreakvqwkvdnalqsgnsqesvteqds kdstyslsstltlskadyekhkvyacevthqglsspvtksfnrgec | 27 |
| PD1 Tim3 IL2v - Tim3 LC (VL-CL (RK)) | divmtqsplslpvtpgepasiscrasqsvdnyvawylqkpgqspqlliyyasnryigvp drfsgsgsgtdftlkisrveaedvgvyycqqhysspytfgqgtkveikrtvaapsvfifpp sdrklksgtasvvcllnnfypreakvqwkvdnalqsgnsqesvteqdskdstyslsstltl skadyekhkvyacevthqglsspvtksfnrgec | 28 |
| PD-1 IL2v-HC with IL2v (Fc knob, LALAPG) | evqllesgggglvqpggslrlscaasgfsfssytmswvrqapgkglewvatisgggrdiyy pdsvkgrftisrdnskntlylqmnslraedtavyycvlltgrvyfaldswgqgtlvtvssas tkgpsvfplapsskstsggtaalgclvkdyfpepvtvswnsgaltsgvhtfpavlqssgly slssvvtvpssslgtqtyicnvnhkpsntkvdkkvepkscdkthtcppcpapeaaggps vflfppkpkdtlmisrtpevtcvvvdvshedpevkfnwyvdgvevhnaktkpreeqy nstyrvvsvltvlhqdwlngkeykckvsnkalgapiektiskakgqprepqvytlppcr deltknqvslwclvkgfypsdiavewesngqpennykttppvldsdgsfflyskltvdk srwqqgnvfscsvmhealhnhytqkslslspggggggsggggsggggsapassstkktq lqlehllldlqmilnginnyknpkltrmltakfampkkatelkhlqcleeelkpleevlng aqsknfhlrprdlisninvivlelkgsettfmceyadetativeflnrwitfaqsiistlt | 29 |

(continued)

| | Amino Acid Sequence | SEQ ID NO |
|---|---|---|
| PD-1 IL2v-HC without IL2v (Fc hole, LALAPG) | evqllesgggglvqpggsslrlscaasgfsfssytmswvrqapgkglewvatisggggrdiyy pdsvkgrftisrdnskntlylqmnslraedtavyycvlltgrvyfaldswgqgtlvtvssas tkgpsvfplapsskstsggtaalgclvkdyfpepvtvswnsgaltsgvhtfpavlqssgly slssvvtvpssslgtqtyicnvnhkpsntkvdkkvepkscdkthtcppcapeaaggps vflfppkpkdtlmisrtpevtcvvvdvshedpevkfnwyvdgvevhnaktkpreeqy nstyrvvsvltvlhqdwlngkeykckvsnkalgapiektiskakgqprepqvctlppsr deltknqvslscavkgfypsdiavewesngqpennykttppvldsdgsfflvskltvdks rwqqgnvfscsvmhealhnrftqkslslsp | 30 |
| PD-1 IL2v - LC | divmtqspdslavslgeratinckasesvdtsdnsfihwyqqkpgqspklliyrsstlesg vpdrfsgsgsgtdftltisslqaedvavyycqqnydvpwtfgqgtkveikrtvaapsvfif ppsdeqlksgtasvvcllnnfypreakvqwkvdnalqsgnsqesvteqdskdstyslsst ltlskadyekhkvyacevthqglsspvtksfnrgec | 31 |
| hIL-2 signal peptide | MYRMQLLSCIALSLALVTNS | 32 |
| hPD-1 (without signal sequence ) | PGWFLDSPDRPWNPPTFSPALLVVTEGDNATFTCSFSNTS ESFVLNWYRMSPSNQTDKLAAFPEDRSQPGQDCRFRVTQ LPNGRDFHMSVVRARRNDSGTYLCGAISLAPKAQIKESLR AELRVTERRAEVPTAHPSPSRPAGQFQTLVVGVVGGLLG SLVLLVWVLAVICSRAARGTIGARRTGQPLKEDPSAVPVF SVDYGELDFQWREKTPEPPVPCVPEQTEYATIVFPSGMGT SSPARRGSADGPRSAQPLRPEDGHCSWPL | 33 |
| hPD-1 (with signal sequence ) | MQIPQAPWPVVWAVLQLGWRPGWFLDSPDRPWNPPTFS PALLVVTEGDNATFTCSFSNTSESFVLNWYRMSPSNQTDK LAAFPEDRSQPGQDCRFRVTQLPNGRDFHMSVVRARRND SGTYLCGAISLAPKAQIKESLRAELRVTERRAEVPTAHPSP SPRPAGQFQTLVVGVVGGLLGSLVLLVWVLAVICSRAAR GTIGARRTGQPLKEDPSAVPVFSVDYGELDFQWREKTPEP PVPCVPEQTEYATIVFPSGMGTSSPARRGSADGPRSAQPL RPEDGHCSWPL | 34 |
| hPD-1 Extracellular Domain (ECD) | PGWFLDSPDRPWNPPTFSPALLVVTEGDNATFTCSFSNTS ESFVLNWYRMSPSNQTDKLAAFPEDRSQPGQDCRFRVTQ LPNGRDFHMSVVRARRNDSGTYLCGAISLAPKAQIKESLR AELRVTERRAEVPTAHPSPSRPAGQFQTLV | 35 |
| hTim-3 (without signal sequence ) | SEVEYRAEVGQNAYLPCFYTPAAPGNLVPVCWGKGACP VFECGNVVLRTDERDVNYWTSRYWLNGDFRKGDVSLTI ENVTLADSGIYCCRIQIPGIMNDEKFNLKLVIKPAKVTPAP TRQRDFTAAFPRMLTTRGHGPAETQTLGSLPDINLTQISTL ANELRDSRLANDLRDSGATIRIGIYIGAGICAGLALALIFG ALIFKWYSHSKEKIQNLSLISLANLPPSGLANAVAEGIRSE ENIYTIEENVYEVEEPNEYYCYVSSRQQPSQPLGCRFAMP | 36 |

(continued)

| | Amino Acid Sequence | SEQ ID NO |
|---|---|---|
| hTim-3 (with signal sequence ) | MFSHLPFDCVLLLLLLLLTRSSEVEYRAEVGQNAYLPCFY TPAAPGNLVPVCWGKGACPVFECGNVVLRTDERDVNYW TSRYWLNGDFRKGDVSLTIENVTLADSGIYCCRIQIPGIMN DEKFNLKLVIKPAKVTPAPTRQRDFTAAFPRMLTTRGHGP AETQTLGSLPDINLTQISTLANELRDSRLANDLRDSGATIRI GIYIGAGICAGLALALIFGALIFKWYSHSKEKIQNLSLISLA NLPPSGLANAVAEGIRSEENIYTIEENVYEVEEPNEYYCYV SSRQQPSQPLGCRFAMP | 37 |
| hTim-3 Extracellular Domain (ECD) | SEVEYRAEVGQNAYLPCFYTPAAPGNLVPVCWGKGACP VFECGNVVLRTDERDVNYWTSRYWLNGDFRKGDVSLTI ENVTLADSGIYCCRIQIPGIMNDEKFNLKLVIKPAKVTPAP TRQRDFTAAFPRMLTTRGHGPAETQTLGSLPDINLTQISTL ANELRDSRLANDLRDSGATIRIG | 38 |
| Human IL-2 (C125A) | APTSSSTKKTQLQLEHLLLDLQMILNGINNYKNPKLTRML TFKFYMPKKATELKHLQCLEEELKPLEEVLNLAQSKNFHL RPRDLISNINVIVLELKGSETTFMCEYADETATIVEFLNRW ITFAQSIISTLT | 39 |
| Human IgG1 Fc domain | DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSP | 40 |
| Human kappa CL domain | RTVAAPSVFIFPPSDEQLKSGTASVVCLLNNFYPREAKVQ WKVDNALQSGNSQESVTEQDSKDSTYSLSSTLTLSKADY EKHKVYACEVTHQGLSSPVTKSFNRGEC | 41 |
| Human lambda CL domain | QPKAAPSVTLFPPSSEELQANKATLVCLISDFYPGAVTVA WKADSSPVKAGVETTTPSKQSNNKYAASSYLSLTPEQWK SHRSYSCQVTHEGSTVEKTVAPTECS | 42 |
| Human IgG1 heavy chain constant region (CH1-CH2-CH3) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSR DELTKNQVSLTCLVKGFYPSDIAVEWESNGQPENNYKTT PPVLDSDGSFFLYSKLTVDKSRWQQGNVFSCSVMHEALH NHYTQKSLSLSP | 43 |

## Examples

The following are examples of methods and compositions of the invention. They illustrate the invention, but are not limiting its scope, which is defined by the claims.

## Example 1

### Cloning and Expression of human PD1-Tim3-IL-2v

Expression vectors for the PD1-Tim3-ILv construct (SEQ ID NOs 25, 26, 27 and 28) were prepared as described in PCT patent application no. PCT/EP2016/073192.

The human anti-PD1-Tim3-IL-2v construct was generated by co-transfection of HEK293F cells (Invitrogen) with the appropriate plasmids in the plasmid ratio of 1:1:1:1 in shaking flasks. After 1 week supernatant was harvested and filtrated through sterile filters and purified by standard methods (see e.g. PCT patent application no. PCT/EP2016/073192). Briefly, purification from supernatant was done by a combination of Protein A affinity chromatography and size exclusion chromatography. The obtained products were characterized for identity by mass spectrometry and analytical properties such as purity by capillary electrophoresis (CE-SDS), monomer content and stability. The immunoconjugate could be produced in good yields and was stable.

## Example 2

### Example 2A. Binding of PD1-TIM3-IL2v to activated CD8 and CD4 T cells

Freshly isolated PBMCs from healthy donors were stimulated overnight with CD3 and CD28 to induce upregulation of PD1 on T cells. PBMCs were seeded in medium (RPMI1640, 10% FCS, 2 mM Glutamine) into cell culture flasks that were coated for 1 h at 37°C with 1 $\mu$g/ml CD3 (clone OKT3, 317304, BioLegend). CD28 was added in solution to the PBMCs at a concentration of 2 $\mu$g/ml (clone CD28.2, 302914, BioLegend). On the next day PBMCs were harvested and transferred into a 96 well round bottom plate (200'000 cells per well). The cells were washed with FACS buffer (PBS, 2% FBS, 5 mM EDTA, 0.025% NaN$_3$) and stained with 40 $\mu$l of the indicated molecules (PD1-TIM3-IL2v, PD1 IgG, CEA-IL2v) in FACS buffer for 30 min at 4°C. The cells were washed twice with FACS buffer to remove unbound molecules. Then 40 $\mu$l of the diluted PE anti-human Fc specific secondary antibody (109-116-170, Jackson ImmunoResearch) was added to the cells. After 30 min incubation at 4°C the cells were washed twice with FACS buffer. To detect T cells, PBMCs were stained with 40 $\mu$l of a mixture of CD3 FITC (clone UCHT1, 300406, BioLegend), CD4 APC (clone RPA-T4, 300514, BioLegend) and CD8 BV421 (clone RPA-T8, 301036, BioLegend) for 30 min at 4°C. The unbound antibodies were removed by washing twice with FACS buffer. Finally the cells were fixed with 1% PFA in FACS buffer and measured using a BD Fortessa gating on CD3+CD4+ cells (CD4 T cells) and CD3+CD8+ cells (CD8 T cells).

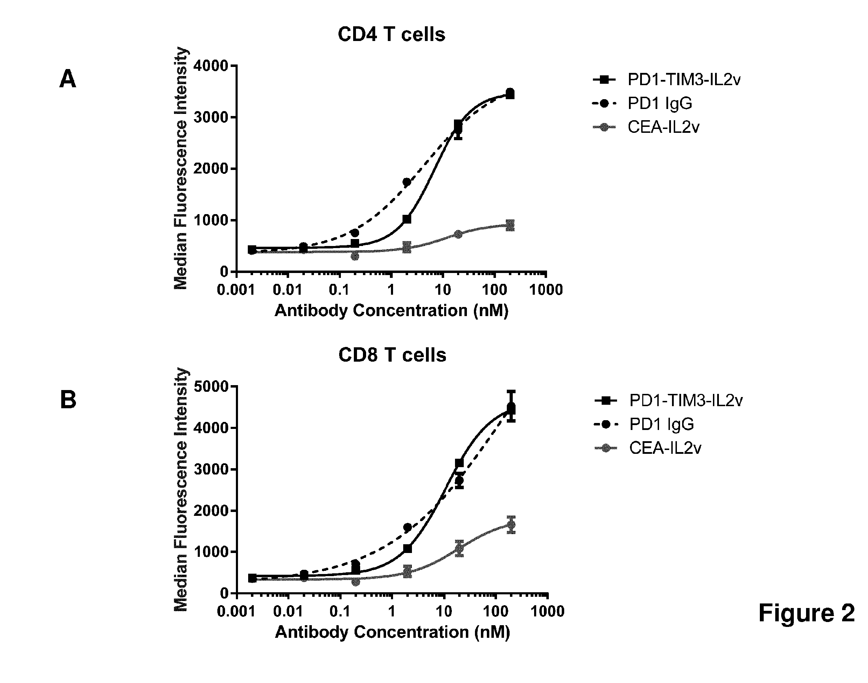

**Figure 2** shows that PD1-TIM3-IL2v and a corresponding PD1 IgG bind similarly to CD4 and CD8 T cells. An analogous fusion protein targeted to CEA, CEA-IL2v, was added as an untargeted control to compare binding of IL2v alone to T cells versus binding of PD1-TIM3-IL2v to T cells.

### Example 2B. Proliferation of NK92 with PD1-TIM3-IL2v

NK92 cells were harvested, counted and assessed for viability. Cells were washed three times with PBS to remove residual IL-2 and were re-suspended in medium (RPMI1640, 10% FCS, 1% Glutamine) without IL-2. The washed NK92 cells were incubated for two hours in cell incubator (IL-2 starvation). After starvation, cells were re-suspended in fresh medium without IL-2 to 200'000 cells per ml and 50 $\mu$l of the cell suspension was transferred in a 96-well cell culture treated flat bottom plate and supplemented with 50 $\mu$l of the diluted molecules PD1-Tim3-IL2v (in medium without IL-2), Proleukin (1.5 $\mu$g/ml final concentration) or medium (control wells) to reach a final volume of 100 $\mu$l per well. The plate was incubated for 2 days in the incubator.

After 2 days the CellTiter-Glo (Promega) reagents and the cell culture plate were equilibrated to room temperature. The CellTiter-Glo solution was prepared as described in the manufacturer's instructions and 100 $\mu$l of the solution was added to each well. After 10 min of incubation remaining aggregates were re-suspended by pipetting and 150 $\mu$l of the mixture was transferred to a white flat bottom plate. The luminescence was measured with Tecan Spark 10M multimode reader.

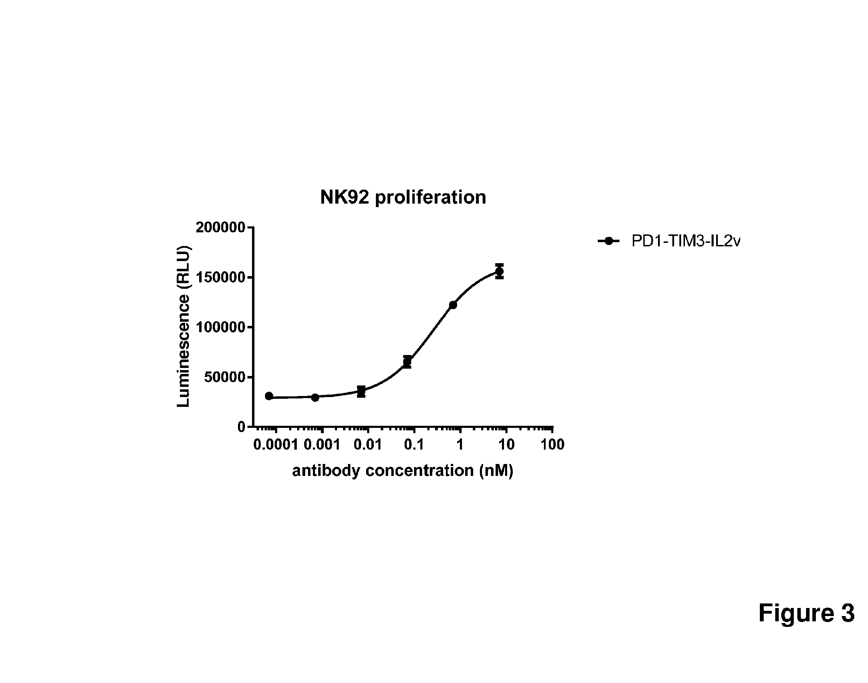

**Figure 3** shows that PD1-TIM3-IL2v is able to induce proliferation of NK92 cells in a concentration dependent manner.

**Example 3**

**Effect of IL-2v delivery to exhausted virus-specific T cells through either PD-1 or PD-1 and TIM-3 blockade**

Recently TIM-3, an additional immune-checkpoint, has been identified on the surface of those PD-1-positive virus-specific T cells bearing a greater degree of dysfunction than PD-1 single positive ones. We therefore assessed the effect of PD-1 and TIM-3 co-targeting to deliver IL-2v to highly dysfunctional virus-specific T cells.

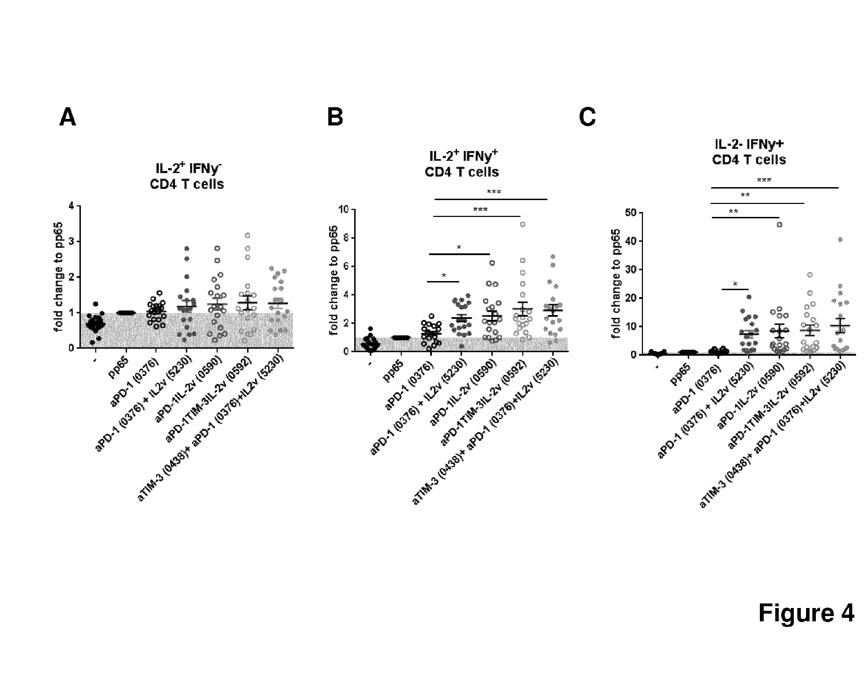

The results, shown in **Figure 4,** highlight the combined effect of targeting PD-1 and TIM-3 to deliver IL-2v to virus-specific T cells versus PD-1 targeting alone. Interestingly the PD1-TIM3-IL-2v construct highly significantly increases the frequencies of protective CMV-specific CD4 T cells able to co-secrete IL-2 and IFN-$\gamma$ **(Figure 4B,** $p \leq 0.001$,) as well IFN-$\gamma$ alone **(Figure 4C,** $p \leq 0.01$) when compared to PD-1 blockade alone. PD1-IL2v (an analogous molecule targeting only PD-1; see SEQ ID NOs 29, 30 and 31) shows a similar trend to the PD1-TIM3-IL2v in significantly expanding protective polyfunctional as well as IFN-$\gamma$-single secreting CMV-specific CD4 T cells when compared to PD-1 blockade alone **(Figure 4B,** $p \leq 0.05$ and **Figure 4C,** $p \leq 0.01$). Both PD-1-IL-2v and PD-1-TIM-3-IL-2v did not significantly increase the frequencies of IL-2-single secreting CD4 T cells.

The ability of PD1-TIM3-IL2v to expand/enhance the effector functions of protective virus-specific polyfunctional T cells is a relevant feature for potential applications of this molecule to target exhausted/dysfunctional antigen-specific T cells in chronic infections as well as in cancer.

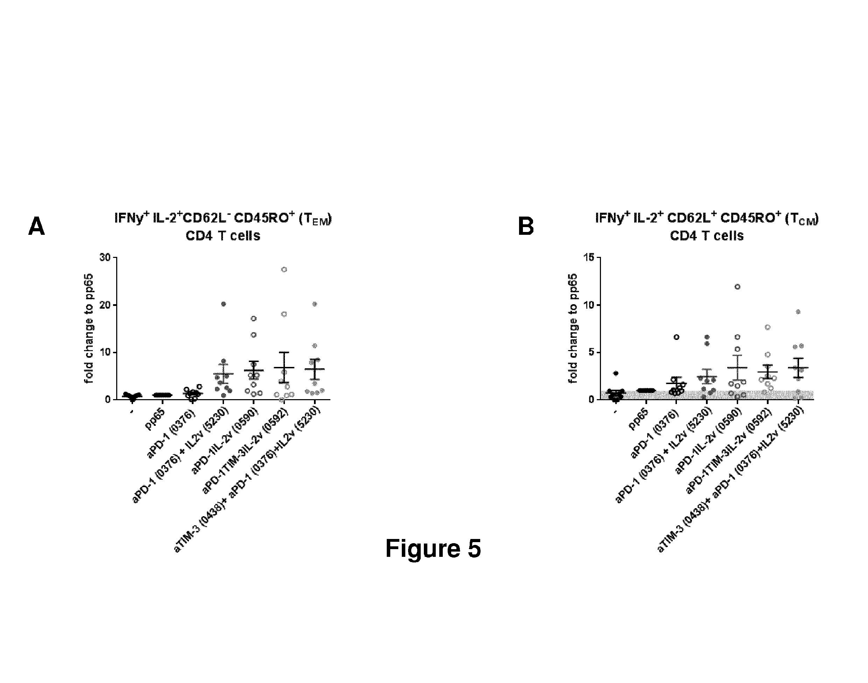

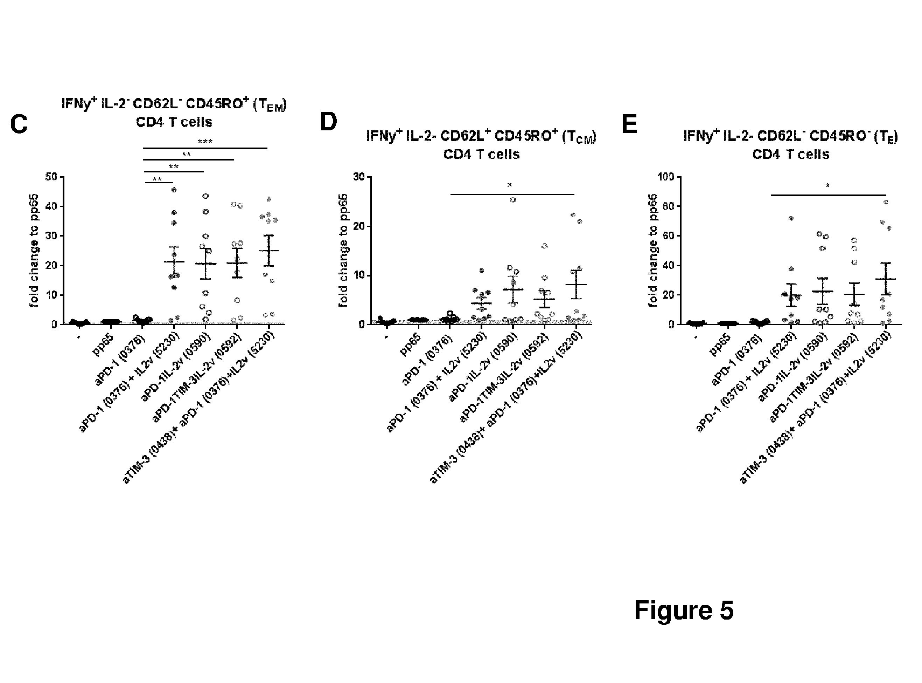

Since polyfunctional CD4 T cells have been described as endowed with an intrinsic proliferation capacity and therefore with the ability to self-maintain over a life time, we characterized their differentiation state upon pp65 re-stimulation in presence of the different constructs. Interestingly, we observed a trend of increase in the effector memory (CD45RO$^+$ CD62L$^-$) and central memory pool (CD45RO$^+$ CD62L$^+$) within the polyfunctional CD4 T cells upon treatment with PD1-TIM3-IL2v and PD1-IL2v **(Figure 5A** and **B).**

Single IFN-$\gamma$ secreting CD4 T cells have been described to derive from the polyfunctional counterpart, to be more differentiated and, hence, to have lost their ability to proliferate. In this subpopulation of CMV-specific CD4 T cells we not only noticed an increase in frequencies of effector and central memory cells **(Figure 5C,** $p \leq 0.01$ and **Figure 5D),** but also of terminally differentiated effectors (CD45RO$^-$ CD62L$^-$) **(Figure 5C)** upon treatment with PD1-TIM3-IL2v and PD1-IL2v.

Taken all together, the data show that PD1-TIM3-IL2v increases the frequencies of life-long protective virus-specific polyfunctional T cells and allows also the expansion of those virus-specific effector CD4 T cells which lack the intrinsic ability to proliferate.

**Example 4**

**Effect of IL-2v delivery to exhausted virus-specific T cells through either PD-1 or PD-1 and TIM-3 blockade**

PD-1 expression has been described for the first time on exhausted virus-specific T cells as result of chronic-exposure to viral antigens and it has been associated with T-cell inability to mount an effective anti-viral response. Virus-specific CD4 T cells able to simultaneously secrete IL-2 and IFN-$\gamma$ confer protection from viral re-activation in chronic infections. Indeed, the polyfunctional signature of CD4 T cells has been associated with viral-control in healthy individuals infected by Cytomegalovirus (CMV), Epstein-Barr virus (EBV) and Herpes Simplex virus (HSV) as well as in those individuals infected with Human Immunodeficiency virus (HIV), who remain symptoms-free for several years.

Recently TIM-3, an additional immune-checkpoint, has been identified on the surface of those PD-1-positive virus-specific T cells bearing a greater degree of dysfunction than PD-1 single positive ones. Therefore, an in-vitro assay was developed to evaluate the effect of PD-1 and TIM-3 targeting to deliver a mutated version of IL-2 (IL-2v) to dysfunctional antigen-specific T cells. To avoid restrictions on the amount of suitable donors for the assay, it was opted for a CMV immunogenic viral-protein (pp65) as re-call antigen for T cells given that roughly 80% of the population is CMV-seropositive. Hence, healthy human donor peripheral blood mononuclear cells (PBMCs) were stimulated with CMV-pp65 (Miltenyi) in presence of the constructs at the concentration of 10 $\mu$g/ml. 43 hours later the protein transport from the Golgi was blocked by adding Golgi Plug (BD Bioscience, Brefeldin A) and Golgi Stop (BD Bioscience, Monensin) and the cells were incubated at 37°C for additional 5 hours. The cells were then washed, stained on the surface with anti-human CD3, CD4, CD8, CD62L and CD45RO antibodies before being fixed/permeabilized with the FoxP3 Transcription FactorStaining Buffer Set (eBioscience). At last, intracellular staining for IL-2, IFN-$\gamma$ and Ki67 (both from eBioscience) was performed.

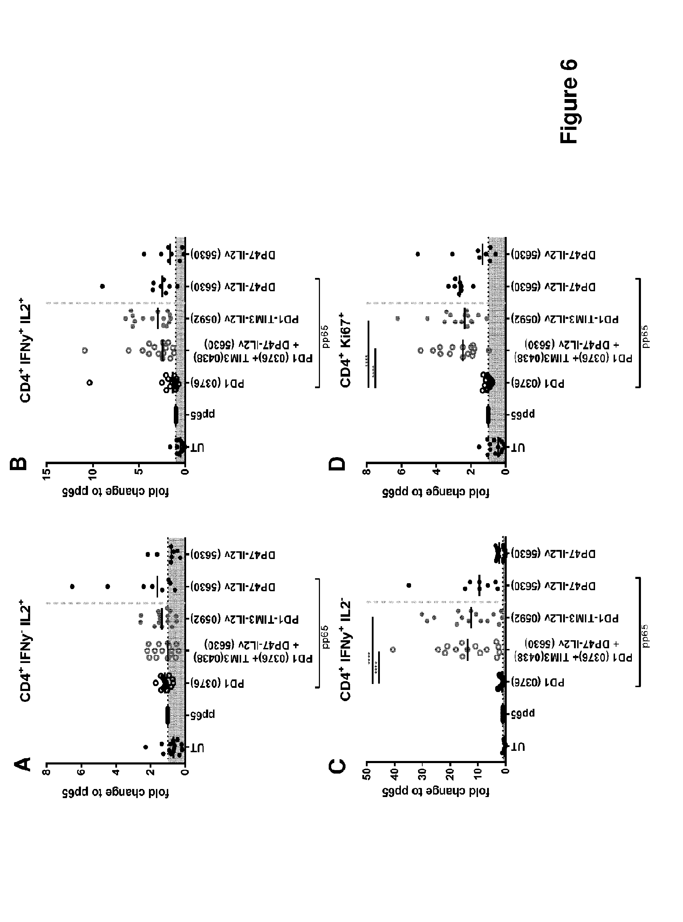

Figure 6 shows the ability of CD4 T cells to secrete IL-2 (A), IL-2 and IFN-$\gamma$ (B) or IFN-$\gamma$ (C) and to proliferate (D) upon 48 hours recall with CMV immunogenic protein pp65 in presence of either anti-PD-1 alone, in combination with TIM-3 and IL-2v, or as fusion protein. The results, shown in Figures 6, highlight the combined effect of blocking PD-1 and TIM-3 while delivering IL-2v to virus-specific T cells versus either PD-1 blockade alone or untargeted IL-2v. Interestingly, the PD1-TIM3-IL-2v construct increased the frequencies of CMV-specific CD4 T cells able to co-secrete IL-2 and IFN-γ (Figure 6B) as well as single IFN-γ (Figure 6C, P≤0.0001) when compared to PD-1 blockade alone. Conversely, DP47-IL2v increased the frequencies of IL-2 mono-functional CD4 T cells (Figure 6A). As expected all cells treated with targeted or untargeted IL-2v proliferated as indicated by the positivity to Ki67 staining (Figure 6D).

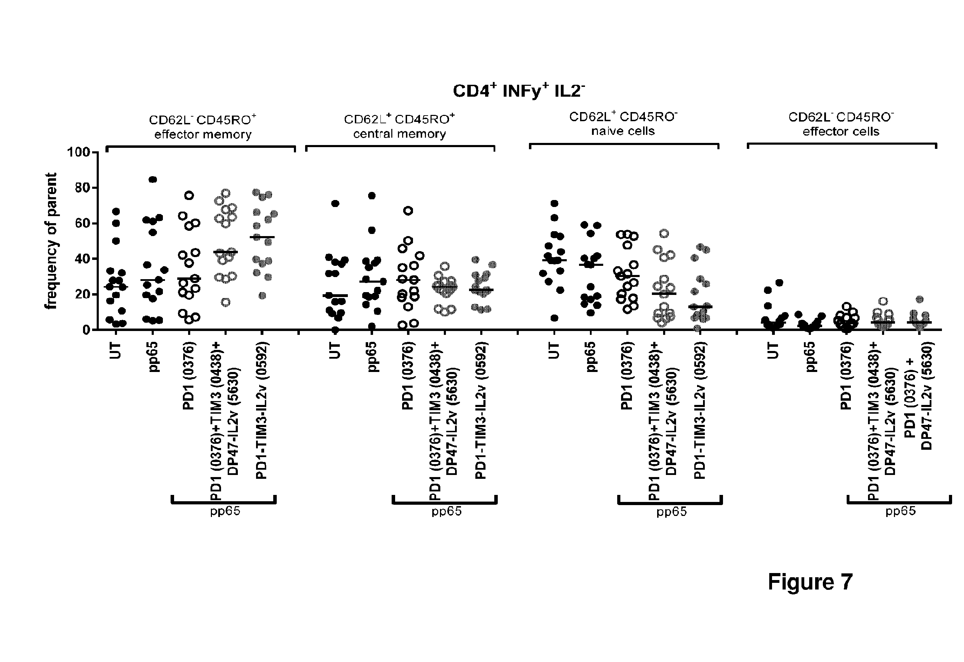

Figure 7 shows the differentiation state, as per expression of CD45RO and CD62L, of virus-specific CD4 T cells secreting IFN-γ upon 48 hours recall with CMV immunogenic protein pp65 in presence of either anti-PD-1 alone, in combination with TIM-3 and IL-2v, or as fusion protein. The phenotype characterization of the expanded IFN-γ-secreting virus-specific CD4 T cells (Figure 7) revealed an effector-memory (CD45RO$^+$CD62L$^-$) profile.

It can thus be concluded that delivering IL-2v to the exhausted CMV-specific CD4 T cells through the PD1-TIM3-IL2v fusion protein resulted in the expansion of a long-lived protective virus-specific population characterized by a differentiated memory profile and the ability to secrete both IL-2 and IFN-γ. This is a relevant feature for potential applications of this molecule to target exhausted/dysfunctional antigen-specific T cells in chronic infections as well as in cancer.

## Example 5

### Example 5A. Preferential binding of PD1-TIM3-IL2v to activated conventional T cells over activated regulatory T cells

The binding properties of PD1-TIM3-IL2v to activated conventional and regulatory Tcells were assessed in a competitive binding assay. CD4$^+$ CD25$^+$ CD127$^{dim}$ Regulatory T cells (Treg) were isolated with the two-step Regulatory T cell Isolation Kit (Miltenyi, #130-094-775). In parallel the CD4$^+$ CD25$^-$ conventional T cells (Tconv) were isolated by collecting the negative fraction of a CD25 positive selection (Miltenyi, #130-092-983) followed by a CD4$^+$ enrichment (Miltenyi, #130-045-101). The Tconv were labelled with CFSE (eBioscience, #65-0850-84) and the Treg were labelled with Cell Trace Violet (ThermoFisher scientific, C34557) to track the proliferation of both populations. Tconv and Treg were together seeded into a culture plate that were coated overnight at 4°C with 1 μg/ml CD3 (clone OKT3, #317315, BioLegend). CD28 was added in solution at a concentration of 1 μg/ml CD28 (clone CD28.2, #302923, BioLegend). After 5 days of stimulation a binding assay was conducted with PD1 (0376) and PD1-TIM3-IL2v (0592), which were both labelled in-house with AF647.

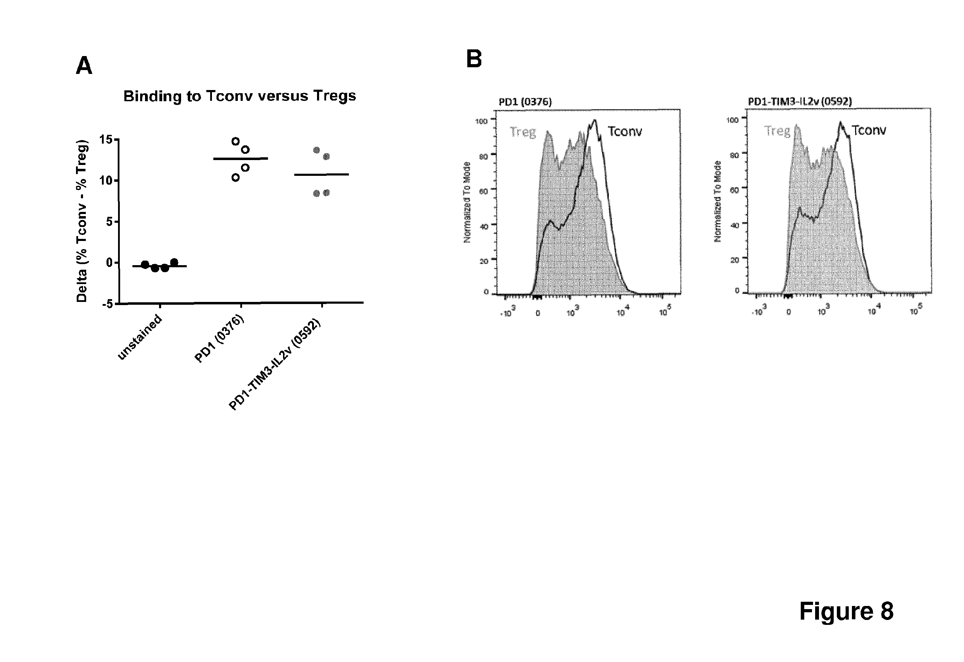

Figure 8A shows the Delta of the frequency of a given antibody bound on Tconv versus Treg within the same sample, wherein each symbol represents a separate donor, horizontal lines indicate medians with N=4. Figure 8B shows data from one representative donor showing the binding to Tconv (black line) and Treg (grey). The PD1-TIM3-IL2v trispecific antibody shows a comparable binding profile as PD1 (Figure 8A and 8B). Both molecules show higher binding capacity to Tconv over Treg due to higher expression levels of PD-1 on Tconv than on Treg. Hence the PD1-IL2v bispecific antibody maintains the binding properties of PD1 despite the IL2v being coupled to the antibody.

### Example 5B. Rescue of Tconv effector function upon PD1-TIM3-IL2v treatment in Treg suppression assay

In a next step it was tested, if the PD1-TIM3-IL2v can reverse the Treg suppression of Tconv. We therefore established a suppressive-function assay where Tconv and Treg are cultured together for 5 days, with or without blocking antibodies, in presence of CD4$^-$ CD25$^-$ from an unrelated donor for allospecific stimulation. For this purpose Tconv and Treg were isolated and labelled as described above. The accumulation of cytokines in the Golgi complex was enhanced by applying Protein Transport Inhibitors (GolgiPlug #555029, BD and GolgiStop #554724, BD) for 5 hours prior to the FACS staining.

The ability of the proliferated Tconv to secrete granzyme B (GrzB) and interferon gamma (IFNγ) in presence and absence of Treg was measured. The Treg suppression was calculated with the following formula:

$$\% \textit{ cytokine suppression} = 100 - (\% \textit{ cytokine}_{Tconv+Treg\pm blocking\ antibody})/(\% \textit{ cytokine}_{Tconv\ untreated}) *100)$$

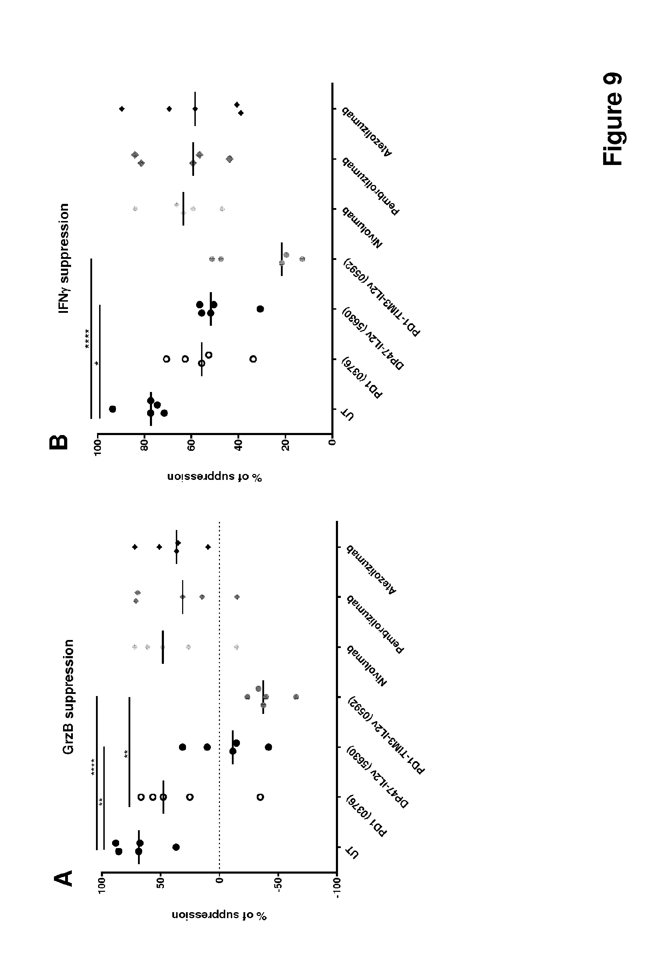

, where % cytokine$_{(Tconv+Treg\pm blocking\ antibody)}$ % cytokine$_{Tconv+Treg\pm blocking\ antibody}$ is the level of cytokine secreted by Tconv in the presence of Treg ± blocking antibody, % cytokine$_{Tconv\ untreated}$ % cytokine$_{(Tconv\ untreated)}$ is the level of cytokine secreted by Tconv in the absence of Treg. In Figure 9, the percentage of suppression by Tregs of granzyme B (Figure 9A) and interferon-γ (Figure 9B) secreted by Tconv after 5 days of coculture is shown. Each symbol represents a separate donor, horizontal lines indicate medians with N=5, dotted lines at 0% represents no suppression by Treg. P was calculated using one-way ANOVA (*p<0.05, **p<0.01, ***p<0.001, ****p<0.0001).

Treatment with the PD1 antibody (0376) results in a median of 47.7% of Tconv function suppression, compared to a median of 68.6 % suppression in the untreated group (no statistical significance). Likewise the blocking of PD-1/PD-L1 interaction with Atezolizumab, Nivolumab and Pembrolizumab showed the same tendency as our in house PD1. Interestingly DP47-IL2v (median= -11.3%, p= 0.0011) and PD1-TIM3-IL2v (median= -37.3%, p < 0.0001) rescued Tconv GrzB effector function from Treg suppression. Furthermore PD1-TIM3-IL2v was even significantly (p=0.0016) more potent than the PD1 alone. In parallel, the same analysis were performed for INFy suppression of Tconv by Treg. DP47-IL2v (median= 51.77%, p= 0.0251) and PD1-TIM3-IL2v (median= 21.58%, p= 0.0001) rescued Tconv IFNy effector function from Treg suppression.

## Example 6

### Example 6A. Cell activation of donors 1 and 2 (pSTAT5 assay)

Freshly isolated PBMCs from healthy donors were seeded in warm medium (RPMI1640, 10% FCS, 2 mM Glutamine) into a 96 well round bottom plate (200'000 cells/well). The plates were centrifuged at 300 g for 10 min and the supernatant was removed. The cells were re-suspended in 50 $\mu$l medium containing the IL2 molecules and stimulated for 20 min at 37°C. To preserve the phosphorylation status, the cells were immediately fixed after stimulation with equal amount of pre-warmed Cytofix buffer (554655, BD Bioscience) for 10 min at 37°C. Afterwards the plates were centrifuged for 10 min at 300 g and the supernatant was removed. To allow intracellular staining, the cells were permeabilized in 200 $\mu$l Phosflow Perm buffer III (558050, BD Bioscience) for 30 min at 4°C. Then the cells were washed twice with 150 $\mu$l cold FACS buffer and split in two 96 well round bottom plates and stained each with 20 $\mu$l of the antibody mix I or II for 60 min in the fridge. Antibody mix I was used to stain pSTAT5 in CD4 T cells and regulatory T cells and antibody mix II was used to stain pSTAT5 in CD8 T cells and NK cells. Afterwards the cells were washed twice with FACS buffer and re-suspended in 200 $\mu$l FACS buffer containing 2 % PFA per well. The analysis was performed using a BD Fortessa flow cytometer.

The FACS antibody mixes according to table 1 and table 2 were used.

Table 1. FACS antibody mix I (CD4 T cells and regulatory T cells)

| Antibody | Volume/sample |
| --- | --- |
| CD4 PE/Cy7, clone SK3, mouse IgG1, $\kappa$ (557852, BD Bioscience) | 0.5 $\mu$l / well |
| CD25 APC, clone M-A251, mouse IgG1, $\kappa$ (356110, BioLegend) | 4 $\mu$l / well |
| PE Mouse anti-Human FoxP3 Clone 259D/C7 (560046, BD Bioscience) | 1 $\mu$l / well |
| A488 pSTAT5 (pY694), clone 47, mouse IgG1 (562075, BD Bioscience) | 1 $\mu$l / well |

Table 2. FACS antibody mix II (CD8 T cells and NK cells)

| Antibody | Volume/sample |
| --- | --- |
| CD3 PE/Cy7, clone UCHT1, mouse IgG1, $\kappa$ (300420, BioLegend) | 1 $\mu$l / well |
| CD56 APC, clone HCD56, mouse IgG1, $\kappa$ (318310, BioLegend) | 1 $\mu$l / well |
| CD8 PE, clone HIT8a, mouse IgG1 (555635, BD Bioscience) | 1 $\mu$l / well |
| A488 pSTAT5 (pY694), clone 47, mouse IgG1 (BD Bioscience) | 1 $\mu$l / well |

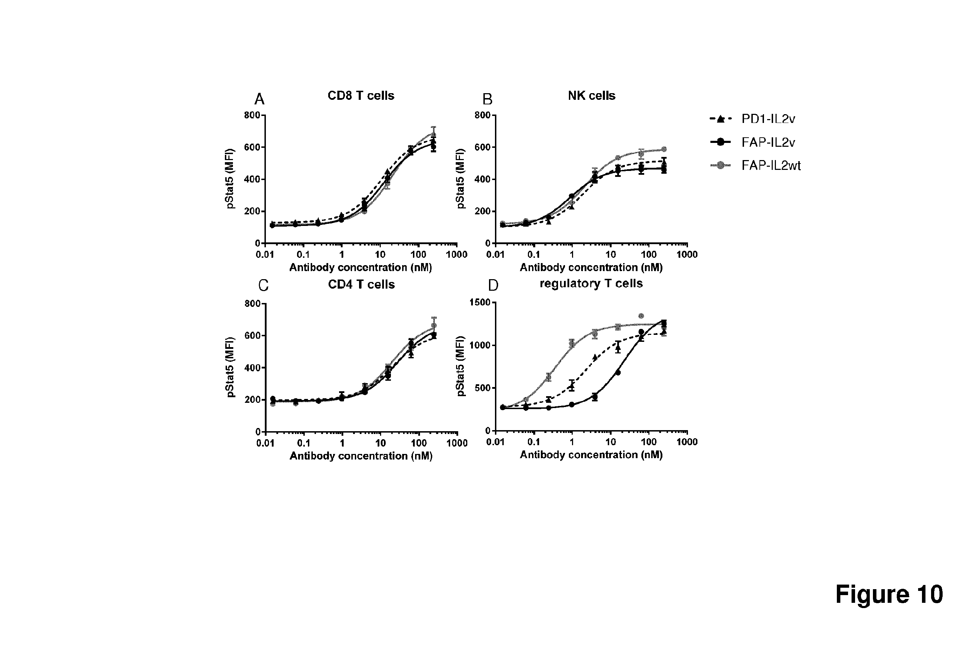

Figure 10 shows STAT5 phosphorylation in CD8 T-cells (A), NK cells (B), CD4 T-cells (C) and regulatory T-cells (D) upon treatment of resting PBMCs of donor 1 with PD1-IL2v, FAP-IL2v and FAP-IL2wt. All three tested molecules are equally potent on CD8 T-cells, NK cells and CD4 T-cells (excluding Tregs). FAP-IL2wt is more potent in inducing STAT5 phosphorylation in Tregs followed by PD1-IL2v. FAP-IL2v has the lowest activity on Tregs.

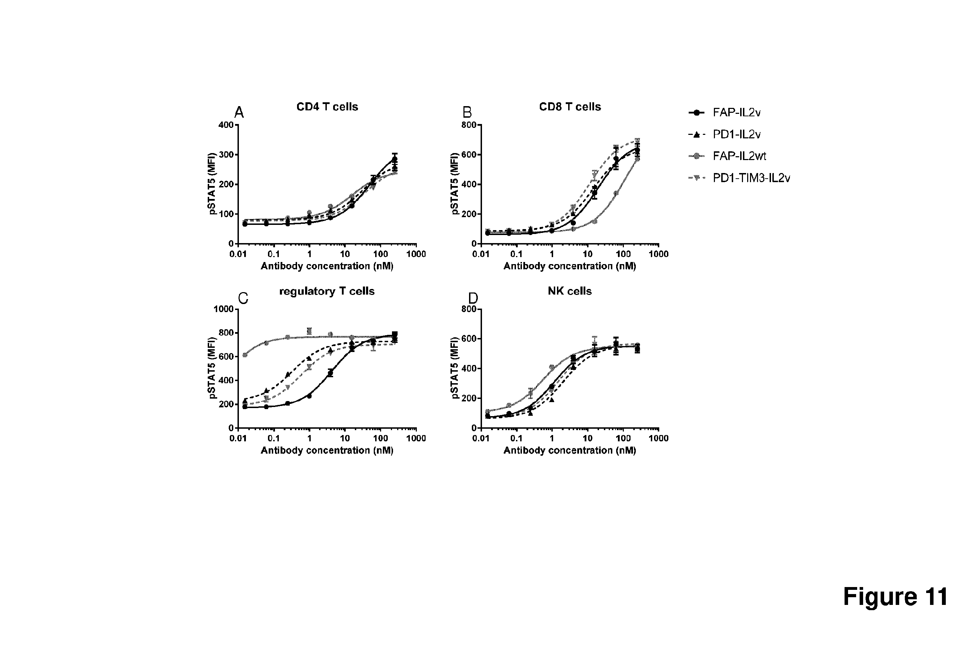

Figure 11 shows STAT5 phosphorylation in CD4 T-cells (A), CD8 T-cells (B), regulatory Tcells (C) and NK cells (D) upon treatment of resting PBMCs of donor 2 with FAP-IL2v, PD1-IL2c, FAP-IL2wt and PD1-TIM3-IL2v. All four tested molecules are comparable active on CD8 T-cells, NK cells and CD4 T-cells (excluding Tregs). FAP-IL2wt is more potent in inducing STAT5 phosphorylation in Tregs followed by PD1-EL2v. FAP-IL2v has the lowest activity on Tregs.

### Example 6B. Cell activation of donors 3 and 4 (pSTAT5 assay)

Frozen PBMCs isolated from healthy donors were thawed and cultured overnight at 37°C. On the next day the cells were seeded in warm medium (RPMI1640, 10% FCS, 2 mM Glutamine) into a 96 well round bottom plate (200'000 cells/well). The plates were centrifuged at 300 g for 10 min and the supernatant was removed. The cells were re-suspended in 50 µl medium containing the IL2 molecules and stimulated for 20 min at 37°C. To preserve the phosphorylation status, the cells were immediately fixed after stimulation with equal amount of pre-warmed Cytofix buffer (554655, BD Bioscience) for 10 min at 37°C. Afterwards the plates were centrifuged for 10 min at 300 g and the supernatant was removed. To allow intracellular staining, the cells were permeabilized in 200 µl Phosflow Perm buffer III (558050, BD Bioscience) for 30 min at 4°C. Then the cells were washed twice with 150 µl cold FACS buffer and split in two 96 well round bottom plates and stained each with 20 µl of the antibody mix I or II for 60 min in the fridge. Antibody mix I was used to stain pSTAT5 in CD4 T cells and regulatory T cells and antibody mix II was used to stain pSTAT5 in CD8 T cells and NK cells. Afterwards the cells were washed twice with FACS buffer and re-suspended in 200 µl FACS buffer containing 2 % PFA per well. The analysis was performed using a BD Fortessa flow cytometer. The FACS antibody mixes according to table 3 and table 4 were used.

Table 3. FACS antibody mix I (CD4 T cells and regulatory T cells)

| Antibody | Volume/sample |
|---|---|
| CD4 PE/Cy7, clone SK3, mouse IgG1, κ (557852, BD Bioscience) | 0.5 µl / well |
| CD25 APC, clone M-A251, mouse IgG1, κ (356110, BioLegend) | 4 µl / well |
| PE Mouse anti-Human FoxP3 Clone 259D/C7 (560046, BD Bioscience) | 1 µl / well |
| A488 pSTAT5 (pY694), clone 47, mouse IgG1 (562075, BD Bioscience) | 1 µl / well |

Table 4. FACS antibody mix II (CD8 T cells and NK cells)

| Antibody | Volume/sample |
|---|---|
| CD3 PE/Cy7, clone UCHT1, mouse IgG1, κ (300420, BioLegend) | 1 µl / well |
| CD56 APC, clone HCD56, mouse IgG1, κ (318310, BioLegend) | 1 µl / well |
| CD8 PE, clone HIT8a, mouse IgG1 (555635, BD Bioscience) | 1 µl / well |
| A488 pSTAT5 (pY694), clone 47, mouse IgG1 (BD Bioscience) | 1 µl / well |

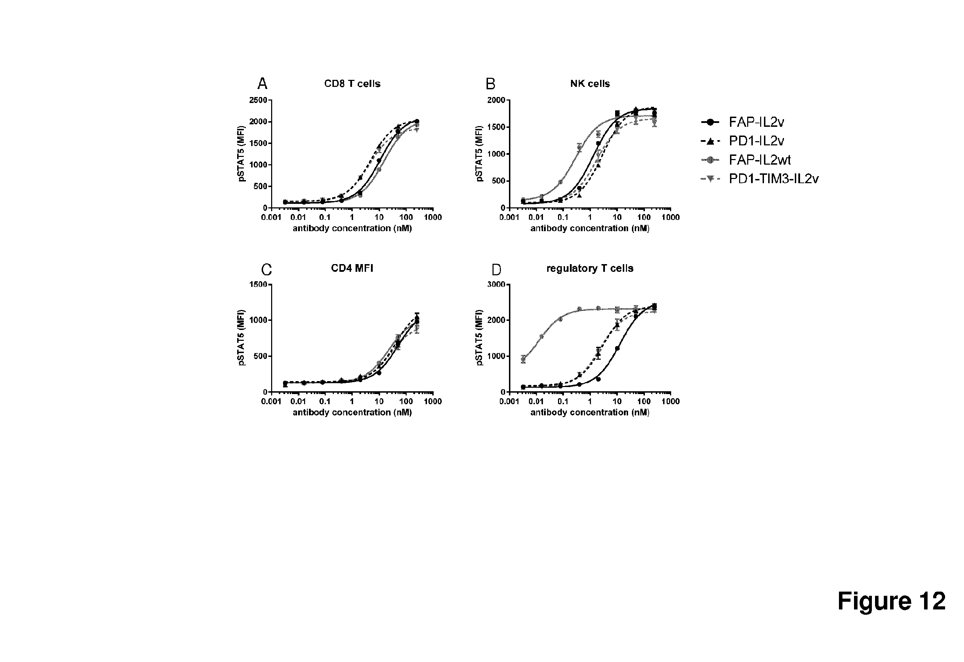

Figure 12 shows STAT5 phosphorylation in CD8 T-cells (A), NK cells (B), CD4 T-cells (C) and regulatory T-cells (D) upon treatment of resting PBMCs of donor 3 with FAP-IL2v, PD1-IL2v, FAP-IL2wt, PD1-TIM3-IL2v. All four tested molecules are comparable active on CD8 T-cells, NK cells and CD4 T-cells (excluding Tregs). FAP-IL2wt is more potent in inducing STAT5 phosphorylation in Tregs followed by PD1-IL2v. FAP-IL2v has the lowest activity on Tregs.

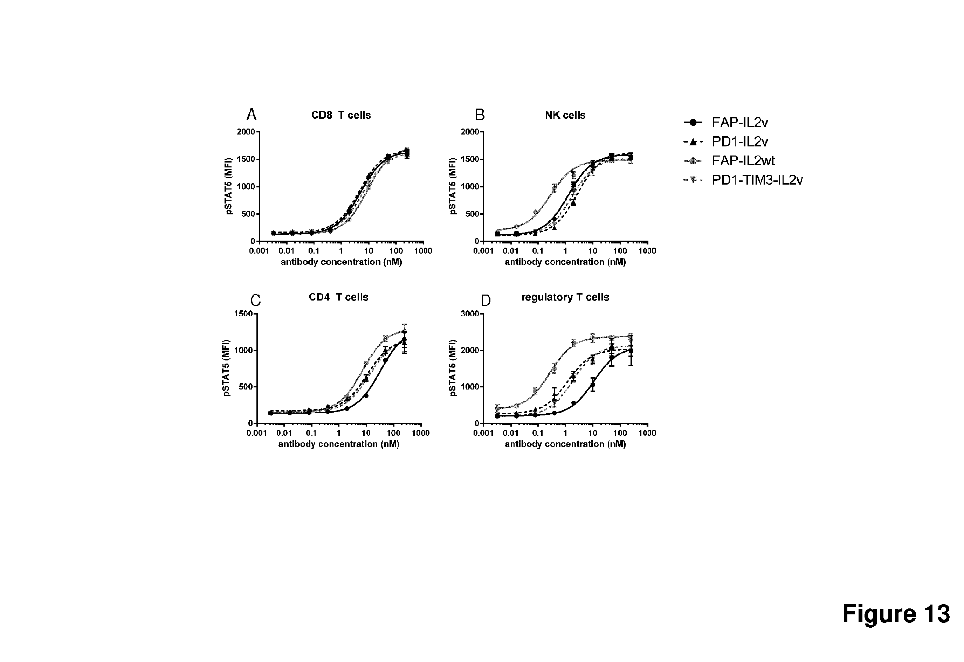

Figure 13 shows STAT5 phosphorylation in CD8 T-cells (A), NK cells (B), CD4 T-cells (C) and regulatory T-cells (D) upon treatment of resting PBMCs of donor 4 with FAP-IL2v, PD1-IL2v, FAP-IL2wt, PD1-TIM3-IL2v. All four tested molecules are comparable active on CD8 T cells, NK cells and CD4 T cells (excluding Tregs). FAP-IL2wt is more potent in inducing STAT5 phosphorylation in Tregs followed by PD1-IL2v. FAP-IL2v has the lowest activity on Tregs.

Claims

1. An immunoconjugate comprising a mutant IL-2 polypeptide and a bispecific antigen binding molecule that binds to PD-1 and Tim-3,

   wherein the mutant IL-2 polypeptide is a human IL-2 molecule comprising the amino acid substitutions F42A, Y45A and L72G, the numbering being relative to the human IL-2 sequence SEQ ID NO: 22, wherein the mutant IL-2 has reduced or abolished affinity to the alpha subunit of the IL-2 receptor but preserves affinity to the intermediate affinity IL-2 receptor; and
   wherein the bispecific antigen binding molecule comprises

      (i) a first antigen binding moiety that binds to PD-1, and
      (ii) a second antigen binding moiety that binds to Tim-3.

2. The immunoconjugate of claim 1, wherein the first antigen binding moiety comprises
(a) a heavy chain variable region (VH) comprising a HVR-H1 comprising the amino acid sequence of SEQ ID NO:1, a HVR-H2 comprising the amino acid sequence of SEQ ID NO:2, and a HVR-H3 comprising the amino acid sequence of SEQ ID NO:3, and (b) a light chain variable region (VL) comprising a HVR-L1 comprising the amino acid sequence of SEQ ID NO:4, a HVR-L2 comprising the amino acid sequence of SEQ ID NO:5, and a HVR-L3 comprising the amino acid sequence of SEQ ID NO:6.

3. The immunoconjugate of claim 1 or 2, wherein the first antigen binding moiety comprises (a) a heavy chain variable region (VH) comprising an amino acid sequence that is at least about 95%, 96%, 97%, 98%, 99% or 100% identical to the amino acid sequence of SEQ ID NO:7, and (b) a light chain variable region (VL) comprising an amino acid sequence that is at least about 95%, 96%, 97%, 98%, 99% or 100% identical to an amino acid sequence selected from the group consisting of SEQ ID NO:8, SEQ ID NO:9, SEQ ID NO: 10, and SEQ ID NO:11.

4. The immunoconjugate of any one of claims 1 to 3, wherein the second antigen binding moiety comprises
(a) a heavy chain variable region (VH) comprising a HVR-H1 comprising the amino acid sequence of SEQ ID NO:12, a HVR-H2 comprising the amino acid sequence of SEQ ID NO:13, and a HVR-H3 comprising the amino acid sequence of SEQ ID NO:14, and (b) a light chain variable region (VL) comprising a HVR-L1 comprising the amino acid sequence of SEQ ID NO:15, a HVR-L2 comprising the amino acid sequence of SEQ ID NO:16, and a HVR-L3 comprising the amino acid sequence of SEQ ID NO:17.

5. The immunoconjugate of any one of claims 1 to 4, wherein the second antigen binding moiety comprises

(a) a heavy chain variable region (VH) comprising an amino acid sequence that is at least about 95%, 96%, 97%, 98%, 99% or 100% identical to the amino acid sequence of SEQ ID NO:18, and (b) a light chain variable region (VL) comprising an amino acid sequence that is at least about 95%, 96%, 97%, 98%, 99% or 100% identical to the amino acid sequence of SEQ ID NO:19, or
(a) a heavy chain variable region (VH) comprising an amino acid sequence that is at least about 95%, 96%, 97%, 98%, 99% or 100% identical to the amino acid sequence of SEQ ID NO:20, and (b) a light chain variable region (VL) comprising an amino acid sequence that is at least about 95%, 96%, 97%, 98%, 99% or 100% identical to the amino acid sequence of SEQ ID NO:21.

6. The immunoconjugate of any one of claims 1 to 5, wherein the mutant IL-2 polypeptide further comprises the amino acid substitution T3A and/or the amino acid substitution C125A.

7. The immunoconjugate of any one of claims 1 to 6, wherein the mutant IL-2 polypeptide comprises the sequence of SEQ ID NO: 23.

8. The immunoconjugate of any one of claims 1 to 7, wherein the immunoconjugate comprises not more than one mutant IL-2 polypeptide.

9. The immunoconjugate of any one of claims 1 to 8, wherein the first and/or the second antigen binding moiety is a Fab molecule.

10. The immunoconjugate of any one of claims 1 to 9, wherein the first or the second antigen binding moiety is a Fab molecule wherein the variable domains VL and VH or the constant domains CL and CH1, particularly the variable domains VL and VH, of the Fab light chain and the Fab heavy chain are replaced by each other.

11. The immunoconjugate of any one of claims 1 to 10, wherein the first or the second antigen binding moiety is a Fab molecule wherein in the constant domain the amino acid at position 124 is substituted independently by lysine (K), arginine (R) or histidine (H) in the numbering according to Kabat, and the amino acid at position 123 is substituted independently by lysine (K), arginine (R) or histidine (H) in the numbering according to Kabat, and in the constant domain CH1 the amino acid at position 147 is substituted independently by glutamic acid (E), or aspartic acid (D) in the numbering according to Kabat EU index and the amino acid at position 213 is substituted independently by glutamic acid (E), or aspartic acid (D) in the numbering according to Kabat EU index.

12. The immunoconjugate of any one of claim 1 to 11, wherein the first antigen binding moiety is a Fab molecule wherein the variable domains VL and VH of the Fab light chain and the Fab heavy chain are replaced by each other, and the second antigen binding moiety is a Fab molecule wherein in the constant domain the amino acid at position 124 is substituted lysine (K) in the numbering according to Kabat, and the amino acid at position 123 is substituted independently by lysine (K) or arginine (R), particularly by arginine (R) in the numbering according to Kabat, and in the constant domain CHI the amino acid at position 147 is substituted by glutamic acid (E) in the numbering according to Kabat EU index, and the amino acid at position 213 is substituted by glutamic acid (E) in the numbering according to Kabat EU index.

13. The immunoconjugate of any one of claims 1 to 6, wherein the bispecific antigen binding molecule further comprises an Fc domain composed of a first and a second subunit.

14. The immunoconjugate of claim 13, wherein the Fc domain is an IgG class, particularly an IgG$_1$ subclass, Fc domain.

15. The immunoconjugate of claim 13 or 14, wherein the Fc domain is a human Fc domain.

16. The immunoconjugate of any one of claims 13 to 15, wherein the Fc domain comprises a modification promoting the association of the first and the second subunit of the Fc domain.

17. The immunoconjugate of any one of claims 13 to 16, wherein in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

18. The immunoconjugate of any one of claims 13 to 17, wherein in the first subunit of the Fc domain the threonine residue at position 366 is replaced with a tryptophan residue (T366W), and in the CH3 domain of te second subunit of the Fc domain the tyrosine residue at position 407 is replaced with a valine residue (Y407V) and optionally the threonine residue at position 366 is replaced with a serine residue (T366S) and the leucine residue at position 368 is replaced with an alanine residue (L368A) in the numberings according to Kabat EU index.

19. The immunoconjugate of claim 18, wherein in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C) in the numberings according to Kabat EU index.

20. The immunoconjugate of any one of claims 13 to 19, wherein the mutant IL-2 polypeptide is fused at its amino-terminal amino acid to the carboxy-terminal amino acid of one of the subunits of the Fc domain, particularly the first subunit of the Fc domain, optionally through a linker peptide.

21. The immunoconjugate of claim 20, wherein the linker peptide has the amino acid sequence of SEQ ID NO:24.

22. The immunoconjugate of any one of claims 13 to 21, wherein the first antigen binding moiety is a Fab molecule and is fused at the C-terminus of the Fab heavy chain to the N-terminus of one of the subunits of the Fc domain, particularly to the first subunit of the Fc domain, and the second antigen binding moiety is a Fab molecule and is fused at the C-terminus of the Fab heavy chain to the N-terminus of one of the subunits of the Fc domain, particularly to the second subunit of the Fc domain

23. The immunoconjugate of claim 22, wherein the first and the second antigen binding moiety are each fused to the Fc domain through an immunoglobulin hinge region.

24. The immunoconjugate of any one of claims 13 to 23, wherein the Fc domain comprises one or more amino acid substitution that reduces binding to an Fc receptor, particularly an Fcγ receptor, and/or effector function, particularly antibody-dependent cell-mediated cytotoxicity (ADCC).

25. The immunoconjugate of claim 24, wherein said one or more amino acid substitution is at one or more position selected from the group of L234, L235, and P329 in the Kabat EU index numbering.

26. The immunoconjugate of any one of claims 13 to 25, wherein each subunit of the Fc domain comprises the amino acid substitutions L234A, L235A and P329G in the Kabat EU index numbering.

27. The immunoconjugate of any one of claims 1 to 26, comprising a polypeptide comprising an amino acid sequence that is at least about 80%, 85%, 90%, 95%, 96%, 97%, 98%, 99% or 100% identical to the sequence of SEQ ID NO:25, a polypeptide comprising an amino acid sequence that is at least about 80%, 85%, 90%, 95%, 96%, 97%, 98%, 99% or 100% identical to the sequence of SEQ ID NO:26, a polypeptide comprising an amino acid sequence that is at least about 80%, 85%, 90%, 95%, 96%, 97%, 98%, 99% or 100% identical to the sequence of SEQ ID NO:27, and a polypeptide comprising an amino acid sequence that is at least about 80%, 85%, 90%, 95%, 96%, 97%, 98%, 99% or 100% identical to the sequence of SEQ ID NO:28.

28. The immunoconjugate of any one of claims 1 to 27, essentially consisting of a mutant IL-2 polypeptide and an IgG₁ immunoglobulin molecule wherein the heavy and light chain variable or constant regions in one of the Fab molecules are replaced by each other, joined by a linker sequence.

29. One or more isolated polynucleotide encoding the immunoconjugate of any one of claims 1 to 28.

30. One or more vector, particularly expression vector, comprising the polynucleotide(s) of claim 29.

31. A host cell comprising the polynucleotide(s) of claim 29 or the vector(s) of claim 30.

32. A method of producing an immunoconjugate comprising a mutant IL-2 polypeptide and a bispecific antigen binding molecule that binds to PD-1 and Tim-3, comprising (a) culturing the host cell of claim 31 under conditions suitable for the expression of the immunoconjugate, and optionally (b) recovering the immunoconjugate.

33. A pharmaceutical composition comprising the immunoconjugate of any one of claims 1 to 28 and a pharmaceutically acceptable carrier.

34. The immunoconjugate of any one of claims 1 to 28 for use as a medicament.

35. The immunoconjugate of any one of claims 1 to 28 for use in the treatment of cancer.

**Patentansprüche**

1. Immunkonjugat, umfassend ein mutiertes IL-2-Polypeptid und ein bispezifisches antigenbindendes Molekül, das an PD-1 und Tim-3 bindet,

   wobei das mutierte IL-2-Polypeptid ein humanes IL-2-Molekül ist, das die Aminosäuresubstitutionen F42A, Y45A und L72G umfasst, wobei sich die Nummerierung auf die humane IL-2-Sequenz SEQ ID NO:22 bezieht, wobei das mutierte IL-2 reduzierte oder aufgehobene Affinität zu der alpha-Untereinheit des IL-2-Rezeptors aufweist, jedoch Affinität zu dem mittelaffinen IL-2-Rezeptor erhält; und
   wobei das bispezifische antigenbindende Molekül Folgendes umfasst

      (i) eine erste Antigenbindungseinheit, die an PD-1 bindet, und
      (ii) eine zweite Antigenbindungseinheit, die an Tim-3 bindet.

2. Immunkonjugat nach Anspruch 1, wobei die erste Antigenbindungseinheit Folgendes umfasst

      (a) eine variable Region der schweren Kette (VH), umfassend eine HVR-H1, umfassend die Aminosäuresequenz von SEQ ID NO:1, eine HVR-H2, umfassend die Aminosäuresequenz von SEQ ID NO:2, und eine HVR-H3, umfassend die Aminosäuresequenz von SEQ ID NO:3, und
      (b) eine variable Region der leichten Kette (VL), umfassend eine HVR-L1, umfassend die Aminosäuresequenz von SEQ ID NO:4, eine HVR-L2, umfassend die Aminosäuresequenz von SEQ ID NO:5, und eine HVR-L3, umfassend die Aminosäuresequenz von SEQ ID NO:6.

3. Immunkonjugat nach Anspruch 1 oder 2, wobei die erste Antigenbindungseinheit Folgendes umfasst (a) eine variable Region der schweren Kette (VH), umfassend eine Aminosäuresequenz, die zu mindestens etwa 95 %, 96 %, 97 %, 98 %, 99 % oder 100 % identisch ist mit der Aminosäuresequenz von SEQ ID NO:7, und (b) eine variable Region der leichten Kette (VL), umfassend eine Aminosäuresequenz, die zu mindestens etwa 95 %, 96 %, 97 %, 98 %, 99 % oder 100 % identisch ist mit einer Aminosäuresequenz, ausgewählt aus der Gruppe, bestehend aus SEQ ID NO:8, SEQ ID NO:9, SEQ ID NO:10 und SEQ ID NO:11.

4. Immunkonjugat nach einem der Ansprüche 1 bis 3, wobei die zweite Antigenbindungseinheit Folgendes umfasst (a) eine variable Region der schweren Kette (VH), umfassend eine HVR-H1, umfassend die Aminosäuresequenz von SEQ ID NO:12, eine HVR-H2, umfassend die Aminosäuresequenz von SEQ ID NO:13, und eine HVR-H3, umfassend die Aminosäuresequenz von SEQ ID NO:14, und (b) eine variable Region der leichten Kette (VL), umfassend eine HVR-L1, umfassend die Aminosäuresequenz von SEQ ID NO:15, eine HVR-L2, umfassend die Aminosäuresequenz von SEQ ID NO:16, und eine HVR-L3, umfassend die Aminosäuresequenz von SEQ ID NO:17.

5. Immunkonjugat nach einem der Ansprüche 1 bis 4, wobei die zweite Antigenbindungseinheit Folgendes umfasst

(a) eine variable Region der schweren Kette (VH), umfassend eine Aminosäuresequenz, die zu mindestens etwa 95 %, 96 %, 97 %, 98 %, 99 % oder 100 % identisch ist mit der Aminosäuresequenz von SEQ ID NO:18, und (b) eine variable Region der leichten Kette (VL), umfassend eine Aminosäuresequenz, die zu mindestens etwa 95 %, 96 %, 97 %, 98 %, 99 % oder 100 % identisch ist mit der Aminosäuresequenz von SEQ ID NO:19, oder (a) eine variable Region der schweren Kette (VH), umfassend eine Aminosäuresequenz, die zu mindestens etwa 95 %, 96 %, 97 %, 98 %, 99 % oder 100 % identisch ist mit der Aminosäuresequenz von SEQ ID NO:20, und (b) eine variable Region der leichten Kette (VL), umfassend eine Aminosäuresequenz, die zu mindestens etwa 95 %, 96 %, 97 %, 98 %, 99 % oder 100 % identisch ist mit der Aminosäuresequenz von SEQ ID NO:21.

6. Immunkonjugat nach einem der Ansprüche 1 bis 5, wobei das mutierte IL-2-Polypeptid ferner die Aminosäuresubstitution T3A und/oder die Aminosäuresubstitution C125A umfasst.

7. Immunkonjugat nach einem der Ansprüche 1 bis 6, wobei das mutierte IL-2-Polypeptid die Sequenz von SEQ ID NO:23 umfasst.

8. Immunkonjugat nach einem der Ansprüche 1 bis 7, wobei das Immunkonjugat nicht mehr als ein mutiertes IL-2-Polypeptid umfasst.

9. Immunkonjugat nach einem der Ansprüche 1 bis 8, wobei die erste und/oder die zweite Antigenbindungseinheit ein Fab-Molekül sind/ist.

10. Immunkonjugat nach einem der Ansprüche 1 bis 9, wobei die erste oder die zweite Antigenbindungseinheit ein Fab-Molekül ist, wobei die variablen Domänen VL und VH oder die konstanten Domänen CL und CH1, insbesondere die variablen Domänen VL und VH, der leichten Kette des Fab und der schweren Kette des Fab gegeneinander ausgetauscht sind.

11. Immunkonjugat nach einem der Ansprüche 1 bis 10, wobei die erste oder die zweite Antigenbindungseinheit ein Fab-Molekül ist, wobei in der konstanten Domäne die Aminosäure an Position 124 unabhängig voneinander durch Lysin (K), Arginin (R) oder Histidin (H) ersetzt ist, Nummerierung gemäß Kabat, und die Aminosäure an Position 123 unabhängig voneinander durch Lysin (K), Arginin (R) oder Histidin (H) ersetzt ist, Nummerierung gemäß Kabat, und in der konstanten Domäne CH1 die Aminosäure an Position 147 unabhängig voneinander durch Glutaminsäure (E) oder Asparaginsäure (D) ersetzt ist, Nummerierung gemäß Kabat-EU-Index, und die Aminosäure an Position 213 unabhängig voneinander durch Glutaminsäure (E) oder Asparaginsäure (D) ersetzt ist, Nummerierung gemäß Kabat-EU-Index.

12. Immunkonjugat nach einem von Anspruch 1 bis 11, wobei die erste Antigenbindungseinheit ein Fab-Molekül ist, wobei die variablen Domänen VL und VH der leichten Kette des Fab und der schweren Kette des Fab gegeneinander ausgetauscht sind und die zweite Antigenbindungseinheit ein Fab-Molekül ist, wobei in der konstanten Domäne die Aminosäure an Position 124 durch Lysin (K) ersetzt ist, Nummerierung gemäß Kabat, und die Aminosäure an Position 123 unabhängig voneinander durch Lysin (K) oder Arginin (R), insbesondere durch Arginin (R) ersetzt ist, Nummerierung gemäß Kabat, und in der konstanten Domäne CH1 die Aminosäure an Position 147 durch Glutaminsäure (E) ersetzt ist, Nummerierung gemäß Kabat-EU-Index, und die Aminosäure an Position 213 durch Glutaminsäure (E) ersetzt ist, Nummerierung gemäß Kabat-EU-Index.

13. Immunkonjugat nach einem der Ansprüche 1 bis 6, wobei das bispezifische antigenbindende Molekül ferner eine Fc-Domäne umfasst, die aus einer ersten und einer zweiten Untereinheit besteht.

**14.** Immunkonjugat nach Anspruch 13, wobei die Fc-Domäne eine Fc-Domäne der IgG-Klasse, insbesondere der IgG$_1$-Unterlasse ist.

**15.** Immunkonjugat nach Anspruch 13 oder 14, wobei die Fc-Domäne eine humane Fc-Domäne ist.

**16.** Immunkonjugat nach einem der Ansprüche 13 bis 15, wobei die Fc-Domäne eine Modifikation umfasst, welche die Assoziation der ersten und der zweiten Untereinheit der Fc-Domäne unterstützt.

**17.** Immunkonjugat nach einem der Ansprüche 13 bis 16, wobei in der CH3-Domäne der ersten Untereinheit der Fc-Domäne ein Aminosäurerest durch einen Aminosäurerest mit einem größeren Seitenkettenvolumen ersetzt ist, wodurch eine Protuberanz in der CH3-Domäne der ersten Untereinheit erzeugt wird, die in einer Vertiefung in der CH3-Domäne der zweiten Untereinheit positioniert werden kann, und in der CH3-Domäne der zweiten Untereinheit der Fc-Domäne ein Aminosäurerest durch einen Aminosäurerest mit einem kleineren Seitenkettenvolumen ersetzt ist, wodurch eine Vertiefung in der CH3-Domäne der zweiten Untereinheit erzeugt wird, in welcher die Protuberanz in der CH3-Domäne der ersten Untereinheit positioniert werden kann.

**18.** Immunkonjugat nach einem der Ansprüche 13 bis 17, wobei in der ersten Untereinheit der Fc-Domäne der Threonin-Rest an Position 366 durch einen Tryptophan-Rest ersetzt ist (T366W) und in der CH3-Domäne der zweiten Untereinheit der Fc-Domäne der Tyrosin-Rest an Position 407 durch einen Valin-Rest ersetzt ist (Y407V) und gegebenenfalls der Threonin-Rest an Position 366 durch einen Serin-Rest ersetzt ist (T366S) und der Leucin-Rest an Position 368 durch einen Alanin-Rest ersetzt ist (L368A), Nummerierungen gemäß Kabat-EU-Index.

**19.** Immunkonjugat nach Anspruch 18, wobei in der ersten Untereinheit der Fc-Domäne zusätzlich der Serin-Rest an Position 354 durch einen Cystein-Rest ersetzt ist (S354C) oder der Glutaminsäure-Rest an Position 356 durch einen Cystein-Rest ersetzt ist (E356C) und in der zweiten Untereinheit der Fc-Domäne zusätzlich der Tyrosin-Rest an Position 349 durch einen Cystein-Rest ersetzt ist (Y349C), Nummerierungen gemäß Kabat-EU-Index.

**20.** Immunkonjugat nach einem der Ansprüche 13 bis 19, wobei das mutierte IL-2-Polypeptid an seiner aminoterminalen Aminosäure gegebenenfalls durch ein Linker-Peptid an die carboxyterminale Aminosäure einer der Untereinheiten der Fc-Domäne, insbesondere der ersten Untereinheit der Fc-Domäne, fusioniert ist.

**21.** Immunkonjugat nach Anspruch 20, wobei das Linker-Peptid die Aminosäuresequenz von SEQ ID NO:24 aufweist.

**22.** Immunkonjugat nach einem der Ansprüche 13 bis 21, wobei die erste Antigenbindungseinheit ein Fab-Molekül ist und am C-Terminus der schweren Kette des Fab an den N-Terminus einer der Untereinheiten der Fc-Domäne, insbesondere der ersten Untereinheit der Fc-Domäne, fusioniert ist und die zweite Antigenbindungseinheit ein Fab-Molekül ist und am C-Terminus der schweren Kette des Fab an den N-Terminus einer der Untereinheiten der Fc-Domäne, insbesondere der zweiten Untereinheit der Fc-Domäne, fusioniert ist.

**23.** Immunkonjugat nach Anspruch 22, wobei die erste und die zweite Antigenbindungseinheit durch eine Immunglobulin-Gelenkregion jeweils an die Fc-Domäne fusioniert sind.

**24.** Immunkonjugat nach einem der Ansprüche 13 bis 23, wobei die Fc-Domäne eine oder mehrere Aminosäuresubstitution(en) umfasst, die das Binden an einen Fc-Rezeptor, insbesondere einen Fcγ-Rezeptor, und/oder die Effektorfunktion, insbesondere Antikörperabhängige zellvermittelte Zytotoxizität (ADCC), reduziert/reduzieren.

**25.** Immunkonjugat nach Anspruch 24, wobei sich die eine oder mehreren Aminosäuresubstitution(en) an einer oder mehreren Position(en) befindet/befinden, die aus der Gruppe von L234, L235 und P329 nach der Kabat-EU-Index-Nummerierung ausgewählt ist/sind.

**26.** Immunkonjugat nach einem der Ansprüche 13 bis 25, wobei jede Untereinheit der Fc-Domäne die Aminosäuresubstitutionen L234A, L235A und P329G nach der Kabat-EU-Index-Nummerierung umfasst.

**27.** Immunkonjugat nach einem der Ansprüche 1 bis 26, umfassend ein Polypeptid, umfassend eine Aminosäuresequenz, die zu mindestens etwa 80 %, 85 %, 90 %, 95 %, 96 %, 97 %, 98 %, 99 % oder 100 % identisch ist mit der Sequenz von SEQ ID NO:25, ein Polypeptid, umfassend eine Aminosäuresequenz, die zu mindestens etwa 80 %, 85 %, 90 %, 95 %, 96 %, 97 %, 98 %, 99 % oder 100 % identisch ist mit der Sequenz von SEQ ID NO:26, ein Polypeptid, umfassend eine Aminosäuresequenz, die zu mindestens etwa 80 %, 85 %, 90 %, 95 %, 96 %, 97 %, 98 %, 99 % oder 100 % identisch ist mit der Sequenz von SEQ ID NO:27, und ein Polypeptid, umfassend eine Aminosäuresequenz, die zu mindestens etwa 80 %, 85 %, 90 %, 95 %, 96 %, 97 %, 98 %, 99 % oder 100 % identisch ist mit der Sequenz von SEQ ID NO:28.

28. Immunkonjugat nach einem der Ansprüche 1 bis 27, das im Wesentlichen aus einem mutierten IL-2-Polypeptid und einem IgG$_1$-Immunglobulinmolekül, die durch eine Linker-Sequenz verbunden sind, besteht, wobei die variablen oder konstanten Regionen der schweren und der leichten Kette in einem der Fab-Moleküle gegeneinander ausgetauscht sind.

29. Ein isoliertes oder mehrere isolierte Polynukleotid(e), das/die für das Immunkonjugat nach einem der Ansprüche 1 bis 28 kodiert/kodieren.

30. Ein oder mehrere Vektor(en), insbesondere Expressionsvektor(en), umfassend das/die Polynukleotid(e) nach Anspruch 29.

31. Wirtszelle, umfassend das/die Polynukleotid(e) nach Anspruch 29 oder den/die Vektor(en) nach Anspruch 30.

32. Verfahren zum Produzieren eines Immunkonjugats, umfassend ein mutiertes IL-2-Polypeptid und ein bispezifisches antigenbindendes Molekül, das an PD-1 und Tim-3 bindet, umfassend (a) das Kultivieren der Wirtszelle nach Anspruch 31 unter Bedingungen, die für die Expression des Immunkonjugats geeignet sind, und gegebenenfalls (b) Gewinnen des Immunkonjugats.

33. Pharmazeutische Zusammensetzung, umfassend das Immunkonjugat nach einem der Ansprüche 1 bis 28 und einen pharmazeutisch unbedenklichen Träger.

34. Immunkonjugat nach einem der Ansprüche 1 bis 28 zur Verwendung als ein Medikament.

35. Immunkonjugat nach einem der Ansprüche 1 bis 28 zur Verwendung bei der Behandlung von Krebs.


**Revendications**

1. Immunoconjugué comprenant un polypeptide d'IL-2 mutante et une molécule bispécifique de liaison à l'antigène qui se lie à PD-1 et Tim-3,

   dans lequel le polypeptide d'IL-2 mutante est une molécule d'IL-2 humaine comprenant les substitutions d'acides aminés F42A, Y45A et L72G, la numérotation étant relative à la séquence d'IL-2 humaine de SEQ ID NO : 22, dans lequel l'IL-2 mutante a réduit ou aboli l'affinité avec la sous-unité alpha du récepteur de l'IL-2 mais conserve l'affinité avec le récepteur de l'IL-2 d'affinité intermédiaire ; et dans lequel la molécule bispécifique de liaison à l'antigène comprend

   (i) une première fraction de liaison à l'antigène qui se lie à PD-1, et
   (ii) une seconde fraction de liaison à l'antigène qui se lie à Tim-3.

2. Immunoconjugué selon la revendication 1, dans lequel la première fraction de liaison à l'antigène comprend (a) une région variable de chaîne lourde (VH) comprenant une HVR-H1 comprenant la séquence d'acides aminés de SEQ ID NO : 1, une HVR-H2 comprenant la séquence d'acides aminés de SEQ ID NO : 2 et une HVR-H3 comprenant la séquence d'acides aminés de SEQ ID NO : 3, et (b) une région variable de chaîne légère (VL) comprenant une HVR-L1 comprenant la séquence d'acides aminés de SEQ ID NO : 4, une HVR-L2 comprenant la séquence d'acides aminés de SEQ ID NO : 5 et une HVR-L3 comprenant la séquence d'acides aminés de SEQ ID NO : 6.

3. Immunoconjugué selon la revendication 1 ou 2, dans lequel la première fraction de liaison à l'antigène comprend (a) une région variable de chaîne lourde (VH) comprenant une séquence d'acides aminés qui est au moins environ 95 %, 96 %, 97 %, 98 %, 99 % ou 100 % identique à la séquence d'acides aminés de SEQ ID NO : 7, et (b) une région variable de chaîne légère (VL) comprenant une séquence d'acides aminés qui est au moins environ 95 %, 96 %, 97 %, 98 %, 99 % ou 100 % identique à une séquence d'acides aminés choisie dans le groupe constitué par SEQ ID NO : 8, SEQ ID NO : 9, SEQ ID NO : 10 et SEQ ID NO : 11.

4. Immunoconjugué selon l'une quelconque des revendications 1 à 3, dans lequel la seconde fraction de liaison à l'antigène comprend
(a) une région variable de chaîne lourde (VH) comprenant une HVR-H1 comprenant la séquence d'acides aminés de SEQ ID NO : 12, une HVR-H2 comprenant la séquence d'acides aminés de SEQ ID NO : 13 et une HVR-H3 comprenant la séquence d'acides aminés de SEQ ID NO : 14, et (b) une région variable de chaîne légère (VL) comprenant une HVR-L1 comprenant la séquence d'acides aminés de SEQ ID NO : 15, une HVR-L2 comprenant la séquence d'acides aminés de SEQ ID NO : 16 et une HVR-L3 comprenant la séquence d'acides aminés de SEQ ID NO : 17.

5. Immunoconjugué selon l'une quelconque des revendications 1 à 4, dans lequel la seconde fraction de liaison à l'antigène comprend

(a) une région variable de chaîne lourde (VH) comprenant une séquence d'acides aminés qui est au moins environ 95 %, 96 %, 97 %, 98 %, 99 % ou 100 % identique à la séquence d'acides aminés de SEQ ID NO : 18, et (b) une région variable de chaîne légère (VL) comprenant une séquence d'acides aminés qui est au moins environ 95 %, 96 %, 97 %, 98 %, 99 % ou 100 % identique à la séquence d'acides aminés de SEQ ID NO : 19, ou (a) une région variable de chaîne lourde (VH) comprenant une séquence d'acides aminés qui est au moins environ 95 %, 96 %, 97 %, 98 %, 99 % ou 100 % identique à la séquence d'acides aminés de SEQ ID NO : 20, et (b) une région variable de chaîne légère (VL) comprenant une séquence d'acides aminés qui est au moins environ 95 %, 96 %, 97 %, 98 %, 99 % ou 100 % identique à la séquence d'acides aminés de SEQ ID NO : 21.

6. Immunoconjugué selon l'une quelconque des revendications 1 à 5, dans lequel le polypeptide d'IL-2 mutante comprend en outre la substitution d'acide aminé T3A et/ou la substitution d'acide aminé C125A.

7. Immunoconjugué selon l'une quelconque des revendications 1 à 6, dans lequel le polypeptide d'IL-2 mutante comprend la séquence de SEQ ID NO : 23.

8. Immunoconjugué selon l'une quelconque des revendications 1 à 7, dans lequel l'immunoconjugué ne comprend pas plus d'un polypeptide d'IL-2 mutante.

9. Immunoconjugué selon l'une quelconque des revendications 1 à 8, dans lequel la première et/ou la seconde fraction de liaison à l'antigène est une molécule Fab.

10. Immunoconjugué selon l'une quelconque des revendications 1 à 9, dans lequel la première ou la seconde fraction de liaison à l'antigène est une molécule Fab dans laquelle les domaines variables VL et VH ou les domaines constants CL et CH1, en particulier les domaines variables VL et VH, de la chaîne légère de Fab et de la chaîne lourde de Fab sont remplacés les uns par les autres.

11. Immunoconjugué selon l'une quelconque des revendications 1 à 10, dans lequel la première ou la seconde fraction de liaison à l'antigène est une molécule Fab dans laquelle dans le domaine constant l'acide aminé en position 124 est substitué indépendamment par la lysine (K), l'arginine (R) ou l'histidine (H) dans la numérotation selon Kabat et l'acide aminé en position 123 est substitué indépendamment par la lysine (K), l'arginine (R) ou l'histidine (H) dans la numérotation selon Kabat, et dans le domaine constant CH1 l'acide aminé en position 147 est substitué indépendamment par l'acide glutamique (E) ou l'acide aspartique (D) dans la numérotation selon l'index EU de Kabat et l'acide aminé en position 213 est substitué indépendamment par l'acide glutamique (E) ou l'acide aspartique (D) dans la numérotation selon l'index EU de Kabat.

12. Immunoconjugué selon l'une quelconque des revendications 1 à 11, dans lequel la première fraction de liaison à l'antigène est une molécule Fab dans laquelle les domaines variables VL et VH de la chaîne légère de Fab et de la chaîne lourde de Fab sont remplacés les uns par les autres, et la seconde fraction de liaison à l'antigène est une molécule Fab dans laquelle dans le domaine constant l'acide aminé en position 124 est substitué par la lysine (K) dans la numérotation selon Kabat et l'acide aminé en position 123 est substitué indépendamment par la lysine (K) ou l'arginine (R), en particulier par l'arginine (R) dans la numérotation selon Kabat, et dans le domaine constant CH1 l'acide aminé en position 147 est substitué par l'acide glutamique (E) dans la numérotation selon l'index EU de Kabat et l'acide aminé en position 213 est substitué par l'acide glutamique (E) dans la numérotation selon l'index EU de Kabat.

13. Immunoconjugué selon l'une quelconque des revendications 1 à 6, dans lequel la molécule bispécifique de liaison à l'antigène comprend en outre un domaine Fc composé d'une première et d'une seconde sous-unité.

14. Immunoconjugué selon la revendication 13, dans lequel le domaine Fc est un domaine Fc de la classe des IgG, en particulier de la sous-classe IgG₁.

15. Immunoconjugué selon la revendication 13 ou 14, dans lequel le domaine Fc est un domaine Fc humain.

16. Immunoconjugué selon l'une quelconque des revendications 13 à 15, dans lequel le domaine Fc comprend une modification favorisant l'association de la première et de la seconde sous-unité du domaine Fc.

17. Immunoconjugué selon l'une quelconque des revendications 13 à 16, dans lequel dans le domaine CH3 de la première sous-unité du domaine Fc un résidu d'acide aminé est remplacé par un résidu d'acide aminé ayant un plus grand volume de chaîne latérale, générant ainsi une protubérance au sein du domaine CH3 de la première sous-unité qui peut être positionnée dans une cavité au sein du domaine CH3 de la seconde sous-unité, et dans le domaine CH3 de la seconde sous-unité du domaine Fc un résidu d'acide aminé est remplacé par un résidu d'acide aminé ayant un plus petit volume de chaîne latérale, générant ainsi une cavité au sein du domaine CH3 de la seconde sous-unité au sein de laquelle la protubérance au sein du domaine CH3 de la première sous-unité peut être positionnée.

18. Immunoconjugué selon l'une quelconque des revendications 13 à 17, dans lequel dans la première sous-unité du domaine Fc le résidu thréonine en position 366 est remplacé par un résidu tryptophane (T366W), et dans le domaine CH3 de la seconde sous-unité du domaine Fc le résidu tyrosine en position 407 est remplacé par un résidu valine (Y407V) et éventuellement le résidu thréonine en position 366 est remplacé par un résidu sérine (T366S) et le résidu leucine en position 368 est remplacé par un résidu alanine (L368A) dans les numérotations selon l'index EU de Kabat.

19. Immunoconjugué selon la revendication 18, dans lequel dans la première sous-unité du domaine Fc en outre le résidu sérine en position 354 est remplacé par un résidu cystéine (S354C) ou le résidu acide glutamique en position 356 est remplacé par un résidu cystéine (E356C), et dans la seconde sous-unité du domaine Fc en outre le résidu tyrosine en position 349 est remplacé par un résidu cystéine (Y349C) dans les numérotations selon l'index EU de Kabat.

20. Immunoconjugué selon l'une quelconque des revendications 13 à 19, dans lequel le polypeptide d'IL-2 mutante est fusionné au niveau de son acide aminé d'extrémité aminoterminale à l'acide aminé d'extrémité carboxy-terminale de l'une des sous-unités du domaine Fc, en particulier la première sous-unité du domaine Fc, éventuellement par le biais d'un lieur peptidique.

21. Immunoconjugué selon la revendication 20, dans lequel le lieur peptidique a la séquence d'acides aminés de SEQ ID NO : 24.

22. Immunoconjugué selon l'une quelconque des revendications 13 à 21, dans lequel la première fraction de liaison à l'antigène est une molécule Fab et est fusionnée au niveau de l'extrémité C-terminale de la chaîne lourde de Fab à l'extrémité N-terminale de l'une des sous-unités du domaine Fc, en particulier à la première sous-unité du domaine Fc, et la seconde fraction de liaison à l'antigène est une molécule Fab et est fusionnée au niveau de l'extrémité C-terminale de la chaîne lourde de Fab à l'extrémité N-terminale de l'une des sous-unités du domaine Fc, en particulier à la seconde sous-unité du domaine Fc.

23. Immunoconjugué selon la revendication 22, dans lequel la première et la seconde fraction de liaison à l'antigène sont chacune fusionnées au domaine Fc par le biais d'une région charnière d'immunoglobuline.

24. Immunoconjugué selon l'une quelconque des revendications 13 à 23, dans lequel le domaine Fc comprend une ou plusieurs substitutions d'acides aminés qui diminuent la liaison à un récepteur Fc, en particulier un récepteur Fcγ, et/ou une fonction effectrice, en particulier la cytotoxicité à médiation cellulaire dépendante des anticorps (ADCC).

25. Immunoconjugué selon la revendication 24, dans lequel ladite ou lesdites substitutions d'acides aminés sont au niveau d'une ou de plusieurs positions choisies dans le groupe constitué par L234, L235 et P329 dans la numérotation selon l'index EU de Kabat.

26. Immunoconjugué selon l'une quelconque des revendications 13 à 25, dans lequel chaque sous-unité du domaine Fc comprend les substitutions d'acides aminés L234A, L235A et P329G dans la numérotation selon l'index EU de Kabat.

27. Immunoconjugué selon l'une quelconque des revendications 1 à 26, comprenant un polypeptide comprenant une séquence d'acides aminés qui est au moins environ 80 %, 85 %, 90 %, 95 %, 96 %, 97 %, 98 %, 99 % ou 100 % identique à la séquence de SEQ ID NO : 25, un polypeptide comprenant une séquence d'acides aminés qui est au moins environ 80 %, 85 %, 90 %, 95 %, 96 %, 97 %, 98 %, 99 % ou 100 % identique à la séquence de SEQ ID NO : 26, un polypeptide comprenant une séquence d'acides aminés qui est au moins environ 80 %, 85 %, 90 %, 95 %, 96 %, 97 %, 98 %, 99 % ou 100 % identique à la séquence de SEQ ID NO : 27 et un polypeptide comprenant une séquence d'acides aminés qui est au moins environ 80 %, 85 %, 90 %, 95 %, 96 %, 97 %, 98 %, 99 % ou 100 % identique à la séquence de SEQ ID NO : 28.

28. Immunoconjugué selon l'une quelconque des revendications 1 à 27, essentiellement constitué d'un polypeptide d'IL-2 mutante et d'une molécule d'immunoglobuline IgG$_1$ dans laquelle les régions variables ou constantes de chaîne lourde et légère dans l'une des molécules Fab sont remplacées les unes par les autres, liés par une séquence de lieur.

29. Polynucléotide isolé ou polynucléotides isolés codant pour l'immunoconjugué selon l'une quelconque des revendications 1 à 28.

30. Vecteur ou vecteurs, en particulier vecteurs d'expression, comprenant le ou les polynucléotides selon la revendication 29.

31. Cellule hôte comprenant le ou les polynucléotides selon la revendication 29 ou le ou les vecteurs selon la revendication 30.

32. Procédé de production d'un immunoconjugué comprenant un polypeptide d'IL-2 mutante et une molécule bispécifique de liaison à l'antigène qui se lie à PD-1 et Tim-3, comprenant (a) la culture de la cellule hôte selon la revendication 31 dans des conditions appropriées pour l'expression de l'immunoconjugué et éventuellement (b) la récupération de l'immunoconjugué.

33. Composition pharmaceutique comprenant l'immunoconjugué selon l'une quelconque des revendications 1 à 28 et un véhicule pharmaceutiquement acceptable.

34. Immunoconjugué selon l'une quelconque des revendications 1 à 28 pour une utilisation comme médicament.

35. Immunoconjugué selon l'une quelconque des revendications 1 à 28 pour une utilisation dans le traitement d'un cancer.

**Figure 1**

EP 3 606 947 B1

**CD4 T cells**

A

**CD8 T cells**

B

EP 3 606 947 B1

**Figure 2**

**Figure 3**

Figure 4

Figure 5

Figure 5

Figure 6

CD4+ INFy+ IL2-

Figure 7

EP 3 606 947 B1

Figure 8

Figure 9

Figure 10

Figure 11

Figure 12

Figure 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030124678 A
- US 5229109 A
- US 20070036752 A, Gillies
- WO 20080034473 A, Gillies
- WO 2009061853 A, Wittrup
- WO 2012107417 A
- EP 2016073192 W
- WO 9316185 A
- US 5571894 A
- US 5587458 A
- US 5869046 A
- EP 404097 A
- WO 199301161 A
- US 6248516 B1
- WO 2006082515 A
- WO 2012130831 A
- US 4518584 A
- US 5206344 A
- US 5116943 A
- WO 2011020783 A
- WO 2012146628 A
- WO 2015150447 A
- WO 2009080252 A
- WO 2009080253 A
- WO 9627011 A
- WO 98050431 A
- EP 1870459 A
- WO 2007110205 A
- WO 2007147901 A
- WO 2009089004 A
- WO 2010129304 A
- WO 201190754 A
- WO 2011143545 A
- WO 2012058768 A
- WO 2013157954 A
- WO 2013096291 A
- US 5731168 A
- US 7695936 B
- WO 2013157953 A
- WO 2011090762 A
- US 6737056 B
- US 7332581 B
- US 5500362 A
- US 5821337 A
- WO 2006029879 A
- WO 2005100402 A
- WO 2013120929 A
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 7985840 B
- US 7785903 B
- US 8679490 B
- US 20050079574 A
- US 20070117126 A
- US 20070237764 A
- US 20070292936 A
- WO 8700056 A

**Non-patent literature cited in the description**

- **SMITH.** *Science,* 1988, vol. 240, 1169-76
- **BAZAN.** *Science,* 1992, vol. 257, 410-413
- **MINAMI et al.** *Annu Rev Immunol,* 1993, vol. 11, 245-268
- **KRIEG et al.** *Proc Natl Acad Sci,* 2010, vol. 107, 11906-11
- **FONTENOT et al.** *Nature Immunol,* 2005, vol. 6, 1142-51
- **WALDMANN.** *Nat Rev Immunol,* 2009, vol. 6, 595-601
- **OLEJNICZAK ; KASPRZAK.** *Med Sci Monit,* 2008, vol. 14, RA179-89
- **MALEK.** *Annu Rev Immunol,* 2008, vol. 26, 453-79
- **HU et al.** *Clin. Exp. Immunol.,* 2016, vol. 186, 106-114

- **LENARDO.** *Nature,* 1991, vol. 353, 858-61
- **D'CRUZ ; KLEIN.** *Nature Immunol,* 2005, vol. 6, 1152-59
- **MALOY ; POWRIE.** *Nature Immunol,* 2005, vol. 6, 1171-72
- **IMAI et al.** *Cancer Sci,* 2007, vol. 98, 416-23
- **KRIEG et al.** *Proc Nat Acad Sci USA,* 2010, vol. 107, 11906-11
- **KAMIMURA et al.** *J Immunol,* 2006, vol. 177, 306-14
- **BOYMAN et al.** *Science,* 2006, vol. 311, 1924-27
- **HU et al.** *Blood,* 2003, vol. 101, 4853-4861
- **SHANAFELT et al.** *Nature Biotechnol,* 2000, vol. 18, 1197-1202
- **HEATON et al.** *Cancer Res,* 1993, vol. 53, 2597-602
- **KO et al.** *J Immunother,* 2004, vol. 27, 232-239
- **KLEIN et al.** *Oncoimmunology,* 2017, vol. 6 (3), e1277306
- **GHASHEMI et al.** *Nat Comm,* 2016, vol. 7, 12878
- **OKAZAKI et al.** *Curr. Opin. Immunol.,* 2002, vol. 14, 391779-82
- **BENNETT et al.** *J Immunol,* 2003, vol. 170, 711-8
- **FREEMAN et al.** *J Exp Med,* 2000, vol. 192, 1027-34
- **LATCHMAN et al.** *Nat Immunol,* 2001, vol. 2, 261-8
- **CARTER et al.** *Eur J Immunol,* 2002, vol. 32, 634-43
- **DONG et al.** *Nat. Med,* 2002, vol. 8, 787-9
- **DONG et al.** *J. Mol. Med.,* 2003, vol. 81, 281-7
- **BLANK et al.** *Cancer Immunol. Immunother.,* 2005, vol. 54, 307-314
- **KONISHI et al.** *Clin. Cancer Res.,* 2004, vol. 10, 5094-100
- **IWAI et al.** *Proc. Nat 7. Acad. ScL USA,* 2002, vol. 99, 12293-7
- **BROWN et al.** *J. Immunol.,* 2003, vol. 170, 1257-66
- **GEHRING et al.** *Gastroenterology,* 2009, vol. 137, 682-690
- **SAKUISHI.** *J. Experimental Med.,* 2010, vol. 207, 2187-2194
- **MONNEY et al.** *Nature,* 2002, vol. 415, 536-541
- **ZHOU.** *Blood,* 2011, vol. 117, 4501-4510
- **MAJETI R et al.** *PNAS,* 2009, vol. 106, 3396-3401
- **OLEJNICZAK ; KASPRZAK.** *Med Sci Monit,* 2008, vol. 14, RA179-189
- **SAKAGUCHI.** *Annu Rev Immunol,* 2004, vol. 22, 531-62
- **LILJEBLAD et al.** *Glyco J,* 2000, vol. 17, 323-329
- **HEELEY.** *Endocr Res,* 2002, vol. 28, 217-229
- **W. R. PEARSON ; D. J. LIPMAN.** Improved Tools for Biological Sequence Analysis. *PNAS,* 1988, vol. 85, 2444-2448
- **W. R. PEARSON.** Effective protein sequence comparison. *Meth. Enzymol.,* 1996, vol. 266, 227-258
- **PEARSON.** *Genomics,* 1997, vol. 46, 24-36
- **FLATMAN et al.** *J. Chromatogr. B,* 2007, vol. 848, 79-87
- **HOLLIGER ; HUDSON.** *Nature Biotechnology,* 2005, vol. 23, 1126-1136
- **PLÜCKTHUN.** The Pharmacology of Monoclonal Antibodies. Springer-Verlag, 1994, vol. 113, 269-315
- **HUDSON et al.** *Nat Med,* 2003, vol. 9, 129-134
- **HOLLINGER et al.** *Proc Natl Acad Sci USA,* 1993, vol. 90, 6444-6448
- **KINDT et al.** Kuby Immunology. W.H. Freeman and Co, 2007, 91
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **MACCALLUM et al.** *J. Mol. Biol.,* 1996, vol. 262, 732-745
- **WEIGER et al.** *Eur J Biochem,* 1989, vol. 180, 295-300
- **SCHAEFER et al.** *PNAS,* 2011, vol. 108, 11187-11191
- **STUBENRAUCH et al.** *Drug Metabolism and Disposition,* 2010, vol. 38, 84-91
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **KING et al.** *J Clin Oncol,* 2004, vol. 22, 4463-4473
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **GAZZANO-SANTORO et al.** *J Immunol Methods,* 1996, vol. 202, 163
- **CRAGG et al.** *Blood,* 2003, vol. 101, 1045-1052
- **CRAGG ; GLENNIE.** *Blood,* 2004, vol. 103, 2738-2743
- **PETKOVA, S.B. et al.** *Int'l. Immunol.,* 2006, vol. 18 (12), 1759-1769

- **TANIGUCHI et al.** *Nature,* 1983, vol. 302, 305-10
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates and Wiley Interscience, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **LERNER et al.** *Nature Reviews,* 2016, vol. 16, 498-508
- **FRENZEL et al.** *mAbs,* 2016, vol. 8, 1177-1194
- **BAZAN et al.** *Human Vaccines and Immunotherapeutics,* 2012, vol. 8, 1817-1828
- **ZHAO et al.** *Critical Reviews in Biotechnology,* 2016, vol. 36, 276-289
- **HOOGENBOOM et al.** Methods in Molecular Biology. Human Press, 2001, vol. 178, 1-37
- **MARKS ; BRADBURY.** Methods in Molecular Biology. Human Press, 2003, vol. 248, 161-175
- **WINTER et al.** *Annual Review of Immunology,* 1994, vol. 12, 433-455
- **GRIFFITHS et al.** *EMBO Journal,* 1993, vol. 12, 725-734
- **HOOGENBOOM ; WINTER.** *Journal of Molecular Biology,* 1992, vol. 227, 381-388
- **SCHOLLER et al.** *Methods in Molecular Biology,* 2012, vol. 503, 135-56
- **CHERF et al.** *Methods in Molecular biology,* 2015, vol. 1319, 155-175
- **ZHAO et al.** *Methods in Molecular Biology,* 2012, vol. 889, 73-84
- **HE et al.** *Nucleic Acids Research,* 1997, vol. 25, 5132-5134
- **HANES et al.** *PNAS,* 1997, vol. 94, 4937-4942
- Remington's Pharmaceutical Sciences. Mack Printing Company, 1990
- Remington's Pharmaceutical Sciences. Mack Printing Company, 1990, 1289-1329
- **FINGL et al.** The Pharmacological Basis of Therapeutics. 1975, 1